# EUROPEAN PATENT SPECIFICATION

(11) **EP 3 877 388 B1**
(45) Date of publication and mention of the grant of the patent: **26.07.2023**
(21) Application number: 19805549.3
(22) Date of filing: 07.11.2019
(51) Int. Cl.: C07D 471/04, A61K 31/437, A61K 31/4709, A61K 31/5377, A61K 31/397, A61K 31/4545, A61P 37/02, A61P 37/06

(54) **5-[6-[[3-(4,5,6,7-TETRAHYDROPYRAZOLO[4,3-C]PYRIDIN-1-YL)AZETIDIN-1-YL]METHYL]MORPHOLIN-4-YL]QUINOLINE-8-CARBONITRILE DERIVATIVES AND SIMILAR COMPOUNDS AS TLR7-9 ANTAGONISTS FOR TREATING SYSTEMIC LUPUS ERYTHEMATOSUS**
5-[6-[[3-(4,5,6,7-TETRAHYDROPYRAZOLO[4,3-C]PYRIDIN-1-YL)AZETIDIN-1-YL]METHYL]MORPHOLIN-4-YL]CHINOLIN-8-CARBONITRIL-DERIVATE UND ÄHNLICHE VERBINDUNGEN ALS TLR7-9 ANTAGONISTEN ZUR BEHANDLUNG VON SYSTEMISCHEM LUPUS ERYTHEMATOSUS
DÉRIVÉS DE 5-[6-[[3-(4,5,6,7-TETRAHYDROPYRAZOLO[4,3-C]PYRIDIN-1-YL)AZÉTIDIN-1-YL]MÉTHYL]MORPHOLIN-4-YL]QUINOLINE-8-CARBONITRILE ET COMPOSÉS SIMILAIRES EN TANT QU'ANTAGONISTES TLR7-9 POUR LE TRAITEMENT DE LUPUS ERYTHEMATOSUS SYSTÈMIQUE

(30) Priority: 09.11.2018 WO PCT/CN2018/114856
(43) Date of publication of application: 15.09.2021
(73) Proprietor: F. Hoffmann-La Roche AG, 4070 Basel (CH)
(72) Inventor: LIU, Haixia, Shanghai, 201203 (CN); SHEN, Hong, Shanghai, 201203 (CN); ZHU, Wei, Shanghai, 201203 (CN); DEY, Fabian, 4070 Basel (CH); WANG, Xiaoqing, Shanghai, 201203 (CN)
(74) Representative: Belkacemi, Doria
(86) International application number: PCT/EP2019/080454
(87) International publication number: WO 2020/094749

(56) References cited:
- WO-A1-2017/106607
- US-A1- 2015 105 370

## Description

The present invention relates to organic compounds useful for therapy and/or prophylaxis in a mammal, and in particular to antagonist of TLR7 and/or TLR8 and/or TLR9 useful for treating systemic lupus erythematosus or lupus nephritis.

### FIELD OF THE INVENTION

Autoimmune connective tissue disease (CTD) include prototypical autoimmune syndromes such as Systemic Lupus Erythematosus (SLE), primary Sjögren's syndrome (pSjS), mixed connective tissue disease (MCTD), Dermatomyositis/Polymyositis (DM/PM), Rheumatoid Arthritis (RA), and systemic sclerosis (SSc). With the exception of RA, no really effective and safe therapies are available to patients. SLE represents the prototypical CTD with a prevalence of 20-150 per 100,000 and causes broad inflammation and tissue damage in distinct organs, from commonly observed symptoms in the skin and joints to renal, lung, or heart failure. Traditionally, SLE has been treated with nonspecific anti-inflammatory or immunosuppressive drugs. However, long term usage of immunosuppressive drug, e.g. corticosteroids is only partially effective, and is associated with undesirable toxicity and side effects. Belimumab is the only FDA-approved drug for lupus in the last 50 years, despite its modest and delayed efficacy in only a fraction of SLE patients (Navarra, S. V. et al Lancet 2011, 377, 721.). Other biologics, such as anti-CD20 mAbs, mAbs against or soluble receptors of specific cytokines, have failed in most clinical studies. Thus, novel therapies are required that provide sustained improvement in a greater proportion of patient groups and are safer for chronic use in many autoimmune as well as autoinflammation diseases.

Toll Like Receptors (TLR) are an important family of pattern recognition receptors (PRR) which can initiate broad immune responses in a wide variety of immune cells. As natural host defense sensors, endosomal TLRs 7, 8 and 9 recognize nucleic acids derived from viruses, bacteria; specifically, TLR7/8 and TLR9 recognize single-stranded RNA (ssRNA) and single-stranded CpG-DNA, respectively. However, aberrant nucleic acid sensing of TRL7,8,9 is considered as a key node in a broad of autoimmune and auto-inflammatory diseases (Krieg, A. M. et al. Immunol. Rev. 2007, 220, 251. Jiménez-Dalmaroni, M. J. et al Autoimmun Rev. 2016, 15, 1. Chen, J. Q., et al. Clinical Reviews in Allergy & Immunology 2016, 50, 1.). Anti-RNA and anti-DNA antibodies are well established diagnostic markers of SLE, and these antibodies can deliver both self-RNA and self-DNA to endosomes. While self-RNA complexes can be recognized by TLR7 and TLR8, self-DNA complexes can trigger TLR9 activation. Indeed, defective clearance of self-RNA and self-DNA from blood and/or tissues is evident in SLE (Systemic Lupus Erythematosus) patients. TLR7 and TLR9 have been reported to be upregulated in SLE tissues, and correlate with chronicity and activity of lupus nephritis, respectively. In B cells of SLE patients, TLR7 expression correlates with anti-RNP antibody production, while TLR9 expression with IL-6 and anti-dsDNA antibody levels. Consistently, in lupus mouse models, TLR7 is required for anti-RNA antibodies, and TLR9 is required for anti-nucleosome antibody. On the other hand, overexpression of TLR7 or human TLR8 in mice promotes autoimmunity and autoinflammation. Moreover, activation of TLR8 specifically contributes to inflammatory cytokine secretion of mDC/macrophages, neutrophil NETosis, induction of Th17 cells, and suppression of Treg cells. In addition to the described role of TLR9 in promoting autoantibody production of B cells, activation of TLR9 by self-DNA in pDC also leads to induction of type I IFNs and other inflammatory cytokines. Given these roles of TLR9 in both pDC and B cells, both as key contributors to the pathogenesis of autoimmune diseases, and the extensive presence of self-DNA complexes that could readily activate TLR9 in many patients with autoimmune diseases, it may have extra benefit to further block self-DNA mediated TLR9 pathways on top of inhibition of TLR7 and TLR8 pathways. Taken together, TLR7, 8, and 9 pathways represent new therapeutic targets for the treatment of autoimmune and auto-inflammatory diseases, for which no effective steroid-free and non-cytotoxic oral drugs exist, and inhibition of all these pathways from the very upstream may deliver satisfying therapeutic effects. As such, we invented oral compounds that target and suppress TLR7, TLR8 and TLR9 for the treatment of autoimmune and auto-inflammatory diseases.

### SUMMARY OF THE INVENTION

The present invention relates to novel compounds of formula (I) or (Ia), 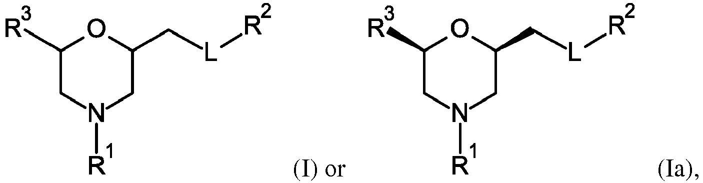 wherein
R¹ is 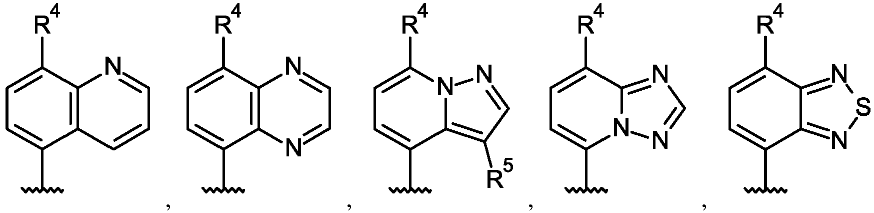 or 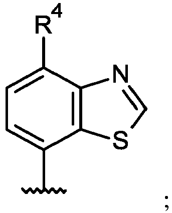 wherein R⁴ is C₁₋₆alkyl, halogen or cyano; R⁵ is halogen;
R² is 4,5,6,7-tetrahydropyrazolo[3,4-c]pyridinyl which is unsubstituted or substituted by C₁₋₆alkyl or haloC₁₋₆alkyl;
   4,5,6,7-tetrahydropyrazolo[4,3-c]pyridinyl which is unsubstituted or substituted one or two times by C₁₋₆alkyl; or
   5,6-dihydro-4H-pyrrolo[3,4-c]pyrazolyl;
R³ is C₁₋₆alkyl;
L is azetidinyl or piperidinyl;
   or a pharmaceutically acceptable salt thereof.

Another object of the present invention is related to novel compounds of formula (I) or (Ia), their manufacture, medicaments based on a compound in accordance with the invention and their production as well as the use of compounds of formula (I) or (Ia) as TLR7 and/or TLR8 and/or TLR9 antagonist, and for the treatment or prophylaxis of systemic lupus erythematosus or lupus nephritis. The compounds of formula (I) or (Ia) show superior TLR7 and/or TLR8 and/or TLR9 antagonism activity. In addition, the compounds of formula (I) or (Ia) also show good cytotoxicity, solubility, hPBMC, human microsome stability and SDPK profiles, as well as low CYP inhibition.

### DETAILED DESCRIPTION OF THE INVENTION

### DEFINITIONS

The term "C₁₋₆alkyl" denotes a saturated, linear or branched chain alkyl group containing 1 to 6, particularly 1 to 4 carbon atoms, for example methyl, ethyl, *n*-propyl, isopropyl, *n*-butyl, isobutyl, *tert*-butyl and the like. Particular "C₁₋₆alkyl" groups are methyl, ethyl and *n*-propyl.

The term "halogen" and "halo" are used interchangeably herein and denote fluoro, chloro, bromo, or iodo.

The term "haloC₁₋₆alkyl" denotes a C₁₋₆alkyl group wherein at least one of the hydrogen atoms of the C₁₋₆alkyl group has been replaced by same or different halogen atoms, particularly fluoro atoms. Examples of haloC₁₋₆alkyl include monofluoro-, difluoro- or trifluoro-methyl, - ethyl or -propyl, for example 3,3,3-trifluoropropyl, 2-fluoroethyl, trifluoroethyl, fluoromethyl, difluoromethyl, difluoroethyl or trifluoromethyl.

The term "LG" denotes a leaving group, which is a molecular fragment that departs with a pair of electrons in heterolytic bond cleavage. Leaving groups can be anions or neutral molecules, but in either case it is crucial that the leaving group be able to stabilize the additional electron density that results from bond heterolysis. Common anionic leaving groups are halides, and sulfonate esters such as OTf, OTs and OMs.

The term "PG" denotes a protecting group, which is introduced into a molecule by chemical modification of a functional group to obtain chemo selectivity in a subsequent chemical reaction. Typical protecting groups are Boc, Cbz and Bn.

The term "diastereomer" denotes a stereoisomer with two or more centers of chirality and whose molecules are not mirror images of one another. Diastereomers have different physical properties, e.g. melting points, boiling points, spectral properties, and reactivities.

The term "pharmaceutically acceptable salts" denotes salts which are not biologically or otherwise undesirable. Pharmaceutically acceptable salts include both acid and base addition salts.

The term "pharmaceutically acceptable acid addition salt" denotes those pharmaceutically acceptable salts formed with inorganic acids such as hydrochloric acid, hydrobromic acid, sulfuric acid, nitric acid, carbonic acid, phosphoric acid, and organic acids selected from aliphatic, cycloaliphatic, aromatic, araliphatic, heterocyclic, carboxylic, and sulfonic classes of organic acids such as formic acid, acetic acid, propionic acid, glycolic acid, gluconic acid, lactic acid, pyruvic acid, oxalic acid, malic acid, maleic acid, maloneic acid, succinic acid, fumaric acid, tartaric acid, citric acid, aspartic acid, ascorbic acid, glutamic acid, anthranilic acid, benzoic acid, cinnamic acid, mandelic acid, embonic acid, phenylacetic acid, methanesulfonic acid, ethanesulfonic acid, *p*-toluenesulfonic acid, and salicyclic acid.

The term "pharmaceutically acceptable base addition salt" denotes those pharmaceutically acceptable salts formed with an organic or inorganic base. Examples of acceptable inorganic bases include sodium, potassium, ammonium, calcium, magnesium, iron, zinc, copper, manganese, and aluminum salts. Salts derived from pharmaceutically acceptable organic nontoxic bases includes salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, 2-diethylaminoethanol, trimethamine, dicyclohexylamine, lysine, arginine, histidine, caffeine, procaine, hydrabamine, choline, betaine, ethylenediamine, glucosamine, methylglucamine, theobromine, purines, piperizine, piperidine, *N*-ethylpiperidine, and polyamine resins.

The term "A pharmaceutically active metabolite" denotes a pharmacologically active product produced through metabolism in the body of a specified compound or salt thereof. After entry into the body, most drugs are substrates for chemical reactions that may change their physical properties and biologic effects. These metabolic conversions, which usually affect the polarity of the compounds of the invention, alter the way in which drugs are distributed in and excreted from the body. However, in some cases, metabolism of a drug is required for therapeutic effect.

The term "therapeutically effective amount" denotes an amount of a compound or molecule of the present invention that, when administered to a subject, (i) treats or prevents the particular disease, condition or disorder, (ii) attenuates, ameliorates or eliminates one or more symptoms of the particular disease, condition, or disorder, or (iii) prevents or delays the onset of one or more symptoms of the particular disease, condition or disorder described herein. The therapeutically effective amount will vary depending on the compound, the disease state being treated, the severity of the disease treated, the age and relative health of the subject, the route and form of administration, the judgement of the attending medical or veterinary practitioner, and other factors.

The term "pharmaceutical composition" denotes a mixture or solution comprising a therapeutically effective amount of an active pharmaceutical ingredient together with pharmaceutically acceptable excipients to be administered to a mammal, e.g., a human in need thereof.

### ANTAGONIST OF TLR7 AND/OR TLR8 AND/OR TLR9

The present invention relates to a compound of formula (I), wherein 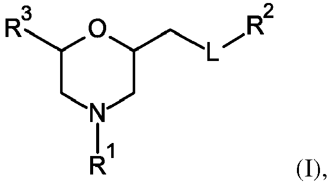
R¹ is 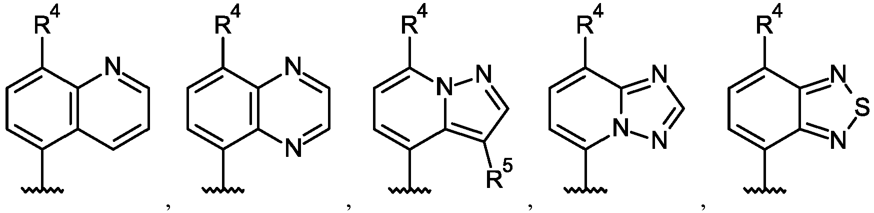 or 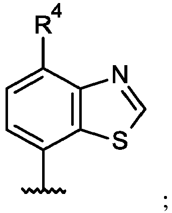 wherein R⁴ is C₁₋₆alkyl, halogen or cyano; R⁵ is halogen;
R² is 4,5,6,7-tetrahydropyrazolo[3,4-c]pyridinyl which is unsubstituted or substituted by C₁₋₆alkyl or haloC₁₋₆alkyl;
   4,5,6,7-tetrahydropyrazolo[4,3-c]pyridinyl which is unsubstituted or substituted one or two times by C₁₋₆alkyl; or
   5,6-dihydro-4H-pyrrolo[3,4-c]pyrazolyl;
R³ is C₁₋₆alkyl;
L is azetidinyl or piperidinyl;
or a pharmaceutically acceptable salt thereof.

Another embodiment of present invention is (ii) a compound of formula (Ia), wherein 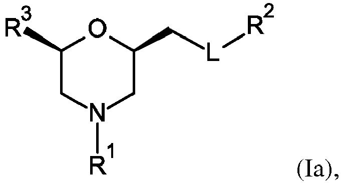
R¹ is 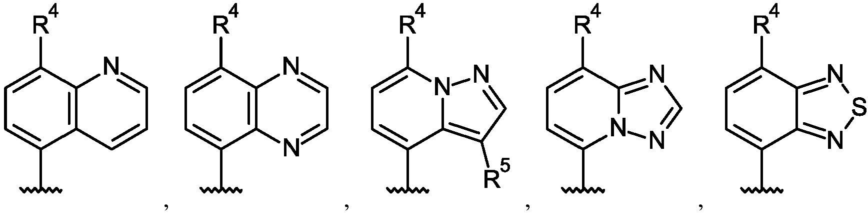 or 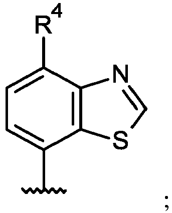 wherein R⁴ is C₁₋₆alkyl, halogen or cyano; R⁵ is halogen;
R² is 4,5,6,7-tetrahydropyrazolo[3,4-c]pyridinyl which is unsubstituted or substituted by C₁₋₆alkyl or haloC₁₋₆alkyl;
   4,5,6,7-tetrahydropyrazolo[4,3-c]pyridinyl which is unsubstituted or substituted one or two times by C₁₋₆alkyl; or
   5,6-dihydro-4H-pyrrolo[3,4-c]pyrazolyl;
R³ is C₁₋₆alkyl;
L is azetidinyl or piperidinyl;
or a pharmaceutically acceptable salt thereof.

A further embodiment of present invention is (iii) a compound of formula (I) or (Ia) according to (i) or (ii), wherein
R¹ is 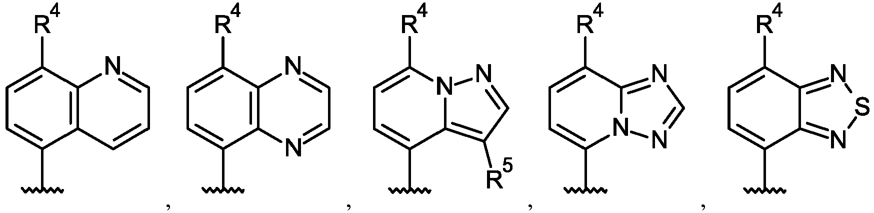 or 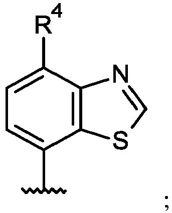 wherein R⁴ is methyl, chloro or cyano; R⁵ is fluoro;
R² is 4,5,6,7-tetrahydropyrazolo[3,4-c]pyridinyl which is unsubstituted or substituted by methyl, ethyl, isopropyl or trifluoromethyl;
   4,5,6,7-tetrahydropyrazolo[4,3-c]pyridinyl which is unsubstituted or substituted one or two times by methyl; or
   5,6-dihydro-4H-pyrrolo[3,4-c]pyrazolyl;
R³ is methyl;
L is azetidinyl or piperidinyl;
   or a pharmaceutically acceptable salt thereof.

A further embodiment of present invention is (iv) a compound of formula (I) or (Ia), or a pharmaceutically acceptable salt thereof, according to any one of (i) to (iii), wherein R¹ is 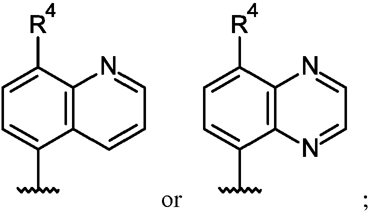 wherein R⁴ is cyano.

A further embodiment of present invention is (v) a compound of formula (I) or (Ia), or a pharmaceutically acceptable salt thereof according to any one of (i) to (iv), wherein L is azetidinyl.

A further embodiment of present invention is (vi) a compound of formula (I) or (Ia), or a pharmaceutically acceptable salt thereof, according to any one of (i) to (v), wherein R² is 4,5,6,7-tetrahydropyrazolo[3,4-c]pyridinyl substituted by C₁₋₆alkyl; or 4,5,6,7-tetrahydropyrazolo[4,3-c]pyridinyl substituted by C₁₋₆alkyl.

A further embodiment of present invention is (vii) a compound of formula (I) or (Ia), or a pharmaceutically acceptable salt thereof, according to any one of (i) to (vi), wherein R² is 4,5,6,7-tetrahydropyrazolo[3,4-c]pyridinyl substituted by methyl; or 4,5,6,7-tetrahydropyrazolo[4,3-c]pyridinyl substituted by methyl.

Another embodiment of present invention is (viii) a compound of formula (I) or (Ia) according to (i) or (ii), wherein
R¹ is 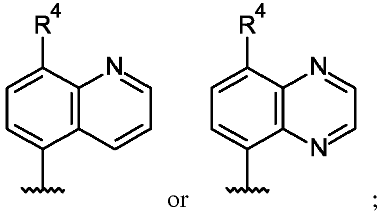 wherein R⁴ is cyano;
R² is 4,5,6,7-tetrahydropyrazolo[3,4-c]pyridinyl substituted by C₁₋₆alkyl; or
   4,5,6,7-tetrahydropyrazolo[4,3-c]pyridinyl substituted by C₁₋₆alkyl;
R³ is C₁₋₆alkyl;
L is azetidinyl;
or a pharmaceutically acceptable salt thereof.

A further embodiment of present invention is (ix) a compound of formula (I) or (Ia) according to (viii), wherein
R¹ is 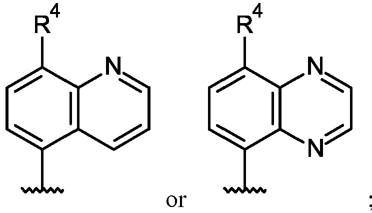 wherein R⁴ is cyano;
R² is 4,5,6,7-tetrahydropyrazolo[3,4-c]pyridinyl substituted by methyl; or
   4,5,6,7-tetrahydropyrazolo[4,3-c]pyridinyl substituted by methyl;
R³ is methyl;
L is azetidinyl;
or a pharmaceutically acceptable salt thereof.

Another embodiment of present invention is that (x) a compound of formula (I) or (Ia) selected from the following:
5-[(2*R*,6*S*)-2-methyl-6-[[3-(4,5,6,7-tetrahydropyrazolo[4,3-c]pyridin-1-yl)azetidin-1-yl] methyl] morpholin-4-yl] quinoline-8-carbonitrile;
5-[(2*R*,6*S*)-2-methyl-6-[[3-(4,5,6,7-tetrahydropyrazolo[4,3-c]pyridin-2-yl)azetidin-1-yl] methyl] morpholin-4-yl] quinoline-8-carbonitrile;
5-[(2*R*,6*S*)-2-methyl-6-[[3-(4,5,6,7-tetrahydropyrazolo[3,4-c]pyridin-1-yl)azetidin-1-yl] methyl] morpholin-4-yl] quinoline-8-carbonitrile;
5-[(2*R*,6*S*)-2-methyl-6-[[3-(4,5,6,7-tetrahydropyrazolo[3,4-c]pyridin-2-yl)azetidin-1-yl] methyl] morpholin-4-yl] quinoline-8-carbonitrile;
5-[(2*R*,6*S*)-2-methyl-6-[[3-(6-methyl-5,7-dihydro-4*H*-pyrazolo[3,4-c]pyridin-2-yl)azetidin-1-yl]methyl]morpholin-4-yl]quinoline-8-carbonitrile;
5-[(2*S*,6*R*)-2-[[3-(6-ethyl-5,7-dihydro-4*H*-pyrazolo[3,4-c]pyridin-1-yl)azetidin-1-yl]methyl]-6-methyl-morpholin-4-yl]quinoline-8-carbonitrile;
5-[(2*S*,6*R*)-2-[[3-(6-ethyl-5,7-dihydro-4*H*-pyrazolo[3,4-c]pyridin-2-yl)azetidin-1-yl]methyl]-6-methyl-morpholin-4-yl]quinoline-8-carbonitrile;
5-[(2*S*,6*R*)-2-[[3-(6-isopropyl-5,7-dihydro-4*H*-pyrazolo[3,4-c]pyridin-1-yl)azetidin-1-yl]methyl]-6-methyl-morpholin-4-yl]quinoline-8-carbonitrile;
5-[(2*S*,6*R*)-2-[[3-(6-isopropyl-5,7-dihydro-4*H*-pyrazolo[3,4-c]pyridin-2-yl)azetidin-1-yl]methyl]-6-methyl-morpholin-4-yl]quinoline-8-carbonitrile;
5-[(2*S*,6*R*)-2-[[3-(5,6-dihydro-4*H*-pyrrolo[3,4-c]pyrazol-1-yl)azetidin-1-yl]methyl]-6-methyl-morpholin-4-yl]quinoline-8-carbonitrile;
5-[(2*S*,6*R*)-2-[[3-(5,6-dihydro-4*H*-pyrrolo[3,4-c]pyrazol-2-yl)azetidin-1-yl]methyl]-6-methyl-morpholin-4-yl]quinoline-8-carbonitrile;
5-[(2*R*,6*S*)-2-methyl-6-[[3-(3-methyl-4,5,6,7-tetrahydropyrazolo[4,3-c]pyridin-1-yl)azetidin-1-yl]methyl]morpholin-4-yl]quinoline-8-carbonitrile;
5-[(2*R*,6*S*)-2-methyl-6-[[3-(3-methyl-4,5,6,7-tetrahydropyrazolo[4,3-c]pyridin-2-yl)azetidin-1-yl]methyl]morpholin-4-yl]quinoline-8-carbonitrile;
5-[(2*S*,6*R*)-2-[[3-(3,5-dimethyl-6,7-dihydro-4*H*-pyrazolo[4,3-c]pyridin-1-yl)azetidin-1-yl]methyl]-6-methyl-morpholin-4-yl]quinoline-8-carbonitrile;
5-[(2*S*,6*R*)-2-[[3-(3,5-dimethyl-6,7-dihydro-4*H*-pyrazolo[4,3-c]pyridin-2-yl)azetidin-1-yl]methyl]-6-methyl-morpholin-4-yl]quinoline-8-carbonitrile;
5-[(2*R*,6*S*)-2-methyl-6-[[3-[(6*R*)-6-methyl-4,5,6,7-tetrahydropyrazolo[4,3-c]pyridin-1-yl] azetidin-1-yl]methyl] morpholin-4-yl] quinoline-8-carbonitrile;
5-[(2*R*,6*S*)-2-methyl-6-[[3-[(6*R*)-6-methyl-4,5,6,7-tetrahydropyrazolo[4,3-c]pyridin-2-yl] azetidin-1-yl]methyl] morpholin-4-yl] quinoline-8-carbonitrile;
5-[(2*R*,6*S*)-2-methyl-6-[[3-[(6*S*)-6-methyl-4,5,6,7-tetrahydropyrazolo[4,3-c]pyridin-1-yl] azetidin-1-yl]methyl] morpholin-4-yl] quinoline-8-carbonitrile;
5-[(2*R*,6S)-2-methyl-6-[[3-[(6*S*)-6-methyl-4,5,6,7-tetrahydropyrazolo[4,3-c]pyridin-2-yl] azetidin-1-yl]methyl] morpholin-4-yl] quinoline-8-carbonitrile;
5-[(2*R*,6*S*)-2-methyl-6-[[3-[(4*R*)-4-methyl-4,5,6,7-tetrahydropyrazolo[4,3-c]pyridin-1-yl] azetidin-1-yl]methyl] morpholin-4-yl] quinoline-8-carbonitrile;
5-[(2*R*,6*S*)-2-methyl-6-[[3-[(4*R*)-4-methyl-4,5,6,7-tetrahydropyrazolo[4,3-c]pyridin-2-yl] azetidin-1-yl]methyl] morpholin-4-yl] quinoline-8-carbonitrile;
5-[(2*R*,6*S*)-2-methyl-6-[[3-[(4*S*)-4-methyl-4,5,6,7-tetrahydropyrazolo[4,3-c]pyridin-1-yl] azetidin-1-yl]methyl] morpholin-4-yl] quinoline-8-carbonitrile;
5-[(2*R*,6*S*)-2-methyl-6-[[3-[(4*S*)-4-methyl-4,5,6,7-tetrahydropyrazolo[4,3-c]pyridin-2-yl] azetidin-1-yl]methyl] morpholin-4-yl] quinoline-8-carbonitrile;
5-[(2*S*,6*R*)-2-[[3-(7,7-dimethyl-5,6-dihydro-4*H*-pyrazolo[4,3-c]pyridin-2-yl)azetidin-1-yl]methyl]-6-methyl-morpholin-4-yl]quinoline-8-carbonitrile;
5-[(2*R*,6*S*)-2-methyl-6-[[3-[(7*R*)-7-methyl-4,5,6,7-tetrahydropyrazolo[4,3-c]pyridin-2-yl] azetidin-1-yl]methyl] morpholin-4-yl] quinoline-8-carbonitrile;
5-[(2*R*,6*S*)-2-methyl-6-[[3-[(7*S*)-7-methyl-4,5,6,7-tetrahydropyrazolo[4,3-c]pyridin-2-yl] azetidin-1-yl]methyl] morpholin-4-yl] quinoline-8-carbonitrile;
5-[(2*R*,6*S*)-2-methyl-6-[[3-[(5*S*)-5-methyl-4,5,6,7-tetrahydropyrazolo[3,4-c]pyridin-1-yl] azetidin-1-yl]methyl] morpholin-4-yl] quinoline-8-carbonitrile;
5-[(2*R*,6*S*)-2-methyl-6-[[3-[(5*R*)-5-methyl-4,5,6,7-tetrahydropyrazolo[3,4-c]pyridin-1-yl] azetidin-1-yl]methyl] morpholin-4-yl] quinoline-8-carbonitrile;
5-[(2*R*,6*S*)-2-methyl-6-[[3-[(5*S*)-5-methyl-4,5,6,7-tetrahydropyrazolo[3,4-c]pyridin-2-yl] azetidin-1-yl]methyl] morpholin-4-yl] quinoline-8-carbonitrile;
5-[(2*R*,6*S*)-2-methyl-6-[[3-[(5*R*)-5-methyl-4,5,6,7-tetrahydropyrazolo[3,4-c]pyridin-2-yl] azetidin-1-yl]methyl] morpholin-4-yl] quinoline-8-carbonitrile;
5-[(2*R*,6*S*)-2-methyl-6-[[3-[(7*R*)-7-methyl-4,5,6,7-tetrahydropyrazolo[3,4-c]pyridin-1-yl] azetidin-1-yl]methyl] morpholin-4-yl] quinoline-8-carbonitrile;
5-[(2*R*,6*S*)-2-methyl-6-[[3-[(7*S*)-7-methyl-4,5,6,7-tetrahydropyrazolo[3,4-c]pyridin-1-yl] azetidin-1-yl]methyl] morpholin-4-yl] quinoline-8-carbonitrile;
5-[(2*R*,6*S*)-2-methyl-6-[[3-[(7*R*)-7-methyl-4,5,6,7-tetrahydropyrazolo[3,4-c]pyridin-2-yl] azetidin-1-yl]methyl] morpholin-4-yl] quinoline-8-carbonitrile;
5-[(2*R*,6*S*)-2-methyl-6-[[3-[(7*S*)-7-methyl-4,5,6,7-tetrahydropyrazolo[3,4-c]pyridin-2-yl] azetidin-1-yl]methyl] morpholin-4-yl] quinoline-8-carbonitrile;
5-[(2*R*,6*S*)-2-methyl-6-[[4-(4,5,6,7-tetrahydropyrazolo[4,3-c]pyridin-1-yl)-1-piperidyl]methyl]morpholin-4-yl]quinoline-8-carbonitrile;
5-[(2*R*,6*S*)-2-methyl-6-[[4-(4,5,6,7-tetrahydropyrazolo[4,3-c]pyridin-2-yl)-1-piperidyl]methyl]morpholin-4-yl]quinoline-8-carbonitrile;
5-[(2*R*,6*S*)-2-methyl-6-[[3-[3-(trifluoromethyl)-4,5,6,7-tetrahydropyrazolo[3,4-c]pyridin-1-yl] azetidin-1-yl]methyl] morpholin-4-yl] quinoline-8-carbonitrile;
5-[(2*R*,6*S*)-2-methyl-6-[[3-[3-(trifluoromethyl)-4,5,6,7-tetrahydropyrazolo[3,4-c]pyridin-2-yl] azetidin-1 -yl] methyl] morpholin-4-yl] quinoline- 8-carbonitrile;
5-[(2*R*,6*S*)-2-methyl-6-[[3-(3-methyl-4,5,6,7-tetrahydropyrazolo[3,4-c]pyridin-1-yl)azetidin-1-yl]methyl]morpholin-4-yl]quinoline-8-carbonitrile; and
5-[(2*R*,6*S*)-2-methyl-6-[[3-(3-methyl-4,5,6,7-tetrahydropyrazolo[3,4-c]pyridin-2-yl)azetidin-1-yl]methyl]morpholin-4-yl]quinoline-8-carbonitrile;
or a pharmaceutically acceptable salt, enantiomer or diastereomer thereof.

A number of compounds used as reference herein were disclosed in patent US20150105370 showing TLR7 and TLR9 potency data summarized in table 1 (TLR8 data is not available). Compounds in Table 1 are all characterized with an aromatic ring at the terminal position (phenyl or pyridinyl). However, according to the potency data disclosed, only some of the compounds in Table 1 showed good TLR7 potency, and all of which were lack of TLR9 potency. More examples disclosed in US20150105370 with same structural characteristics confirmed such trend, which suggests the terminal aryl/heteroaryl ring is not favorable for TLR9 activity.

Meanwhile, more analogues of the compounds disclosed in US20150105370, such as compound R1, compound R2 which bear some substituents on the terminal aryl ring, were synthesized to confirm the SAR (structure-activity-relationship). But according to the potency data shown in Table 2, the substituents on the terminal aryl ring may not necessarily improve the potency of TLR9. Therefore, the skill of the art shall not obtain any incitation from the information disclosed in US20150105370 to further optimize such chemical structures.

Surprisingly, the compounds of this invention significantly improved TLR9 potency (>8 folds compared to ER-888286) while keeping excellent TLR7 and TLR8 potency. In another embodiment, hERG profile and safety ratio were greatly improved as compared with reference compounds from US20150105370 and reference compounds R1 and R2 synthesized herein (see table 3). The compounds of formula (I) or (Ia) also showed good hPBMC, cytotoxicity, solubility, human microsome stability and SDPK profiles, as well as low CYP inhibition.

**Table 1. TLR7 and TLR9 potency of compounds disclosed in US20150105370**

| **Compound** | **Structure** | **HEK/hTLR7 IC50 (µM)** | **HEK/hTLR9 IC50 (µM)** |
|---|---|---|---|
| ER-887258 | 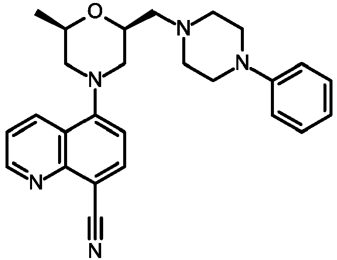 | 0.0852 | >2.0 |
| ER-888285 | 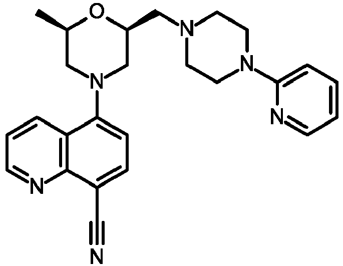 | 0.120 | >2.0 |
| ER-888286 | 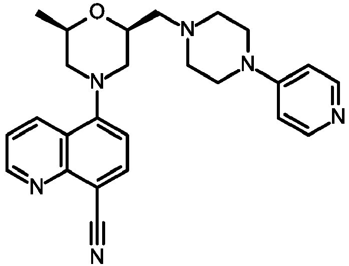 | 1.370 | >2.0 |
| ER-894544 | 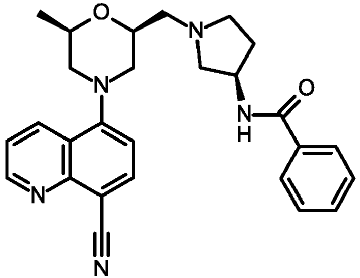 | 0.043 | >6.2 |
| ER-894160 | 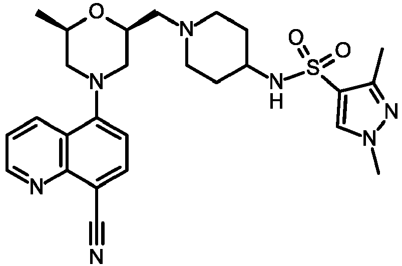 | 0.1990 | >10.0 |
| ER-894155 | 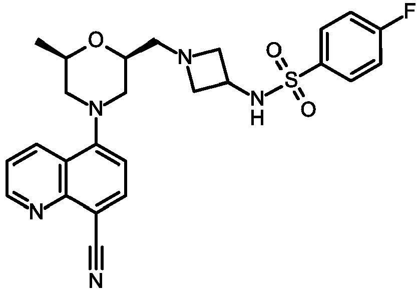 | 0.2820 | >10.0 |

### SYNTHESIS

The compounds of the present invention can be prepared by any conventional means. Suitable processes for synthesizing these compounds as well as their starting materials are provided in the schemes below and in the examples. All substituents, in particular, R¹ to R⁴ are as defined above unless otherwise indicated. Furthermore, and unless explicitly otherwise stated, all reactions, reaction conditions, abbreviations and symbols have the meanings well known to a person of ordinary skill in organic chemistry.

General synthetic routes for preparing the compound of formula (I) or (Ia) are shown below. 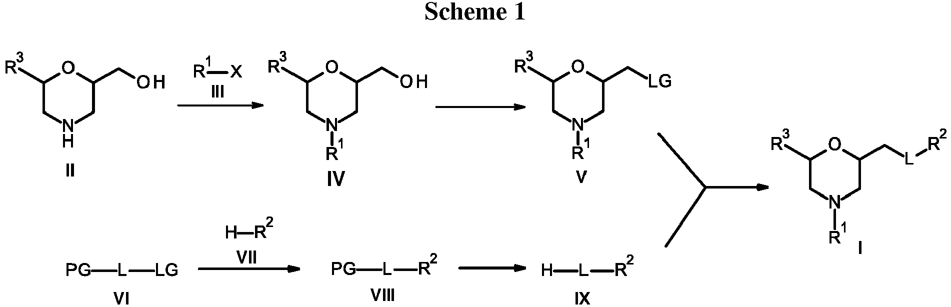 wherein LG is a leaving group, such as halogen, OTf, OTs and OMs; PG is a protecting group, such as Boc and Cbz.

The coupling of compound of formula (II) with R¹-X can be achieved by direct coupling at elevated temperature in the presence of a base, such as DIPEA or K₂CO₃, or under Buchwald-Hartwig amination conditions (ref: Acc. Chem. Res. 1998, 31, 805-818; Chem. Rev. 2016, 116, 12564-12649; Topics in Current Chemistry, 2002, 219, 131-209; and references cited therein) with a catalyst, such as RuPhos Pd G2, and a base, such as Cs₂CO₃ or K₂CO₃, to provide compound of formula (IV). Subsequently the hydroxy group of compound of formula (IV) is converted to a leaving group, such as OTf, OTs, or OMs, under basic condition, such as DIPEA, TEA, K₂CO₃ or 2,6-dimethylpyridine, with Tf₂O, TsCl or MsCl. The coupling of compound of formula (VI) with (VII) can be achieved by direct coupling at high temperature in the presence of a base, such as NaH or Cs₂CO₃ to provide compound of formula (VIII). The protecting group of compound of formula (VIII) can be removed at high temperature or under acidic condition, such as TFA, or under hydrogenation condition with a catalyst, such as Pd/C or Pd(OH)₂/C. Compound of formula (IX) is further coupled with compound of formula (V) in the presence of a base, such as K₂CO₃, DIPEA or Cs₂CO₃, to afford compound of formula (I). In some embodiment, the coupling of compound of formula (IX) and (V) may give a product containing a protecting group, e.g. Boc or Cbz, originated from (IX), which will be removed before affording the final compound of formula (I). And in another embodiment, the compound of formula (I) or (Ia) with a terminal secondary amine is further introduced with an alkyl group, such as methyl, ethyl or isopropyl, through reductive amination reaction under reductive condition, such as NaBH₃CN or NaBH(OAc)₃ with formaldehyde, acetaldehyde or acetone to afford the final compound of formula (I) or (Ia).

Compounds of this invention can be obtained as mixtures of diastereomers or enantiomers, which can be separated by methods well known in the art, e.g. (chiral) HPLC or SFC.

This invention also relates to a process for the preparation of a compound of formula (I) or (Ia) comprising the following step:
a) the coupling of compound of formula (V), 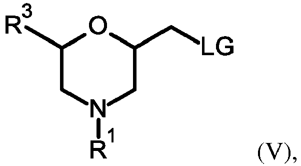 with compound of formula (IX), 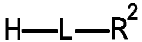 (IX), in the presence of a base;
   wherein
   in step a), the base can be, for example, K₂CO₃, DIPEA or Cs₂CO₃.

A compound of formula (I) or (Ia) when manufactured according to the above process is also an object of the invention.

### INDICATIONS AND METHODS OF TREATMENT

The present invention provides compounds that can be used as TLR7 and/or TLR8 and/or TLR9 antagonist, which inhibits pathway activation through TLR7 and/or TLR8 and/or TLR9 as well as respective downstream biological events including, but not limited to, innate and adaptive immune responses mediated through the production of all types of cytokines and all forms of auto-antibodies. Accordingly, the compounds of the invention are useful for blocking TLR7 and/or TLR8 and/or TLR9 in all types of cells that express such receptor(s) including, but not limited to, plasmacytoid dendritic cell, B cell, T cell, macrophage, monocyte, neutrophil, keratinocyte, epithelial cell. As such, the compounds can be used as a therapeutic or prophylactic agent for systemic lupus erythematosus and lupus nephritis.

The present invention provides methods for treatment or prophylaxis of systemic lupus erythematosus and lupus nephritis in a patient in need thereof.

Another embodiment includes a method of treating or preventing systemic lupus erythematosus and lupus nephritis in a mammal in need of such treatment, wherein the method comprises administering to said mammal a therapeutically effective amount of a compound of formula (I), a stereoisomer, tautomer, or pharmaceutically acceptable salt thereof.

### EXAMPLES

The invention will be more fully understood by reference to the following examples.

### ABBREVIATIONS

The invention will be more fully understood by reference to the following examples. They should not, however, be construed as limiting the scope of the invention.

Abbreviations used herein are as follows:
- ACN:: acetonitrile
- Boc₂O:: *di-tert* butyl dicarbonate
- CbzCl:: benzylchloroformate
- DCM:: dichloromethane
- DEA:: diethylamine
- DIPEA:: *N*,*N*-diisopropylethylamine
- DMA:: dimethylacetamide
- DMF:: *N*,*N*-dimethylformamide
- DMFDMA:: *N*,*N*-dimethylformamide dimethyl acetal
- EtOAc or EA:: ethyl acetate
- FA:: formic acid
- HLM: human liver microsome
- IC₅₀:: half inhibition concentration
- LCMS: liquid chromatography-mass spectrometry
- MS:: mass spectrometry
- PE:: petroleum ether
- prep-HPLC:: preparative high performance liquid chromatography
- rt:: room temperature
- RuPhos Pd G2:: chloro(2-dicyclohexylphosphino-2',6'-diisopropoxy-1,1'-biphenyl)[2-(2'-amino-1,1'-biphenyl)]palladium(II) 2nd generation
- SFC:: supercritical fluid chromatography
- TEA:: trimethylamine
- TFA:: trifluoroacetic acid
- Tf₂O:: trifluoromethanesulfonic anhydride
- THF:: tetrahydrofuran
- v/v:: volume ratio

### GENERAL EXPERIMENTAL CONDITIONS

Intermediates and final compounds were purified by flash chromatography using one of the following instruments: i) Biotage SP1 system and the Quad 12/25 Cartridge module. ii) ISCO combi-flash chromatography instrument. Silica gel brand and pore size: i) KP-SIL 60 Å, particle size: 40-60 µm; ii) CAS registry NO: Silica Gel: 63231-67-4, particle size: 47-60 micron silica gel; iii) ZCX from Qingdao Haiyang Chemical Co., Ltd, pore: 200-300 or 300-400.

Intermediates and final compounds were purified by preparative HPLC on reversed phase column using XBridge^{™} Prep-C18 (5 µm, OBDTM 30 × 100 mm) column, SunFire^{™} Prep-C18 (5 µm, OBD^{™} 30 × 100 mm) column, Phenomenex Synergi-C18 (10 µm, 25 × 150 mm) or Phenomenex Gemini-C18 (10 µm, 25 × 150 mm). Waters AutoP purification System (Sample Manager 2767, Pump 2525, Detector: Micromass ZQ and UV 2487, solvent system: acetonitrile and 0.1% ammonium hydroxide in water; acetonitrile and 0.1% FA in water or acetonitrile and 0.1% TFA in water). Or Gilson-281 purification System (Pump 322, Detector: UV 156, solvent system: acetonitrile and 0.05% ammonium hydroxide in water; acetonitrile and 0.225% FA in water; acetonitrile and 0.05% HCl in water; acetonitrile and 0.075% TFA in water; or acetonitrile and water).

For SFC chiral separation, intermediates were separated by chiral column (Daicel chiralpak IC, 5 µm, 30 × 250 mm), AS (10 µm, 30 × 250 mm) or AD (10 µm, 30 × 250 mm) using Mettler Toledo Multigram III system SFC, Waters 80Q preparative SFC or Thar 80 preparative SFC, solvent system: CO₂ and IPA (0.5% TEA in IPA) or CO₂ and MeOH (0.1% NH₃·H₂O in MeOH), back pressure 100bar, detection UV@ 254 or 220 nm.

LC/MS spectra of compounds were obtained using a LC/MS (Waters^{™} Alliance 2795-Micromass ZQ, Shimadzu Alliance 2020-Micromass ZQ or Agilent Alliance 6110-Micromass ZQ), LC/MS conditions were as follows (running time 3 or 1.5 mins):
Acidic condition I: A: 0.1% TFA in H₂O; B: 0.1% TFA in acetonitrile;
Acidic condition II: A: 0.0375% TFA in H₂O; B: 0.01875% TFA in acetonitrile;
Basic condition I: A: 0.1% NH₃·H₂O in H₂O; B: acetonitrile;
Basic condition II: A: 0.025% NH₃·H₂O in H₂O; B: acetonitrile;
Neutral condition: A: H₂O; B: acetonitrile.

Mass spectra (MS): generally only ions which indicate the parent mass are reported, and unless otherwise stated the mass ion quoted is the positive mass ion (MH)⁺.

NMR Spectra were obtained using Bruker Avance 400 MHz.

The microwave assisted reactions were carried out in a Biotage Initiator Sixty microwave synthesizer. All reactions involving air-sensitive reagents were performed under an argon or nitrogen atmosphere. Reagents were used as received from commercial suppliers without further purification unless otherwise noted.

### PREPARATIVE EXAMPLES

The following examples are intended to illustrate the meaning of the present invention but should by no means represent a limitation within the meaning of the present invention:

### Intermediate A

### [(2R,6R)-4-(8-cyano-5-quinolyl)-6-methyl-morpholin-2-yl]methyl trifluoromethanesulfonate

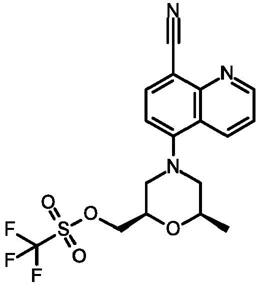

The title compound was prepared according to the following scheme:

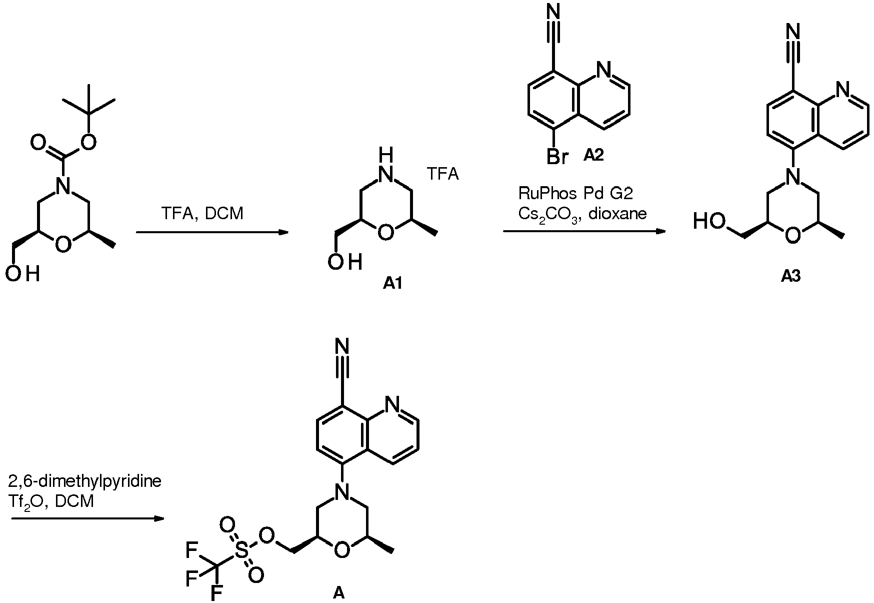

### Step 1: preparation of [(2R,6R)-6-methylmorpholin-2-yl]methanol;2,2,2-trifluoroacetic acid (compound A1)

To a solution of tert-butyl (2*R*,6*R*)-2-(hydroxymethyl)-6-methylmorpholine-4-carboxylate (CAS: 1700609-48-8, Vendor: WuXi Apptec, 1.35 g, 5.84 mmol) in DCM (10 mL) was added 2,2,2-trifluoroacetic acid (2.66 g, 23.30 mmol). The reaction mixture was stirred at rt for 3 hrs, then concentrated *in vacuo* to give the crude product compound **A1** (1.43 g) which was used in next step directly. MS: calc'd 132 (MH⁺), measured 132 (MH⁺).

### Step 2: preparation of 5-[(2R,6R)-2-(hydroxymethyl)-6-methyl-morpholin-4-yl]quinoline-8-carbonitrile (compound A3)

A mixture of 5-bromoquinoline-8-carbonitrile (compound **A2,** CAS: 507476-70-2, Vendor: BePharm, 1.50 g, 6.42 mmol), [(2R,6R)-6-methylmorpholin-2-yl]methanol;2,2,2-trifluoroacetic acid (compound **A1**, 1.43 g, 5.83 mmol), RuPhos Pd G2 (136 mg, 175 µmol) and Cs₂CO₃ (5.70 g, 17.50 mmol) in 1,4-dioxane (10 mL) was heated to 90 °C overnight under N₂. After being cooled down, the solid was filtered off and washed with EA (10 mL) twice. The combined organics was concentrated, the residue was purified by flash column eluting with a gradient of EA/PE (0 to 100%) to afford compound **A3** (0.71 g) as a light yellow solid. MS: calc'd 284 (MH⁺), measured 284 (MH⁺).

### Step 3: preparation of [(2R,6R)-4-(8-cyano-5-quinolyl)-6-methyl-morpholin-2-yl]methyl trifluoromethanesulfonate (Intermediate A)

To a flask was added 5-[(2R,6R)-2-(hydroxymethyl)-6-methyl-morpholin-4-yl]quinoline-8-carbonitrile (compound **A3**, 0.71 g, 2.50 mmol), DCM (10 mL) and 2,6-dimethylpyridine (0.54 g, 577 µL, 5.00 mmol). Then the reaction mixture was cooled with ice bath and trifluoromethanesulfonic anhydride (1.06 g, 634 µL, 3.75 mmol) was added dropwise. After being stirred for 2 hrs, the mixture was concentrated and purified by flash column (EA/PE=0 to 40%) to give product **Intermediate A** (0.72 g) as a yellow solid. MS: calc'd 416 (MH⁺), measured 416 (MH⁺).

### Intermediate B

### [(2R,6R)-4-(8-cyanoquinoxalin-5-yl)-6-methyl-morpholin-2-yl]methyl trifluoromethanesulfonate

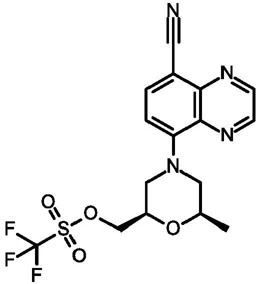

The title compound was prepared in analogy to the preparation of **Intermediate A** by using 8-bromoquinoxaline-5-carbonitrile (Synthesis refers to US 20170174653 A1) instead of 5-bromoquinoline-8-carbonitrile (compound **A2**). **Intermediate B** (825 mg) was obtained as an off-white solid. MS: calc'd 417 (MH⁺), measured 417 (MH⁺).

### Intermediate C

### tert-butyl 5-methyl-2,4,5,7-tetrahydropyrazolo[3,4-c]pyridine-6-carboxylate

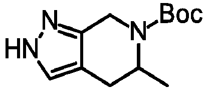

The title compound was prepared according to the following scheme:

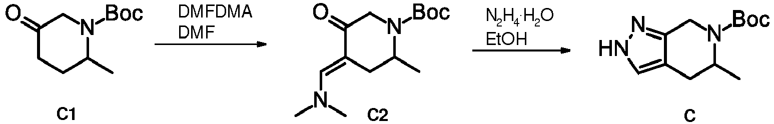

### Step 1: preparation of tert-butyl 4-(dimethylaminomethylene)-2-methyl-5-oxo-piperidine-1-carboxylate (compound C2)

To a solution of *tert*-butyl 2-methyl-5-oxo-piperidine-1-carboxylate (compound **C1**, CAS: 362704-66-3, Vendor: Pharmablock, 5.00 g, 23.44 mmol) in DMF (30 mL) was added *N,N-*dimethylformamide dimethyl acetal (5.58 g, 46.88 mmol). After being heated at 90 °C for 18 hrs, the mixture was cooled down and concentrated to give compound **C2** (6.29 g) as a tangerine liquid which was used in next step without further purification. MS: calc'd 269 (MH⁺), measured 269 (MH⁺).

### Step 2: preparation of tert-butyl 5-methyl-2,4,5,7-tetrahydropyrazolo[3,4-c]pyridine-6-carboxylate (Intermediate C)

To a solution of *tert*-butyl 4-(dimethylaminomethylene)-2-methyl-5-oxo-piperidine-1-carboxylate (compound **C2,** 6.29 g, 23.44 mmol) in ethanol (30 mL) was added hydrazine hydrate (1.76 g, 35.16 mmol). After being stirred at 50 °C for 2 hrs, the mixture was cooled down and diluted with water (100 mL). The mixture was extracted by EA (100 mL) three times. The combined organic phase was dried over Na₂SO₄, filtered and concentrated. The residue was purified by flash column (EA/PE=0 to 60%) to give Intermediate **C** (1.80 g) as a yellow oil. MS: calc'd 238 (MH⁺), measured 238 (MH⁺).

### Intermediate D

### tert-butyl 7-methyl-2,4,5,7-tetrahydropyrazolo[3,4-c]pyridine-6-carboxylate

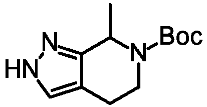

The title compound was prepared in analogy to the preparation of **Intermediate C** by using *tert*-butyl 2-methyl-3-oxo-piperidine-1-carboxylate (CAS: 741737-30-4, Vendor: Bide) instead of *tert*-butyl 2-methyl-5-oxo-piperidine-1-carboxylate (compound **C1**). **Intermediate D** (2.70 g) was obtained as a yellow oil. MS: calc'd 238 (MH⁺), measured 238 (MH⁺).

### Intermediate E

### tert-butyl 7-methyl-2,4,6,7-tetrahydropyrazolo[4,3-c]pyridine-5-carboxylate

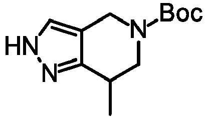

The title compound was prepared in analogy to the preparation of **Intermediate C** by using *tert*-butyl 3-methyl-4-oxo-piperidine-1-carboxylate (CAS: 181269-69-2, Vendor: Pharmablock) instead of *tert*-butyl 2-methyl-5-oxo-piperidine-1-carboxylate (compound **C1**). **Intermediate E** (3.50 g) was obtained as a yellow oil. MS: calc'd 238 (MH⁺), measured 238 (MH⁺).

### Intermediate F

### tert-butyl (6R)-6-methyl-2,4,6,7-tetrahydropyrazolo[4,3-c]pyridine-5-carboxylate

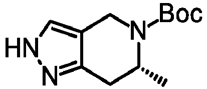

### Intermediate G

### tert-butyl (4R)-4-methyl-2,4,6,7-tetrahydropyrazolo[4,3-c]pyridine-5-carboxylate

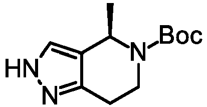

**Intermediate F** and **G** were prepared in analogy to the preparation of **Intermediate C** by using *tert*-butyl (2*R*)-2-methyl-4-oxo-piperidine-1-carboxylate (CAS: 790667-43-5, Vendor: Pharmablock) instead of *tert*-butyl 2-methyl-5-oxo-piperidine-1-carboxylate (compound **C1**). **Intermediate F** (slower eluting) and **Intermediate** G (faster eluting) were separated by chiral SFC (Gradient: 20% MeOH in CO₂, Column: ChiralPak AD, 300×50mm I.D., 10µm). **Intermediate F** (700 mg) was obtained as a yellow oil. MS: calc'd 238 (MH⁺), measured 238 (MH⁺). **Intermediate G** (580 mg) was obtained as a yellow oil. MS: calc'd 238 (MH⁺), measured 238 (MH⁺).

### Intermediate H

### tert-butyl (6S)-6-methyl-2,4,6,7-tetrahydropyrazolo[4,3-c]pyridine-5-carboxylate

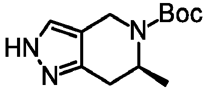

### Intermediate I

### tert-butyl (4S)-4-methyl-2,4,6,7-tetrahydropyrazolo[4,3-c]pyridine-5-carboxylate

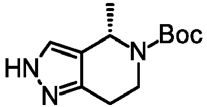

**Intermediate H and I** were prepared in analogy to the preparation of **Intermediate C** by using *tert*-butyl (2S)-2-methyl-4-oxo-piperidine-1-carboxylate (CAS: 790667-49-1, Vendor: Pharmablock) instead of *tert*-butyl 2-methyl-5-oxo-piperidine-1-carboxylate (compound **C1**). **Intermediate H** (slower eluting) and **Intermediate I** (faster eluting) were separated by chiral SFC (Gradient: 5-40% MeOH (0.05% DEA) in CO₂, Column: ChiralPak AY, 150×4.6mm I.D., 3µm). **Intermediate H** (430 mg) was obtained as a yellow oil. MS: calc'd 238 (MH⁺), measured 238 (MH⁺). **Intermediate I (450** mg) was obtained as a yellow oil. MS: calc'd 238 (MH⁺), measured 238 (MH⁺).

### Intermediate J

### tert-butyl 3-methyl-2,4,5,7-tetrahydropyrazolo[3,4-c]pyridine-6-carboxylate

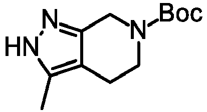

The title compound was prepared according to the following scheme:

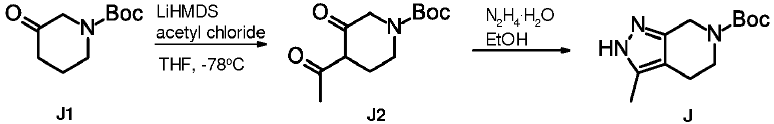

### Step 1: preparation of tert-butyl 4-acetyl-3-oxo-piperidine-1-carboxylate (compound J2)

To a solution of *tert*-butyl 3-oxopiperidine-1-carboxylate (compound **J1**, CAS: 98977-36-7, Vendor: Accela, 8.00 g, 40.14 mmol) in THF (30 mL) in -78°C was added LiHMDS (40 mL, 1.2M in THF, 48.00 mmol). After being stirred at -78 °C for 0.5 hrs, acetyl chloride (4.73 g, 60.20 mmol) was added and the mixture was slowly warmed to rt followed by stirring at rt for 2 hrs. After being quenched by addition of saturated NH₄Cl solution (30 mL), the mixture was extracted by EA (30 mL) three times. The combined organic phase was dried over Na₂SO₄, filtered and concentrated to give Intermediate **J2** (9.65 g) as a tangerine oil which was used in next step without further purification. MS: calc'd 242 (MH⁺), measured 142 (MH⁺-Boc).

### Step 2: preparation of tert-butyl 3-methyl-2,4,5,7-tetrahydropyrazolo[3,4-c]pyridine-6-carboxylate (Intermediate J)

To a solution of *tert*-butyl 4-acetyl-3-oxo-piperidine-1-carboxylate (compound **J2,** 9.65 g, 40.14 mmol) in ethanol (30 mL) was added hydrazine hydrate (3.00 g, 60.00 mmol). After being stirred at 50 °C for 2 hrs, the mixture was cooled down and diluted with water (100 mL). The mixture was extracted by EA (100 mL) three times. The combined organic phase was dried over Na₂SO₄, filtered and concentrated. The residue was purified by flash column (EA/PE=0 to 60%) and then chiral SFC to give Intermediate **J** (2.00 g) as a yellow oil. MS: calc'd 238 (MH⁺), measured 238 (MH⁺).

### Intermediate K

### tert-butyl 3-methyl-2,4,6,7-tetrahydropyrazolo[4,3-c]pyridine-5-carboxylate

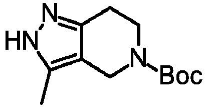

The title compound was prepared according to the following scheme:

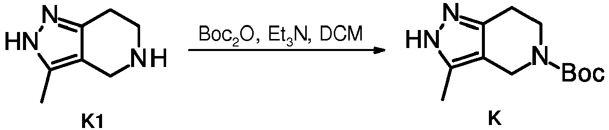

### Step 1: preparation of tert-butyl 3-methyl-2,4,6,7-tetrahydropyrazolo[4,3-c]pyridine-5-carboxylate

To a flask containing 3-methyl-4,5,6,7-tetrahydro-2*H*-pyrazolo[4,3-c]pyridine (compound **K1**, CAS: 740061-36-3, Vendor: BePharm, 300 mg, 2.19 mmol), TEA (332 mg, 457 µL, 3.28 mmol) and DCM (6 mL) was added Boc₂O (501 mg, 2.30 mmol) under ice bath. After being stirred at rt for 1h, the mixture was concentrated and purified by flash column (EA/PE=0 to 100%) to give the desired product **Intermediate K** (490 mg) as a white foam. MS: calc'd 238 (MH⁺), measured 238 (MH⁺).

### Intermediate L

### tert-butyl 1,4,6,7-tetrahydroimidazo[4,5-c]pyridine-5-carboxylate

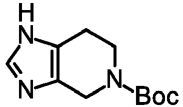

The title compound was prepared in analogy to the preparation of **Intermediate K** by using 4,5,6,7-tetrahydro-1*H*-imidazo[4,5-c]pyridine dihydrochloride (CAS: 62002-31-7, Vendor: Accela) instead of 3-methyl-4,5,6,7-tetrahydro-2*H*-pyrazolo[4,3-c]pyridine (compound **K1**). **Intermediate L** (273 mg) was obtained as a white foam. MS: calc'd 224 (MH⁺), measured 224 (MH⁺).

### Reference Compound R1

### 5-[(2S,6R)-2-[[4-(4-methoxyphenyl)piperazin-1-yl]methyl]-6-methyl-morpholin-4-yl]quinoline-8-carbonitrile

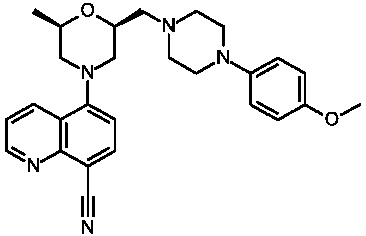

The title compound was prepared according to the following scheme:

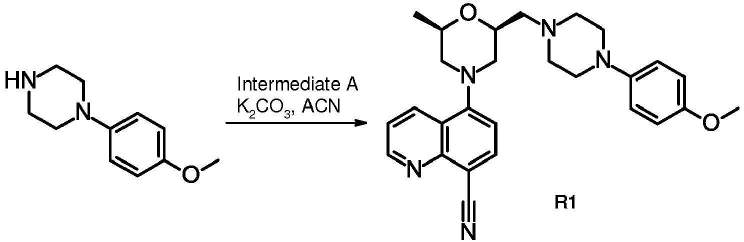

To a flask was added [(2*R*,6*R*)-4-(8-cyano-5-quinolyl)-6-methyl-morpholin-2-yl]methyl trifluoromethanesulfonate (Intermediate **A,** 30 mg, 72 µmol), 1-(4-methoxyphenyl)piperazine (CAS: 38212-30-5, Vendor: Accela, 21 mg, 108 µmol), potassium carbonate (30 mg, 217 µmol) and acetonitrile (4 mL). After being stirred at 85 °C for 2 hrs, the mixture was filtered through celite and purified by prep-HPLC to give compound **R1** (22 mg) as a yellow solid. MS: calc'd 458 (MH⁺), measured 458 (MH⁺). ¹H NMR (400MHz, METHANOL-d4) δ = 9.00 (dd, *J*=1.7, 4.2 Hz, 1H), 8.67 (dd, *J*=1.7, 8.6 Hz, 1H), 8.18 (d, *J*=8.1 Hz, 1H), 7.66 (dd, *J*=4.3, 8.6 Hz, 1H), 7.30 (d, *J*=7.9 Hz, 1H), 7.05 - 6.98 (m, 2H), 6.93 - 6.85 (m, 2H), 4.55 - 4.46 (m, 1H), 4.26 - 4.16 (m, 1H), 3.89 - 3.56 (m, 7H), 3.50 - 3.37 (m, 6H), 3.25 - 3.00 (m, 2H), 2.84 - 2.71 (m, 2H), 1.33 (d, *J*=6.2 Hz, 3H).

### Reference Compound R2

### 5-[(2S,6R)-2-[[4-(4-chlorophenyl)piperazin-1-yl]methyl]-6-methyl-morpholin-4-yl]quinoline-8-carbonitrile

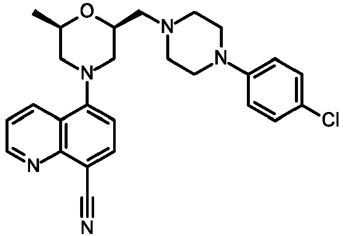

The title compound was prepared in analogy to the preparation of compound **R1** by using 1-(4-cholophenyl)piperazine (CAS: 38212-33-8, Vendor: BePharm) instead of 1-(4-methoxyphenyl)piperazine. Compound **R2** (24 mg) was obtained as a yellow solid. MS: calc'd 462 (MH⁺), measured 462 (MH⁺). ¹H NMR (400MHz, METHANOL-d4) δ = 9.00 (dd, *J*=1.6, 4.3 Hz, 1H), 8.67 (dd, *J*=1.7, 8.6 Hz, 1H), 8.17 (d, *J*=8.1 Hz, 1H), 7.66 (dd, *J*=4.3, 8.6 Hz, 1H), 7.34 - 7.26 (m, 3H), 7.07 - 6.98 (m, 2H), 4.55 - 4.47 (m, 1H), 4.25 - 4.15 (m, 1H), 3.99 - 3.55 (m, 3H), 3.55 - 3.32 (m, 7H), 3.28 - 3.04 (m, 2H), 2.85 - 2.71 (m, 2H), 1.33 (d, *J*=6.2 Hz, 3H).

### Example 1

### 5-[(2R,6S)-2-methyl-6-[[3-(4,5,6,7-tetrahydropyrazolo[4,3-c]pyridin-1-yl)azetidin-1-yl]methyl]morpholin-4-yl]quinoline-8-carbonitrile

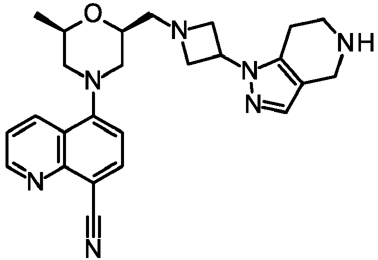

### Example 2

### 5-[(2R,6S)-2-methyl-6-[[3-(4,5,6,7-tetrahydropyrazolo[4,3-c]pyridin-2-yl)azetidin-1-yl]methyl]morpholin-4-yl]quinoline-8-carbonitrile

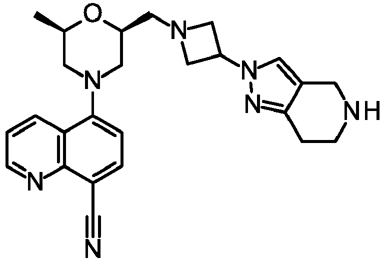

The title compounds were prepared according to the following scheme:

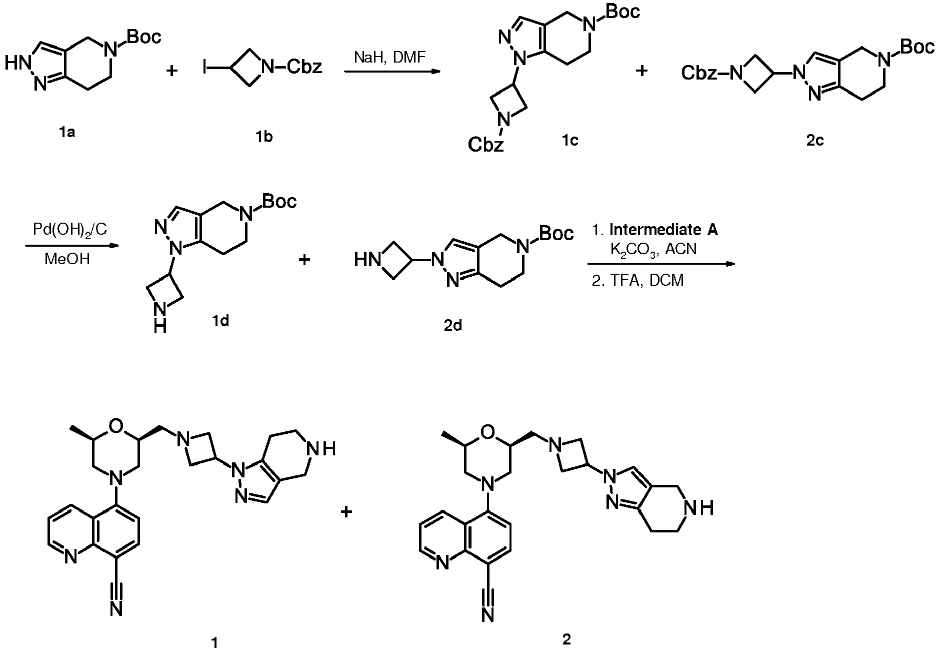

### Step 1: preparation of tert-butyl 1-(1-benzyloxycarbonylazetidin-3-yl)-6,7-dihydro-4H-pyrazolo[4,3-c]pyridine-5-carboxylate (compound 1c) and tert-butyl 2-(1-benzyloxycarbonylazetidin-3-yl)-6,7-dihydro-4H-pyrazolo[4,3-c]pyridine-5-carboxylate (compound 2c)

To a flask was added *tert*-butyl 2,4,6,7-tetrahydropyrazolo[4,3-c]pyridine-5-carboxylate (compound **1a**, CAS: 230301-11-8, Vendor: BePharm, 200 mg, 896 µmol) and DMF (2 mL), the suspension was bubbled with N₂ for 5 mins and NaH (60% in mineral oil, 107 mg, 2.69 mmol) was added. After the mixture was stirred at rt for 0.5 h, benzyl 3-iodoazetidine-1-carboxylate (compound **1b**, CAS: 939759-26-9, Vendor: PharmaBlock, 284 mg, 896 µmol) was added and the mixture was stirred at 60 °C for 2 hrs. After being cooled down, the reaction was quenched by addition of water (10 mL). The mixture was extracted by EA (10 mL) 3 times, and the organic phase was washed by brine (20 mL) twice, dried over Na₂SO₄, filtered and concentrated to give a light yellow oil which was purified by flash column (EA/PE=0 to 70%) to give a mixture of compound **1c** and **2c** (65 mg) as a yellow oil. MS: calc'd 413 (MH⁺), measured 413 (MH⁺).

### Step 2: preparation of tert-butyl 1-(azetidin-3-yl)-6,7-dihydro-4H-pyrazolo[4,3-c]pyridine-5-carboxylate (compound 1d) and tert-butyl 2-(azetidin-3-yl)-6,7-dihydro-4H-pyrazolo[4,3-c]pyridine-5-carboxylate (compound 2d)

To the flask containing *tert*-butyl 1-(1-benzyloxycarbonylazetidin-3-yl)-6,7-dihydro-4*H-*pyrazolo[4,3-c]pyridine-5-carboxylate (compound **1c**) and tert-butyl 2-(1-benzyloxycarbonylazetidin-3-yl)-6,7-dihydro-4*H*-pyrazolo[4,3-c]pyridine-5-carboxylate (compound **2c**) (65 mg, 79 µmol) was added MeOH (5 mL) and Pd(OH)₂/C (10 wt.%, 6 mg, 39 µmol). After being stirred under hydrogen balloon at rt for 2 hrs, the reaction mixture was filtered and concentrated to give a mixture of compound **1d** and **2d** (44 mg) as a light yellow oil which was used in next step without further purification. MS: calc'd 279 (MH⁺), measured 279 (MH⁺).

### Step 3: preparation of 5-[(2R,6S)-2-methyl-6-[[3-(4,5,6,7-tetrahydropyrazolo[4,3-c]pyridin-1-yl)azetidin-1-yl]methyl]morpholin-4-yl]quinoline-8-carbonitrile (Example 1) and 5-[(2R,6S)-2-methyl-6-[[3-(4,5,6,7-tetrahydropyrazolo[4,3-c]pyridin-2-yl)azetidin-1-yl]methyl]morpholin-4-yl]quinoline-8-carbonitrile (Example 2)

To a tube was added [(2*R*,6*R*)-4-(8-cyano-5-quinolyl)-6-methyl-morpholin-2-yl]methyl trifluoromethanesulfonate (Intermediate **A,** 65 mg, 156 µmol), *tert*-butyl 1-(azetidin-3-yl)-6,7-dihydro-4*H*-pyrazolo[4,3-c]pyridine-5-carboxylate (compound **1d**) and *tert*-butyl 2-(azetidin-3-yl)-6,7-dihydro-4*H*-pyrazolo[4,3-c]pyridine-5-carboxylate (compound **2d**) (44 mg, 79 µmol), potassium carbonate (65 mg, 469 µmol) and ACN (5 mL), the mixture was stirred at 55 °C for 2 hrs. After being cooled down, the mixture was filtered through celite and concentrated to give a yellow oil which was dissolved in DCM (5 mL) and TFA (1 mL). The mixture was stirred at rt for 2 hrs and then concentrated to give an oil which was purified by prep-HPLC (Gradient: 10-25% ACN in Water (0.1% TFA), Column: Waters SunFire C18, 30×100mm, 5µm) to give **Example 1** (slower eluting, 16 mg) as a light yellow solid and **Example 2** (faster eluting, 21 mg) as a light yellow solid. The two products were confirmed by Nuclear Overhauser Effect Spectroscopy (NOESY).

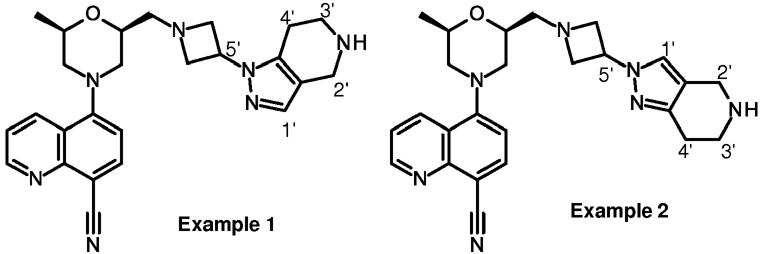

**Example** 1: MS: calc'd 444 (MH⁺), measured 444 (MH⁺). ¹H NMR (400 MHz, METHANOL-d4) δ = 9.02 (dd, *J* = 1.6, 4.2 Hz, 1H), 8.68 (dd, *J* = 1.6, 8.6 Hz, 1H), 8.20 (d, *J* = 7.9 Hz, 1H), 7.73 - 7.57 (m, 2H), 7.31 (d, *J* = 8.1 Hz, 1H), 5.44 - 5.32 (m, 1H), 4.85 - 4.78 (m, 2H), 4.77 - 4.60 (m, 2H), 4.42 - 4.32 (m, 1H), 4.28 (s, 2H), 4.21 - 4.10 (m, 1H), 3.79 - 3.60 (m, 2H), 3.56 (t, *J* = 6.2 Hz, 2H), 3.48 - 3.40 (m, 2H), 3.07 (br t, *J* = 6.5 Hz, 2H), 2.88 - 2.68 (m, 2H), 1.31 (d, *J* = 6.2 Hz, 3H). NOESY showed that H1' was correlated with H2', and H5' was correlated with H4'.

**Example 2:** MS: calc'd 444 (MH⁺), measured 444 (MH⁺). ¹H NMR (400 MHz, METHANOL-d4) δ = 9.02 (dd, *J* = 1.6, 4.3 Hz, 1H), 8.68 (dd, *J* = 1.5, 8.6 Hz, 1H), 8.19 (d, *J* = 8.1 Hz, 1H), 7.70 (s, 1H), 7.67 (dd, *J* = 4.3, 8.6 Hz, 1H), 7.31 (d, *J* = 7.9 Hz, 1H), 5.44 (quin, *J* = 7.3 Hz, 1H), 4.85 - 4.75 (m, 2H), 4.74 - 4.57 (m, 2H), 4.36 (br t, *J* = 9.9 Hz, 1H), 4.31 (s, 2H), 4.21 - 4.10 (m, 1H), 3.74 - 3.54 (m, 4H), 3.44 (br d, *J* = 12.5 Hz, 2H), 3.12 (br t, *J* = 6.1 Hz, 2H), 2.87 - 2.78 (m, 1H), 2.73 (dd, *J* = 10.3, 12.2 Hz, 1H), 1.31 (d, *J* = 6.2 Hz, 3H). NOESY showed that H1' was correlated with H2' and H5'.

### Example 3

### 5-[(2R,6S)-2-methyl-6-[[3-(4,5,6,7-tetrahydropyrazolo[3,4-c]pyridin-1-yl)azetidin-1-yl]methyl]morpholin-4-yl]quinoline-8-carbonitrile

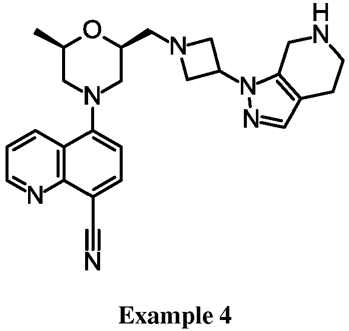

### 5-[(2R,6S)-2-methyl-6-[[3-(4,5,6,7-tetrahydropyrazolo[3,4-c]pyridin-2-yl)azetidin-1-yl]methyl]morpholin-4-yl]quinoline-8-carbonitrile

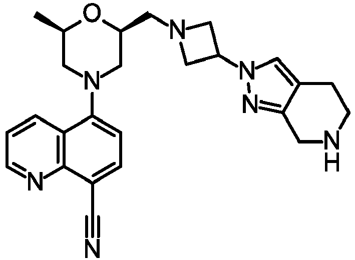

The title compounds were prepared in analogy to the preparation of **Example 1** and **Example 2** by using *tert*-butyl 2,4,5,7-tetrahydropyrazolo[3,4-c]pyridine-6-carboxylate (CAS: 871726-73-7, Vendor: BePharm) instead of *tert*-butyl 2,4,6,7-tetrahydropyrazolo[4,3-c]pyridine-5-carboxylate (compound **1a**). **Example 3** and **Example 4** were separated by prep-HPLC (Gradient: 20-65% ACN in Water (0.1% NH₃), Column: Waters X-Bridge C18, 30×100mm, 5µm). Similar to Example 1 and Example 2, the two products were also confirmed by NOESY.

**Example 3** (faster eluting, 18 mg) was obtained as a light yellow solid. MS: calc'd 444 (MH⁺), measured 444 (MH⁺). ¹H NMR (400 MHz, METHANOL-d4) δ = 8.87 (dd, *J* = 1.7, 4.2 Hz, 1H), 8.54 (dd, *J* = 1.6, 8.6 Hz, 1H), 8.05 (d, *J* = 7.9 Hz, 1H), 7.54 (dd, *J* = 4.3, 8.6 Hz, 1H), 7.26 (s, 1H), 7.15 (d, *J* = 8.1 Hz, 1H), 4.74 - 4.67 (m, 1H), 4.02 - 3.87 (m, 2H), 3.84 - 3.71 (m, 4H), 3.55 (q, *J* = 7.8 Hz, 2H), 3.27 (br d, *J* = 11.1 Hz, 2H), 2.85 (t, *J* = 5.8 Hz, 2H), 2.75 - 2.68 (m, 2H), 2.67 - 2.59 (m, 1H), 2.54 (dd, *J* = 10.3, 11.9 Hz, 1H), 2.47 (t, *J* = 5.7 Hz, 2H), 1.14 (d, *J* = 6.2 Hz, 3H).

**Example 4** (slower eluting, 60 mg) was obtained as a light yellow solid. MS: calc'd 444 (MH⁺), measured 444 (MH⁺). ¹H NMR (400 MHz, METHANOL-d4) δ = 8.99 (d, *J* = 4.2 Hz, 1H), 8.65 (d, *J* = 8.6 Hz, 1H), 8.16 (d, *J* = 7.9 Hz, 1H), 7.65 (dd, *J* = 4.2, 8.5 Hz, 1H), 7.52 (s, 1H), 7.26 (d, *J* = 8.1 Hz, 1H), 5.02 - 4.94 (m, 1H), 4.13 - 3.99 (m, 2H), 3.98 - 3.83 (m, 4H), 3.62 (dt, *J* = 3.7, 7.5 Hz, 2H), 3.39 (br d, *J* = 12.1 Hz, 2H), 3.01 (t, *J* = 5.9 Hz, 2H), 2.80 (d, *J* = 6.1 Hz, 2H), 2.78 - 2.70 (m, 1H), 2.70 - 2.58 (m, 3H), 1.25 (d, *J* = 6.2 Hz, 3H).

### Example 5

### 5-[(2R,6S)-2-methyl-6-[[3-(6-methyl-5,7-dihydro-4H-pyrazolo[3,4-c]pyridin-2-yl)azetidin-1-yl]methyl]morpholin-4-yl]quinoline-8-carbonitrile

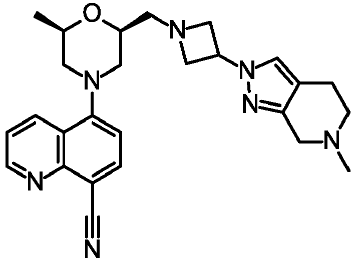

The title compound was prepared according to the following scheme:

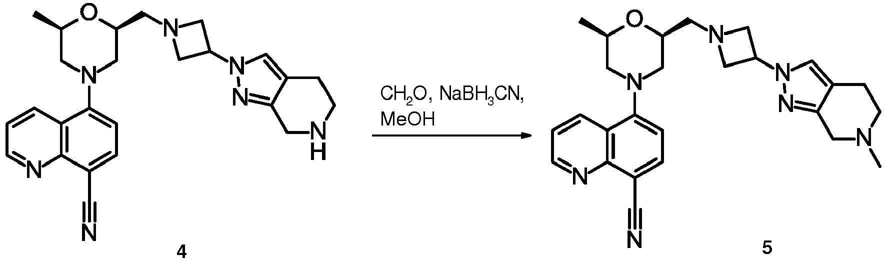

### Step 1: preparation of 5-[(2R,6S)-2-methyl-6-[[3-(6-methyl-5,7-dihydro-4H-pyrazolo[3,4-c]pyridin-2-yl)azetidin-1-yl]methyl]morpholin-4-yl]quinoline-8-carbonitrile (Example 5)

To the flask containing 5-[(2*R*,6*S*)-2-methyl-6-[[3-(4,5,6,7-tetrahydropyrazolo[3,4-c]pyridin-2-yl)azetidin-1-yl]methyl]morpholin-4-yl]quinofine-8-carbonitrile (**Example 4, 30** mg, 68 µmol) and MeOH (2 mL) was added formaldehyde (37% in water, 25 µL, 340 µmol). After the mixture was stirred at rt for 10 mins, NaBH₃CN (13 mg, 204 µmol) was added. After being stirred for another hour, the mixture was directly purified by prep-HPLC to give **Example 5 (19** mg) as a light yellow solid. MS: calc'd 458 (MH⁺), measured 458 (MH⁺). ¹H NMR (400 MHz, METHANOL-d4) δ = 8.99 (dd, *J* = 1.6, 4.3 Hz, 1H), 8.66 (dd, *J* = 1.6, 8.6 Hz, 1H), 8.16 (d, *J* = 8.1 Hz, 1H), 7.65 (dd, *J* = 4.2, 8.6 Hz, 1H), 7.53 (s, 1H), 7.26 (d, *J=* 8.1 Hz, 1H), 5.02 - 4.95 (m, 1H), 4.13 - 3.99 (m, 2H), 3.96 - 3.82 (m, 2H), 3.62 (dt, *J* = 4.0, 7.5 Hz, 2H), 3.56 (s, 2H), 3.43 - 3.36 (m, 2H), 2.84 - 2.76 (m, 2H), 2.76 - 2.62 (m, 6H), 2.50 (s, 3H), 1.25 (d, *J* = 6.2 Hz, 3H).

### Example 6

### 5-[(2S,6R)-2-[[3-(6-ethyl-5,7-dihydro-4H-pyrazolo[3,4-c]pyridin-1-yl)azetidin-1-yl]methyl]-6-methyl-morpholin-4-yl]quinoline-8-carbonitrile

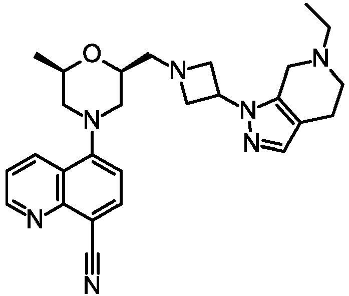

### Example 7

### 5-[(2S,6R)-2-[[3-(6-ethyl-5,7-dihydro-4H-pyrazolo[3,4-c]pyridin-2-yl)azetidin-1-yl]methyl]-6-methyl-morpholin-4-yl]quinoline-8-carbonitrile

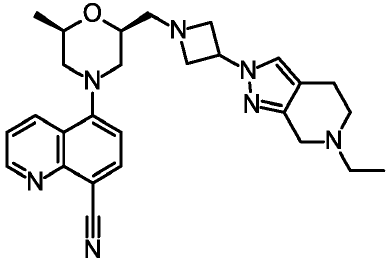

The title compounds were prepared in analogy to the preparation of **Example 5** by using a mixture of **Example 3** and **Example 4** instead of **Example 4** alone; acetaldehyde (5M in THF) instead of formaldehyde (37% in water). **Example 6** and **Example 7** were separated by prep-HPLC (Gradient: 5-25% ACN in Water (0.1% FA), Column: Waters CSH C18, 30×100mm, 5µm).

**Example 6** (slower eluting, 20 mg) was obtained as a yellow solid. MS: calc'd 472 (MH⁺), measured 472 (MH⁺). ¹H NMR (400 MHz, METHANOL-d4) δ = 8.99 (d, *J* = 4.2 Hz, 1H), 8.66 (dd, *J=* 1.6, 8.6 Hz, 1H), 8.17 (d, *J=* 8.1 Hz, 1H), 7.69 - 7.60 (m, 2H), 7.29 (d, *J=* 7.9 Hz, 1H), 5.35 - 5.22 (m, 1H), 4.69 - 4.54 (m, 4H), 4.53 - 4.39 (m, 2H), 4.37 - 4.28 (m, 1H), 4.18 - 4.07 (m, 1H), 3.70 - 3.49 (m, 3H), 3.46 - 3.32 (m, 5H), 2.95 (br s, 2H), 2.84 - 2.67 (m, 2H), 1.48 - 1.40 (m, 3H), 1.28 (d, *J* = 6.2 Hz, 3H).

**Example 7** (faster eluting, 58 mg) was obtained as a yellow solid. MS: calc'd 472 (MH⁺), measured 472 (MH⁺). ¹H NMR (400 MHz, METHANOL-d4) δ = 8.96 (dd, *J* = 1.5, 4.2 Hz, 1H), 8.63 (dd, *J* = 1.5, 8.6 Hz, 1H), 8.13 (d, *J* = 7.9 Hz, 1H), 7.73 - 7.58 (m, 2H), 7.25 (d, *J* = 8.1 Hz, 1H), 5.44 (quin, *J* = 7.3 Hz, 1H), 4.83 - 4.74 (m, 2H), 4.67 (br s, 3H), 4.42 - 4.23 (m, 2H), 4.19 - 4.06 (m, 1H), 3.92 - 3.51 (m, 3H), 3.48 - 3.34 (m, 5H), 3.09 - 2.91 (m, 2H), 2.84 - 2.61 (m, 2H), 1.45 (t, *J* = 7.3 Hz, 3H), 1.28 (d, *J* = 6.2 Hz, 3H).

### Example 8

### 5-[(2S,6R)-2-[[3-(6-isopropyl-5,7-dihydro-4H-pyrazolo[3,4-c]pyridin-1-yl)azetidin-1-yl]methyl]-6-methyl-morpholin-4-yl]quinoline-8-carbonitrile

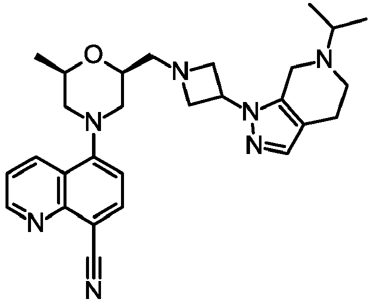

### Example 9

### 5-[(2S,6R)-2-[[3-(6-isopropyl-5,7-dihydro-4H-pyrazolo[3,4-c]pyridin-2-yl)azetidin-1-yl]methyl]-6-methyl-morpholin-4-yl]quinoline-8-carbonitrile

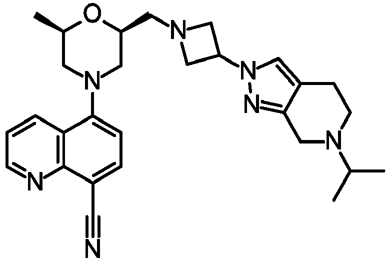

The title compounds were prepared in analogy to the preparation of **Example 6** and **Example 7** by using acetone instead of acetaldehyde (5M in THF), changing the reaction temperature from rt to 50°C, and reaction time from 1h to overnight. **Example 8** and **Example 9** were separated by prep-HPLC (Gradient: 5-25% ACN in Water (0.1% FA), Column: Waters CSH C18, 30×100mm, 5µm).

**Example 8** (slower eluting, 11 mg) was obtained as a yellow solid. MS: calc'd 486 (MH⁺), measured 486 (MH⁺). ¹H NMR (400 MHz, METHANOL-d4) δ = 8.98 (dd, *J* = 1.6, 4.3 Hz, 1H), 8.66 (dd, *J* = 1.6, 8.6 Hz, 1H), 8.16 (d, *J* = 7.9 Hz, 1H), 7.65 (dd, *J* = 4.3, 8.6 Hz, 1H), 7.57 (s, 1H), 7.27 (d, *J* = 7.9 Hz, 1H), 5.26 - 5.14 (m, 1H), 4.55 - 4.47 (m, 2H), 4.43 - 4.31 (m, 4H), 4.29 - 4.19 (m, 1H), 4.16 - 4.06 (m, 1H), 3.65 (td, *J* = 6.6, 13.2 Hz, 1H), 3.46 - 3.33 (m, 6H), 2.90 (t, *J* = 5.9 Hz, 2H), 2.83 - 2.64 (m, 2H), 1.41 (d, *J* = 6.7 Hz, 6H), 1.27 (d, *J=* 6.2 Hz, 3H).

**Example 9** (faster eluting, 61 mg) was obtained as a yellow solid. MS: calc'd 486 (MH⁺), measured 486 (MH⁺). ¹H NMR (400 MHz, METHANOL-d4) δ = 8.95 (br s, 1H), 8.61 (br d, *J* = 8.2 Hz, 1H), 8.18 - 8.00 (m, 1H), 7.74 - 7.57 (m, 2H), 7.31 - 7.14 (m, 1H), 5.22 (br d, *J* = 5.4 Hz, 1H), 4.37 (s, 4H), 4.24 - 4.01 (m, 4H), 3.70 (td, *J* = 6.3, 12.8 Hz, 1H), 3.49 (br t, *J* = 5.7 Hz, 2H), 3.43 - 3.34 (m, 2H), 3.27 - 3.12 (m, 2H), 2.98 (br t, *J* = 5.7 Hz, 2H), 2.81 - 2.59 (m, 2H), 1.44 (d, *J* = 6.6 Hz, 6H), 1.25 (d, *J* = 6.1 Hz, 3H).

### Example 10

### 5-[(2S,6R)-2-[[3-(5,6-dihydro-4H-pyrrolo[3,4-c]pyrazol-1-yl)azetidin-1-yl]methyl]-6-methyl-morpholin-4-yl]quinoline-8-carbonitrile

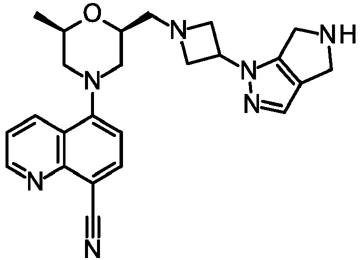

### Example 11

### 5-[(2S,6R)-2-[[3-(5,6-dihydro-4H-pyrrolo[3,4-c]pyrazol-2-yl)azetidin-1-yl]methyl]-6-methyl-morpholin-4-yl]quinoline-8-carbonitrile

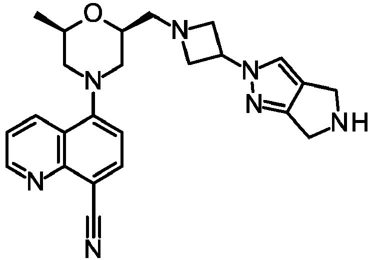

The title compounds were prepared in analogy to the preparation of **Example 1** and **Example 2** by using *tert*-butyl 4,6-dihydro-2*H*-pyrrolo[3,4-c]pyrazole-5-carboxylate (CAS: 1280210-79-8, Vendor: Pharmablock) instead of *tert*-butyl 2,4,6,7-tetrahydropyrazolo[4,3-c]pyridine-5-carboxylate (compound **1a**). **Example 10** and **Example 11** were separated by prep-HPLC (Gradient: 20-65% ACN in Water (0.1% NH₃), Column: Waters X-Bridge C18, 30×100mm, 5µm). Similar to Example 1 and Example 2, the two products were also confirmed by NOESY.

**Example 10** (faster eluting, 19 mg) was obtained as a light yellow solid. MS: calc'd 430 (MH⁺), measured 430 (MH⁺). ¹H NMR (400 MHz, METHANOL-d4) δ = 9.00 (dd, *J* = 1.7, 4.2 Hz, 1H), 8.66 (dd, *J* = 1.6, 8.6 Hz, 1H), 8.17 (d, *J* = 7.9 Hz, 1H), 7.72 - 7.58 (m, 1H), 7.31 (s, 1H), 7.27 (d, *J* = 8.1 Hz, 1H), 4.95 - 4.91 (m, 1H), 4.23 (s, 2H), 4.10 - 3.98 (m, 4H), 3.95 - 3.82 (m, 2H), 3.65 - 3.56 (m, 2H), 3.43 - 3.36 (m, 2H), 2.85 - 2.71 (m, 3H), 2.71 - 2.61 (m, 1H), 1.26 (d, *J* = 6.2 Hz, 3H).

**Example 11** (slower eluting, 55 mg) was obtained as a light yellow solid. MS: calc'd 430 (MH⁺), measured 430 (MH⁺). ¹H NMR (400 MHz, METHANOL-d4) δ = 8.87 (dd, *J* = 1.6, 4.3 Hz, 1H), 8.54 (dd, *J* = 1.6, 8.6 Hz, 1H), 8.04 (d, *J* = 8.1 Hz, 1H), 7.53 (dd, *J* = 4.3, 8.6 Hz, 1H), 7.37 (s, 1H), 7.14 (d, *J* = 8.1 Hz, 1H), 4.93 - 4.84 (m, 1H), 3.99 - 3.73 (m, 8H), 3.54 (dt, *J* = 4.7, 7.5 Hz, 2H), 3.28 (br d, *J* = 12.2 Hz, 2H), 2.72 - 2.59 (m, 3H), 2.54 (dd, *J* = 10.3, 12.0 Hz, 1H), 1.14 (d, *J* = 6.2 Hz, 3H).

### Example 12

### 5-[(2R,6S)-2-methyl-6-[[3-(3-methyl-4,5,6,7-tetrahydropyrazolo[4,3-c]pyridin-1-yl)azetidin-1-yl]methyl]morpholin-4-yl]quinoline-8-carbonitrile

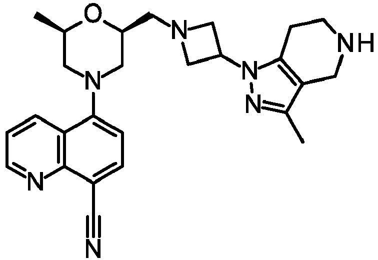

### Example 13

### 5-[(2R,6S)-2-methyl-6-[[3-(3-methyl-4,5,6,7-tetrahydropyrazolo[4,3-c]pyridin-2-yl)azetidin-1-yl]methyl]morpholin-4-yl]quinoline-8-carbonitrile

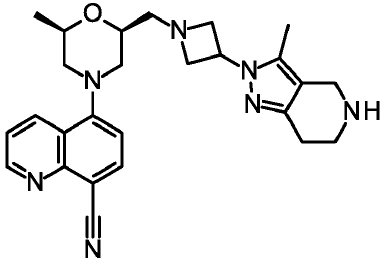

The title compounds were prepared in analogy to the preparation of **Example** 1 and **Example 2** by using **Intermediate K** instead of *tert*-butyl 2,4,6,7-tetrahydropyrazolo[4,3-c]pyridine-5-carboxylate (compound **1a**). **Example 12** and **Example 13** were separated by prep-HPLC (Gradient: 10-25% ACN in Water (0.1% TFA), Column: Waters SunFire C18, 30×100mm, 5µm). Similar to Example 1 and Example 2, the two products were also confirmed by NOESY.

**Example 12** (slower eluting, 60 mg) was obtained as a yellow solid. MS: calc'd 458 (MH⁺), measured 458 (MH⁺). ¹H NMR (400 MHz, METHANOL-d4) δ = 9.01 (dd, *J* = 1.6, 4.3 Hz, 1H), 8.67 (dd, *J* = 1.3, 8.6 Hz, 1H), 8.18 (d, *J* = 8.1 Hz, 1H), 7.67 (dd, *J* = 4.2, 8.6 Hz, 1H), 7.30 (d, *J=* 8.1 Hz, 1H), 5.39 - 5.25 (m, 1H), 5.05 - 4.94 (m, 2H), 4.73 - 4.58 (m, 2H), 4.43 - 4.32 (m, 1H), 4.27 - 4.09 (m, 3H), 3.79 - 3.57 (m, 2H), 3.53 (t, *J* = 6.2 Hz, 2H), 3.49 - 3.39 (m, 2H), 3.03 (br t, *J* = 6.0 Hz, 2H), 2.89 - 2.78 (m, 1H), 2.73 (dd, *J* = 10.5, 12.0 Hz, 1H), 2.27 (s, 3H), 1.31 (d, *J* = 6.2 Hz, 3H).

**Example 13** (faster eluting, 40 mg) was obtained as a yellow solid. MS: calc'd 458 (MH⁺), measured 458 (MH⁺). ¹H NMR (400 MHz, METHANOL-d4) δ = 8.99 - 8.81 (m, 1H), 8.55 (d, *J* = 8.6 Hz, 1H), 8.05 (d, *J* = 8.1 Hz, 1H), 7.54 (dd, *J* = 4.3, 8.6 Hz, 1H), 7.17 (d, *J* = 7.9 Hz, 1H), 5.33 (quin, *J* = 7.1 Hz, 1H), 4.85 (br s, 2H), 4.64 - 4.45 (m, 2H), 4.32 - 4.20 (m, 1H), 4.10 (s, 2H), 4.08 - 3.97 (m, 1H), 3.68 - 3.48 (m, 2H), 3.43 (br t, *J* = 6.3 Hz, 2H), 3.38 - 3.27 (m, 2H), 2.95 (br t, *J=* 6.1 Hz, 2H), 2.76 - 2.66 (m, 1H), 2.61 (dd, *J=* 10.5, 12.0 Hz, 1H), 2.15 (s, 3H), 1.18 (d, *J=* 6.2 Hz, 3H).

### Example 14

### 5-[(2S,6R)-2-[[3-(3,5-dimethyl-6,7-dihydro-4H-pyrazolo[4,3-c]pyridin-1-yl)azetidin-1-yl]methyl]-6-methyl-morpholin-4-yl]quinoline-8-carbonitrile

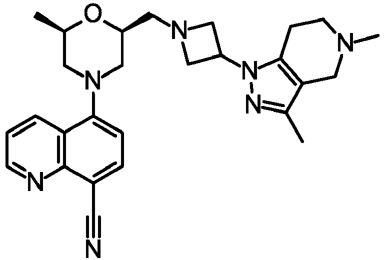

### Example 15

### 5-[(2S,6R)-2-[[3-(3,5-dimethyl-6,7-dihydro-4H-pyrazolo[4,3-c]pyridin-2-yl)azetidin-1-yl]methyl]-6-methyl-morpholin-4-yl]quinoline-8-carbonitrile

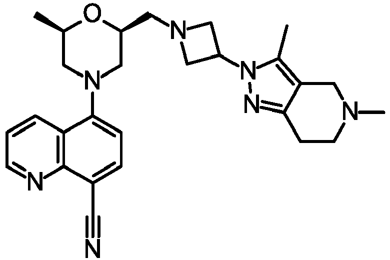

The title compounds were prepared in analogy to the preparation of **Example 5** by using a mixture of **Example 12** and **Example 13** instead of **Example 4. Example 14** and **Example 15** were separated by prep-HPLC (Gradient: 10-30% ACN in Water (0.1% TFA), Column: Waters SunFire C18, 30×100mm, 5µm).

**Example 14** (slower eluting, 18 mg) was obtained as a yellow solid. MS: calc'd 472 (MH⁺), measured 472 (MH⁺). ¹H NMR (400 MHz, METHANOL-d4) δ = 9.00 (dd, *J* = 1.6, 4.3 Hz, 1H), 8.66 (dd, *J* = 1.5, 8.6 Hz, 1H), 8.17 (d, *J* = 7.9 Hz, 1H), 7.65 (dd, *J* = 4.3, 8.6 Hz, 1H), 7.29 (d, *J* = 8.1 Hz, 1H), 5.34 - 5.20 (m, 1H), 4.82 - 4.72 (m, 2H), 4.72 - 4.58 (m, 2H), 4.50 - 4.30 (m, 2H), 4.24 - 4.03 (m, 2H), 3.77 - 3.55 (m, 3H), 3.42 (br dd, *J* = 1.7, 12.2 Hz, 3H), 3.09 (br t, *J* = 5.8 Hz, 2H), 3.05 (s, 3H), 2.85 - 2.76 (m, 1H), 2.72 (dd, *J* = 10.4, 12.1 Hz, 1H), 2.25 (s, 3H), 1.29 (d, *J* = 6.2 Hz, 3H).

**Example 15** (faster eluting, 12 mg) was obtained as a yellow solid. MS: calc'd 472 (MH⁺), measured 472 (MH⁺). ¹H NMR (400 MHz, METHANOL-d4) δ = 8.99 (dd, *J=* 1.6, 4.2 Hz, 1H), 8.65 (dd, *J* = 1.5, 8.6 Hz, 1H), 8.16 (d, *J=* 8.1 Hz, 1H), 7.65 (dd, *J=* 4.3, 8.6 Hz, 1H), 7.28 (d, *J* = 8.1 Hz, 1H), 5.42 (quin, *J* = 7.2 Hz, 1H), 4.84 - 4.74 (m, 2H), 4.73 - 4.56 (m, 2H), 4.53 - 4.40 (m, 1H), 4.39 - 4.29 (m, 1H), 4.18 - 4.04 (m, 2H), 3.87 - 3.53 (m, 3H), 3.50 - 3.37 (m, 3H), 3.19 - 3.07 (m, 2H), 3.05 (s, 3H), 2.80 (dd, *J=* 10.5, 11.7 Hz, 1H), 2.71 (dd, *J=* 10.3, 12.2 Hz, 1H), 2.24 (s, 3H), 1.28 (d, *J* = 6.2 Hz, 3H).

### Example 16

### 5-[(2R,6S)-2-methyl-6-[[3-[(6R)-6-methyl-4,5,6,7-tetrahydropyrazolo[4,3-c]pyridin-1-yl]azetidin-1-yl]methyl]morpholin-4-yl]quinoline-8-carbonitrile

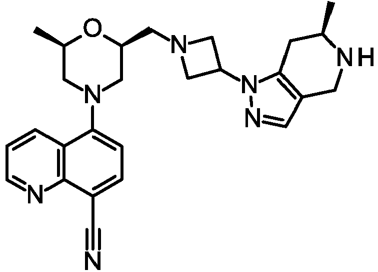

### Example 17

### 5-[(2R,6S)-2-methyl-6-[[3-[(6R)-6-methyl-4,5,6,7-tetrahydropyrazolo[4,3-c]pyridin-2-yl]azetidin-1-yl]methyl]morpholin-4-yl]quinoline-8-carbonitrile

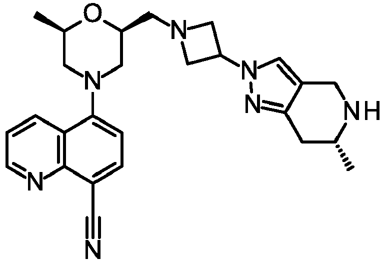

The title compounds were prepared in analogy to the preparation of **Example 1** and **Example 2** by using **Intermediate F** instead of *tert*-butyl 2,4,6,7-tetrahydropyrazolo[4,3-c]pyridine-5-carboxylate (compound **1a**), except that the reaction condition of **step 1** was modified by changing the base and solvent from NaH and DMF to Cs₂CO₃ and ACN, and changing the reaction temperature and reaction time from 60°C and 2 hrs to reflux and overnight. **Example 16** and **Example 17** were separated by chiral SFC (Gradient: 45% Isopropanol (0.1% NH₃H₂O) in CO₂, Column: Daicel chiralcel OD 250×30mm, 5µm). Similar to Example 1 and Example 2, the two products were also confirmed by NOESY.

**Example 16** (faster eluting, 21 mg) was obtained as a light yellow solid. MS: calc'd 458 (MH⁺), measured 458 (MH⁺). ¹H NMR (400 MHz, METHANOL-d₄) δ = 8.88 (dd, *J* = 1.6, 4.3 Hz, 1H), 8.54 (dd, *J* = 1.6, 8.6 Hz, 1H), 8.05 (d, *J* = 7.9 Hz, 1H), 7.54 (dd, *J* = 4.3, 8.6 Hz, 1H), 7.43 (s, 1H), 7.15 (d, *J* = 7.9 Hz, 1H), 4.97 - 4.84 (m, 1H), 4.20 - 4.03 (m, 2H), 4.03 - 3.86 (m, 4H), 3.73 (br s, 2H), 3.56 - 3.44 (m, 1H), 3.28 (br d, *J* = 11.1 Hz, 2H), 3.06 (dd, *J* = 4.9, 16.8 Hz, 1H), 2.82 (br d, *J* = 5.6 Hz, 2H), 2.70 - 2.50 (m, 3H), 1.39 (d, *J* = 6.5 Hz, 3H), 1.15 (d, *J* = 6.4 Hz, 3H).

**Example 17** (slower eluting, 20 mg) was obtained as a light yellow solid. MS: calc'd 458 (MH⁺), measured 458 (MH⁺). ¹H NMR (400 MHz, METHANOL-d₄) δ = 8.87 (dd, *J* = 1.6, 4.3 Hz, 1H), 8.53 (dd, *J* = 1.6, 8.6 Hz, 1H), 8.04 (d, *J* = 8.1 Hz, 1H), 7.53 (dd, *J* = 4.2, 8.6 Hz, 1H), 7.50 (s, 1H), 7.14 (d, *J* = 8.1 Hz, 1H), 4.93 - 4.85 (m, 1H), 4.06 - 3.86 (m, 4H), 3.85 - 3.73 (m, 2H), 3.61 - 3.47 (m, 2H), 3.27 (br d, *J* = 12.0 Hz, 2H), 3.20 - 3.15 (m, 1H), 2.85 (br dd, *J* = 4.3, 16.3 Hz, 1H), 2.69 (br d, *J* = 5.6 Hz, 2H), 2.67 - 2.59 (m, 1H), 2.58 - 2.40 (m, 2H), 1.28 (d, *J* = 6.5 Hz, 3H), 1.13 (d, *J* = 6.2 Hz, 3H).

### Example 18

### 5-[(2R,6S)-2-methyl-6-[[3-[(6S)-6-methyl-4,5,6,7-tetrahydropyrazolo[4,3-c]pyridin-1-yl]azetidin-1-yl]methyl]morpholin-4-yl]quinoline-8-carbonitrile

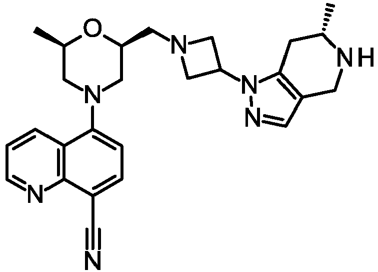

### Example 19

### 5-[(2R,6S)-2-methyl-6-[[3-[(6S)-6-methyl-4,5,6,7-tetrahydropyrazolo[4,3-c]pyridin-2-yl]azetidin-1-yl]methyl]morpholin-4-yl]quinoline-8-carbonitrile

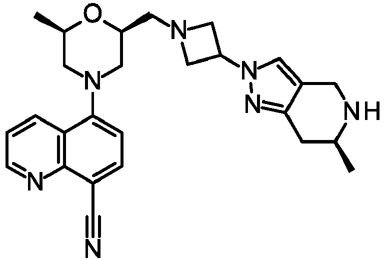

The title compounds were prepared in analogy to the preparation of **Example 16** and **Example 17** by using **Intermediate H** instead of **Intermediate F. Example 18** and **Example 19** were separated by prep-HPLC (Gradient: 5-25% ACN in Water (0.1% TFA), Column: Waters X-Bridge C8, 30×100mm, 5µm).

**Example 18** (slower eluting, 10 mg) was obtained as a light yellow solid. MS: calc'd 458 (MH⁺), measured 458 (MH⁺). ¹H NMR (400 MHz, METHANOL-d₄) δ = 9.00 (dd, *J* = 1.5, 4.3 Hz, 1H), 8.67 (dd, *J* = 1.5, 8.6 Hz, 1H), 8.17 (d, *J* = 7.9 Hz, 1H), 7.72 - 7.59 (m, 2H), 7.29 (d, *J* = 8.1 Hz, 1H), 5.38 (quin, *J* = 7.3 Hz, 1H), 4.87 - 4.77 (m, 2H), 4.69 (br s, 2H), 4.45 - 4.20 (m, 3H), 4.19 - 4.07 (m, 1H), 3.81 - 3.55 (m, 3H), 3.44 (br t, *J* = 10.9 Hz, 2H), 3.20 (br dd, *J* = 4.5, 16.9 Hz, 1H), 2.90 - 2.64 (m, 3H), 1.54 (d, *J* = 6.6 Hz, 3H), 1.30 (d, *J* = 6.2 Hz, 3H).

**Example 19** (faster eluting, 12 mg) was obtained as a light yellow solid. MS: calc'd 458 (MH⁺), measured 458 (MH⁺). ¹H NMR (400 MHz, METHANOL-d₄) δ = 9.01 (dd, *J* = 1.6, 4.3 Hz, 1H), 8.67 (dd, *J* = 1.5, 8.6 Hz, 1H), 8.18 (d, *J* = 8.1 Hz, 1H), 7.71 (s, 1H), 7.67 (dd, *J* = 4.2, 8.6 Hz, 1H), 7.30 (d, *J* = 8.1 Hz, 1H), 5.45 (quin, *J* = 7.5 Hz, 1H), 4.85 - 4.75 (m, 2H), 4.69 (br d, *J* = 6.5 Hz, 2H), 4.42 - 4.31 (m, 2H), 4.31 - 4.23 (m, 1H), 4.20 - 4.10 (m, 1H), 3.78 - 3.54 (m, 3H), 3.49 - 3.39 (m, 2H), 3.21 (dd, *J* = 4.6, 16.9 Hz, 1H), 2.90 - 2.77 (m, 2H), 2.73 (dd, *J* = 10.3, 12.2 Hz, 1H), 1.54 (d, *J* = 6.5 Hz, 3H), 1.30 (d, *J* = 6.2 Hz, 3H).

### Example 20

### 5-[(2R,6S)-2-methyl-6-[[3-[(4R)-4-methyl-4,5,6,7-tetrahydropyrazolo[4,3-c]pyridin-1-yl]azetidin-1-yl]methyl]morpholin-4-yl]quinoline-8-carbonitrile

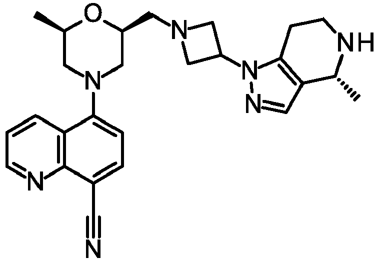

### Example 21

### 5-[(2R,6S)-2-methyl-6-[[3-[(4R)-4-methyl-4,5,6,7-tetrahydropyrazolo[4,3-c]pyridin-2-yl]azetidin-1-yl]methyl]morpholin-4-yl]quinoline-8-carbonitrile

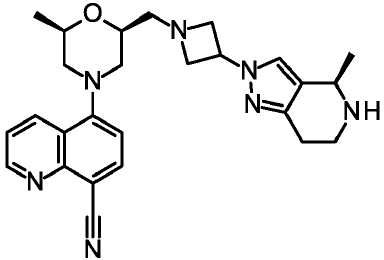

The title compounds were prepared in analogy to the preparation of **Example 16** and **Example 17** by using **Intermediate G** instead of **Intermediate F. Example 20** and **Example 21** were separated by prep-HPLC (Gradient: 10-25% ACN in Water (0.1% TFA), Column: Waters SunFire C18, 30×100mm, 5µm).

**Example 20** (slower eluting, 29 mg) was obtained as a yellow solid. MS: calc'd 458 (MH⁺), measured 458 (MH⁺). ¹H NMR (400 MHz, METHANOL-d4) δ = 9.01 (dd, *J* = 1.5, 4.2 Hz, 1H), 8.68 (dd, *J* = 1.5, 8.6 Hz, 1H), 8.19 (d, *J* = 7.9 Hz, 1H), 7.73 (s, 1H), 7.67 (dd, *J* = 4.3, 8.6 Hz, 1H), 7.31 (d, *J* = 8.1 Hz, 1H), 5.38 (td, *J* = 7.3, 14.5 Hz, 1H), 4.86 - 4.80 (m, 2H), 4.71 (br s, 2H), 4.55 (q, *J* = 6.6 Hz, 1H), 4.42 - 4.33 (m, 1H), 4.21 - 4.11 (m, 1H), 3.79 - 3.55 (m, 3H), 3.49 - 3.39 (m, 3H), 3.13 - 3.03 (m, 2H), 2.87 - 2.79 (m, 1H), 2.73 (dd, *J* = 10.3, 12.2 Hz, 1H), 1.65 (d, *J* = 6.7 Hz, 3H), 1.31 (d, *J* = 6.2 Hz, 3H).

**Example 21** (faster eluting, 48 mg) was obtained as a yellow solid. MS: calc'd 458 (MH⁺), measured 458 (MH⁺). ¹H NMR (400 MHz, METHANOL-d4) δ = 9.02 (dd, *J* = 1.6, 4.2 Hz, 1H), 8.68 (dd, *J* = 1.5, 8.6 Hz, 1H), 8.19 (d, *J* = 7.9 Hz, 1H), 7.78 (s, 1H), 7.67 (dd, *J* = 4.3, 8.6 Hz, 1H), 7.31 (d, *J* = 8.1 Hz, 1H), 5.44 (quin, *J* = 7.4 Hz, 1H), 4.98 - 4.94 (m, 2H), 4.75 - 4.62 (m, 2H), 4.57 (q, *J* = 6.4 Hz, 1H), 4.40 - 4.31 (m, 1H), 4.20 - 4.09 (m, 1H), 3.79 - 3.55 (m, 3H), 3.49 - 3.40 (m, 3H), 3.10 (br dd, *J* = 4.0, 6.9 Hz, 2H), 2.82 (t, *J* = 11.1 Hz, 1H), 2.73 (dd, *J* = 10.5, 12.1 Hz, 1H), 1.63 (d, *J* = 6.7 Hz, 3H), 1.31 (d, *J* = 6.2 Hz, 3H).

### Example 22

### 5-[(2R,6S)-2-methyl-6-[[3-[(4S)-4-methyl-4,5,6,7-tetrahydropyrazolo[4,3-c]pyridin-1-yl]azetidin-1-yl]methyl]morpholin-4-yl]quinoline-8-carbonitrile

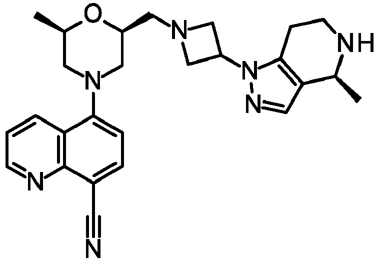

### Example 23

### 5-[(2R,6S)-2-methyl-6-[[3-[(4S)-4-methyl-4,5,6,7-tetrahydropyrazolo[4,3-c]pyridin-2-yl]azetidin-1-yl]methyl]morpholin-4-yl]quinoline-8-carbonitrile

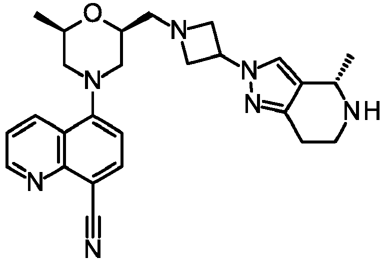

The title compounds were prepared in analogy to the preparation of **Example 16** and **Example 17** by using **Intermediate I** instead of **Intermediate F. Example 22** and **Example 23** were separated by prep-HPLC (Gradient: 10-25% ACN in Water (0.1% TFA), Column: Waters SunFire C18, 30×100mm, 5µm).

**Example 22** (slower eluting, 40 mg) was obtained as a yellow solid. MS: calc'd 458 (MH⁺), measured 458 (MH⁺). ¹H NMR (400 MHz, METHANOL-d4) δ = 9.07 - 8.96 (m, 1H), 8.68 (d, *J* = 7.7 Hz, 1H), 8.20 (d, *J* = 8.1 Hz, 1H), 7.73 (s, 1H), 7.68 (dd, *J* = 4.3, 8.6 Hz, 1H), 7.31 (d, *J* = 8.1 Hz, 1H), 5.42 - 5.33 (m, 1H), 4.84 - 4.79 (m, 2H), 4.74 - 4.61 (m, 2H), 4.59 - 4.50 (m, 1H), 4.37 (dt, *J* = 2.3, 9.9 Hz, 1H), 4.20 - 4.11 (m, 1H), 3.79 - 3.56 (m, 3H), 3.49 - 3.40 (m, 3H), 3.12 - 3.00 (m, 2H), 2.83 (t, *J* = 11.1 Hz, 1H), 2.78 - 2.68 (m, 1H), 1.65 (d, *J* = 6.7 Hz, 3H), 1.31 (d, *J* = 6.2 Hz, 3H).

**Example 23** (faster eluting, 48 mg) was obtained as a yellow solid. MS: calc'd 458 (MH⁺), measured 458 (MH⁺). ¹H NMR (400 MHz, METHANOL-d4) δ = 8.99 (dd, *J* = 1.7, 4.2 Hz, 1H), 8.66 (dd, *J* = 1.6, 8.7 Hz, 1H), 8.16 (d, *J* = 7.9 Hz, 1H), 7.78 (s, 1H), 7.65 (dd, *J* = 4.3, 8.6 Hz, 1H), 7.28 (d, *J* = 8.1 Hz, 1H), 5.45 (quin, *J* = 7.4 Hz, 1H), 4.86 - 4.77 (m, 2H), 4.69 (br s, 2H), 4.57 (q, *J* = 6.6 Hz, 1H), 4.43 - 4.31 (m, 1H), 4.21 - 4.09 (m, 1H), 3.81 - 3.54 (m, 3H), 3.51 - 3.38 (m, 3H), 3.15 - 3.05 (m, 2H), 2.82 (dd, *J=* 10.5, 11.7 Hz, 1H), 2.72 (dd, *J=* 10.3, 12.2 Hz, 1H), 1.63 (d, *J* = 6.7 Hz, 3H), 1.30 (d, *J* = 6.2 Hz, 3H).

### Example 24

### 5-[(2S,6R)-2-[[3-(7,7-dimethyl-5,6-dihydro-4H-pyrazolo[4,3-c]pyridin-2-yl)azetidin-1-yl]methyl]-6-methyl-morpholin-4-yl]quinoline-8-carbonitrile

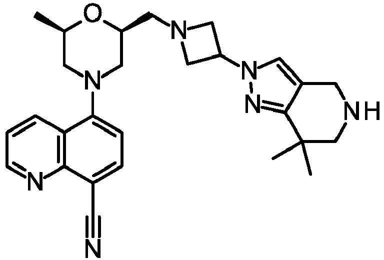

The title compound was prepared in analogy to the preparation of **Example 2** by using tert-butyl 7,7-dimethyl-4,6-dihydro-2*H*-pyrazolo[4,3-c]pyridine-5-carboxylate (CAS: 635712-88-8, Vendor: Accela) instead of tert-butyl 2,4,6,7-tetrahydropyrazolo[4,3-c]pyridine-5-carboxylate (compound **1a**). **Example 24** (108 mg) was obtained as a light yellow solid. MS: calc'd 472 (MH⁺), measured 472 (MH⁺). ¹H NMR (400 MHz, METHANOL-d4) δ = 8.99 (dd, *J* = 1.7, 4.2 Hz, 1H), 8.66 (dd, *J* = 1.7, 8.6 Hz, 1H), 8.16 (d, *J* = 7.9 Hz, 1H), 7.69 (s, 1H), 7.66 (dd, *J* = 4.3, 8.6 Hz, 1H), 7.28 (d, *J* = 8.1 Hz, 1H), 5.27 (quin, *J* = 7.2 Hz, 1H), 4.42 (q, *J* = 8.7 Hz, 2H), 4.33 - 4.18 (m, 5H), 4.18 - 4.06 (m, 1H), 3.46 - 3.38 (m, 2H), 3.37 - 3.33 (m, 2H), 3.32 - 3.22 (m, 2H), 2.79 (dd, *J* = 10.5, 11.8 Hz, 1H), 2.70 (dd, *J* = 10.3, 12.1 Hz, 1H), 1.47 (s, 6H), 1.29 (d, *J* = 6.2 Hz, 3H).

### Example 25 and Example 26

### 5-[(2R,6S)-2-methyl-6-[[3-[(7R)-7-methyl-4,5,6,7-tetrahydropyrazolo[4,3-c]pyridin-2-yl]azetidin-1-yl]methyl]morpholin-4-yl]quinoline-8-carbonitrile and 5-[(2R,6S)-2-methyl-6-[[3-[(7S)-7-methyl-4,5,6,7-tetrahydropyrazolo[4,3-c]pyridin-2-yl]azetidin-1-yl]methyl]morpholin-4-yl]quinoline-8-carbonitrile

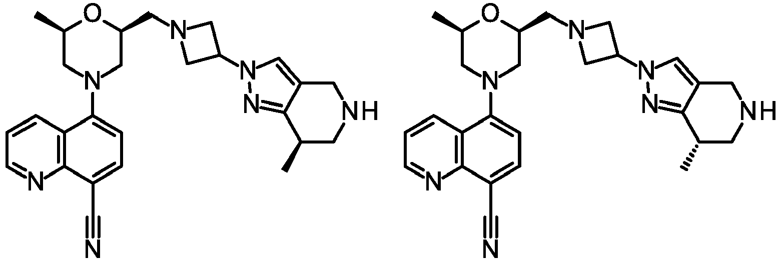

The mixture of title compounds **Example 25** and **Example 26** was prepared in analogy to the preparation of **Example 17** by using **Intermediate E** instead of **Intermediate F. Example 25** and **Example 26** were separated by chiral SFC (Gradient: 50% Isopropanol (0.1% NH₃H₂O) in CO₂, Column: ID, 250×20 mm I.D., 5µm).

**Example 25** (faster eluting, 12 mg) was obtained as a light yellow solid. MS: calc'd 458 (MH⁺), measured 458 (MH⁺). ¹H NMR (400 MHz, METHANOL-d4) δ = 9.00 (dd, *J* = 1.6, 4.2 Hz, 1H), 8.67 (d, *J* = 8.3 Hz, 1H), 8.17 (d, *J* = 7.8 Hz, 1H), 7.66 (dd, *J* = 4.6, 8.7 Hz, 1H), 7.53 (s, 1H), 7.27 (d, *J=* 8.3 Hz, 1H), 5.00 - 4.94 (m, 1H), 4.11 - 4.01 (m, 2H), 3.96 - 3.78 (m, 3H), 3.63 (br t, *J* = 7.3 Hz, 2H), 3.43 -3.37 (m, 2H), 3.01 - 2.93 (m, 2H), 2.86 - 2.72 (m, 4H), 2.70 - 2.59 (m, 2H), 1.29 (d, *J* = 7.0 Hz, 3H), 1.26 (d, *J* = 6.2 Hz, 3H).

**Example 26** (slower eluting, 7 mg) was obtained as a light yellow solid. MS: calc'd 458 (MH⁺), measured 458 (MH⁺). ¹H NMR (400 MHz, METHANOL-d4) δ = 9.00 (d, *J* = 3.1 Hz, 1H), 8.66 (d, *J* = 8.6 Hz, 1H), 8.17 (d, *J* = 8.2 Hz, 1H), 7.66 (dd, *J* = 4.5, 8.6 Hz, 1H), 7.53 (s, 1H), 7.27 (d, *J* = 8.1 Hz, 1H), 5.00 - 4.94 (m, 1H), 4.12 - 4.00 (m, 2H), 3.95 - 3.82 (m, 3H), 3.66 - 3.58 (m, 2H), 3.43 - 3.37 (m, 2H), 2.99 - 2.91 (m, 2H), 2.84 - 2.71 (m, 4H), 2.70 - 2.57 (m, 2H), 1.29 (d, *J* = 7.1 Hz, 3H), 1.26 (d, *J* = 6.2 Hz, 3H).

### Example 27 and Example 28

### 5-[(2R,6S)-2-methyl-6-[[3-[(5S)-5-methyl-4,5,6,7-tetrahydropyrazolo[3,4-c]pyridin-1-yl]azetidin-1-yl]methyl]morpholin-4-yl]quinoline-8-carbonitrile and 5-[(2R,6S)-2-methyl-6-[[3-[(5R)-5-methyl-4,5,6,7-tetrahydropyrazolo[3,4-c]pyridin-1-yl]azetidin-1-yl]methyl]morpholin-4-yl]quinoline-8-carbonitrile

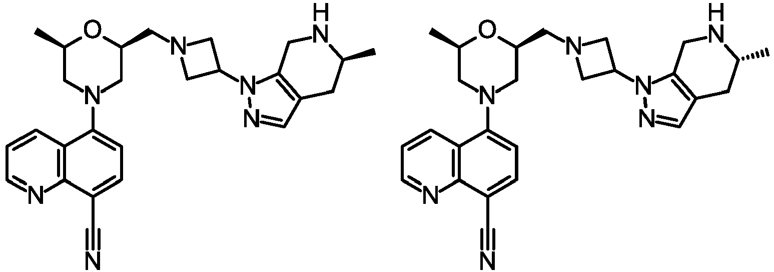

### Example 29 and Example 30

### 5-[(2R,6S)-2-methyl-6-[[3-[(5S)-5-methyl-4,5,6,7-tetrahydropyrazolo[3,4-c]pyridin-2-yl]azetidin-1-yl]methyl]morpholin-4-yl]quinoline-8-carbonitrile and 5-[(2R,6S)-2-methyl-6-[[3-[(5R)-5-methyl-4,5,6,7-tetrahydropyrazolo[3,4-c]pyridin-2-yl]azetidin-1-yl]methyl]morpholin-4-yl]quinoline-8-carbonitrile

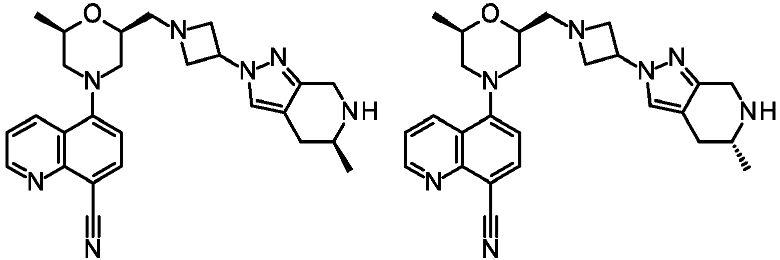

The mixture of title compounds **Example 27** and **Example 28,** and the mixture of title compounds **Example 29** and **Example 30** were prepared in analogy to the preparation of **Example 16** and **Example 17** by using **Intermediate** C instead of **Intermediate F.** The mixture of **Example 27** and **Example 28** and the mixture of **Example 29** and **Example 30** were separated by flash column in **step 1. Example 27** and **Example 28** were separated by chiral SFC (Gradient: 35% Isopropanol (0.1% NH₃H₂O) in CO₂, Column: AS, 250×20 mm I.D., 5µm). **Example 29** and **Example 30** were separated by chiral SFC (Gradient: 40% Isopropanol (0.1% NH₃H₂O) in CO₂, Column: Daicel chiralcel AD 250×30mm, 5µm).

**Example 27** (faster eluting, 9 mg) was obtained as a light yellow solid. MS: calc'd 458 (MH⁺), measured 458 (MH⁺). ¹H NMR (400 MHz, METHANOL-d4) δ = 9.01 - 8.94 (m, 1H), 8.64 (d, *J* = 7.0 Hz, 1H), 8.15 (d, *J* = 8.1 Hz, 1H), 7.64 (dd, *J* = 4.0, 8.7 Hz, 1H), 7.32 (s, 1H), 7.25 (d, *J* = 8.1 Hz, 1H), 5.27 - 5.10 (m, 1H), 4.09 - 3.94 (m, 3H), 3.92 - 3.81 (m, 3H), 3.65 (br t, *J* = 7.7 Hz, 2H), 3.41 - 3.34 (m, 2H), 2.93 - 2.83 (m, 1H), 2.81 - 2.77 (m, 2H), 2.77 - 2.69 (m, 1H), 2.67 - 2.57 (m, 2H), 2.28 - 2.18 (m, 1H), 1.23 (dd, *J* = 2.0, 6.3 Hz, 6H).

**Example 28** (slower eluting, 9 mg) was obtained as a light yellow solid. MS: calc'd 458 (MH⁺), measured 458 (MH⁺). ¹H NMR (400 MHz, METHANOL-d4) δ = 8.97 (dd, *J* = 1.7, 4.2 Hz, 1H), 8.64 (dd, *J* = 1.8, 8.6 Hz, 1H), 8.15 (d, *J* = 8.1 Hz, 1H), 7.64 (dd, *J* = 4.2, 8.6 Hz, 1H), 7.33 (s, 1H), 7.25 (d, *J* = 8.1 Hz, 1H), 5.23 - 5.14 (m, 1H), 4.09 - 3.94 (m, 3H), 3.94 - 3.81 (m, 3H), 3.71 - 3.60 (m, 2H), 3.41 - 3.35 (m, 2H), 2.93 - 2.83 (m, 1H), 2.82 - 2.69 (m, 3H), 2.67 - 2.57 (m, 2H), 2.29 - 2.18 (m, 1H), 1.24 (dd, *J* = 2.8, 6.2 Hz, 6H).

**Example 29** (slower eluting, 21 mg) was obtained as a light yellow solid. MS: calc'd 458 (MH⁺), measured 458 (MH⁺). ¹H NMR (400 MHz, METHANOL-d4) δ = 8.87 (dd, *J* = 1.3, 4.1 Hz, 1H), 8.53 (d, *J* = 8.6 Hz, 1H), 8.03 (d, *J* = 8.1 Hz, 1H), 7.53 (dd, *J* = 4.2, 8.6 Hz, 1H), 7.39 (s, 1H), 7.13 (d, *J* = 8.1 Hz, 1H), 4.90 - 4.80 (m, 1H), 4.01 - 3.71 (m, 6H), 3.50 (dt, *J* = 3.5, 7.5 Hz, 2H), 3.27 (br d, *J* = 12.2 Hz, 2H), 2.94 - 2.79 (m, 1H), 2.72 - 2.48 (m, 5H), 2.20 (dd, *J* = 10.5, 15.3 Hz, 1H), 1.15 (dd, *J* = 6.3, 9.5 Hz, 6H).

**Example 30** (faster eluting, 19 mg) was obtained as a light yellow solid. MS: calc'd 458 (MH⁺), measured 458 (MH⁺). ¹H NMR (400 MHz, METHANOL-d4) δ = 8.87 (dd, *J* = 1.7, 4.2 Hz, 1H), 8.54 (dd, *J* = 1.7, 8.6 Hz, 1H), 8.05 (d, *J=* 8.1 Hz, 1H), 7.54 (dd, *J* = 4.3, 8.6 Hz, 1H), 7.38 (s, 1H), 7.14 (d, *J* = 8.1 Hz, 1H), 4.89 - 4.80 (m, 1H), 4.00 - 3.71 (m, 6H), 3.50 (dt, *J=* 3.5, 7.5 Hz, 2H), 3.32 - 3.24 (m, 2H), 2.88 - 2.77 (m, 1H), 2.73 - 2.47 (m, 5H), 2.18 (dd, *J* = 10.4, 15.3 Hz, 1H), 1.14 (t, *J* = 5.9 Hz, 6H).

### Example 31 and Example 32

### 5-[(2R,6S)-2-methyl-6-[[3-[(7R)-7-methyl-4,5,6,7-tetrahydropyrazolo[3,4-c]pyridin-1-yl]azetidin-1-yl]methyl]morpholin-4-yl]quinoline-8-carbonitrile and 5-[(2R,6S)-2-methyl-6-[[3-[(7S)-7-methyl-4,5,6,7-tetrahydropyrazolo[3,4-c]pyridin-1-yl]azetidin-1-yl]methyl]morpholin-4-yl]quinoline-8-carbonitrile

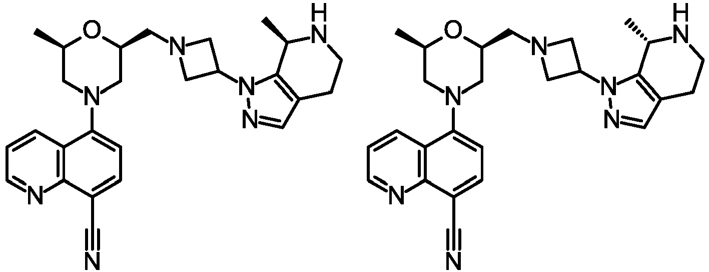

### Example 33 and Example 34

### 5-[(2R,6S)-2-methyl-6-[[3-[(7R)-7-methyl-4,5,6,7-tetrahydropyrazolo[3,4-c]pyridin-2-yl]azetidin-1-yl]methyl]morpholin-4-yl]quinoline-8-carbonitrile and 5-[(2R,6S)-2-methyl-6-[[3-[(7S)-7-methyl-4,5,6,7-tetrahydropyrazolo[3,4-c]pyridin-2-yl]azetidin-1-yl]methyl]morpholin-4-yl]quinoline-8-carbonitrile

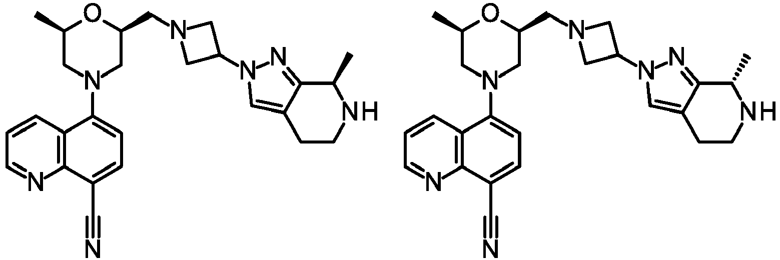

The mixture of title compounds **Example 31** and **Example 32**, and the mixture of title compounds **Example 33** and **Example 34** were prepared in analogy to the preparation of **Example 16** and **Example 17** by using **Intermediate D** instead of **Intermediate F.** The mixture of **Example 31** and **Example 32** (slower eluting) and the mixture of **Example 33** and **Example 34** (faster eluting) were separated by prep-HPLC (Gradient: 10-30% ACN in Water (0.1% TFA), Column: Waters SunFire C18, 30×100mm, 5µm). **Example 31** and **Example 32** were separated by chiral SFC (Gradient: 30% Methanol (0.1% NH₃H₂O) in CO₂, Column: AD, 250×20 mm I.D., 5µm). **Example 33** and **Example 34** were separated by chiral SFC (Gradient: 45% Isopropanol (0.1% NH₃H₂O) in CO₂, Column: ID, 250×20 mm I.D., 5µm).

**Example 31** (faster eluting, 6 mg) was obtained as a light yellow solid. MS: calc'd 458 (MH⁺), measured 458 (MH⁺). ¹H NMR (400 MHz, METHANOL-d4) δ = 8.97 (dd, *J* = 1.6, 4.3 Hz, 1H), 8.64 (dd, *J* = 1.7, 8.6 Hz, 1H), 8.15 (d, *J* = 8.1 Hz, 1H), 7.63 (dd, *J* = 4.3, 8.7 Hz, 1H), 7.35 (s, 1H), 7.25 (d, *J* = 8.1 Hz, 1H), 4.95 - 4.88 (m, 1H), 4.15 (q, *J* = 6.8 Hz, 1H), 4.09 - 3.97 (m, 2H), 3.92 - 3.83 (m, 2H), 3.82 - 3.74 (m, 1H), 3.58 (t, *J* = 7.9 Hz, 1H), 3.41 - 3.35 (m, 2H), 3.05 - 2.97 (m, 1H), 2.93 - 2.70 (m, 4H), 2.64 (dd, *J* = 10.2, 11.9 Hz, 1H), 2.53 (t, *J* = 5.8 Hz, 2H), 1.38 (d, *J* = 6.7 Hz, 3H), 1.23 (d, *J* = 6.2 Hz, 3H).

**Example 32** (slower eluting, 6 mg) was obtained as a light yellow solid. MS: calc'd 458 (MH⁺), measured 458 (MH⁺). ¹H NMR (400 MHz, METHANOL-d4) δ = 8.97 (dd, *J* = 1.7, 4.2 Hz, 1H), 8.64 (dd, *J* = 1.7, 8.6 Hz, 1H), 8.15 (d, *J* = 7.9 Hz, 1H), 7.64 (dd, *J* = 4.3, 8.6 Hz, 1H), 7.35 (s, 1H), 7.25 (d, *J* = 8.1 Hz, 1H), 4.95 - 4.87 (m, 1H), 4.15 (q, *J* = 6.6 Hz, 1H), 4.09 - 3.97 (m, 2H), 3.92 (dt, *J* = 2.2, 7.3 Hz, 1H), 3.84 (dt, *J* = 2.0, 7.3 Hz, 1H), 3.79 - 3.71 (m, 1H), 3.60 (t, *J* = 7.8 Hz, 1H), 3.41 - 3.35 (m, 2H), 3.08 - 2.95 (m, 1H), 2.94 - 2.84 (m, 1H), 2.84 - 2.77 (m, 2H), 2.74 (dd, *J* = 10.6, 11.8 Hz, 1H), 2.64 (dd, *J* = 10.2, 12.0 Hz, 1H), 2.53 (t, *J* = 5.7 Hz, 2H), 1.38 (d, *J* = 6.8 Hz, 3H), 1.23 (d, *J* = 6.2 Hz, 3H).

**Example 33** (slower eluting, 12 mg) was obtained as a light yellow solid. MS: calc'd 458 (MH⁺), measured 458 (MH⁺). ¹H NMR (400 MHz, METHANOL-d4) δ = 8.97 (dd, *J* = 1.7, 4.2 Hz, 1H), 8.64 (dd, *J* = 1.7, 8.6 Hz, 1H), 8.15 (d, *J* = 7.9 Hz, 1H), 7.63 (dd, *J* = 4.3, 8.7 Hz, 1H), 7.50 (s, 1H), 7.24 (d, *J* = 8.1 Hz, 1H), 4.99 - 4.90 (m, 1H), 4.09 - 3.81 (m, 5H), 3.60 (t, *J* = 7.5 Hz, 2H), 3.41 - 3.34 (m, 2H), 3.18 (ddd, *J* = 3.4, 5.3, 12.6 Hz, 1H), 2.88 - 2.52 (m, 7H), 1.44 (d, *J* = 6.7 Hz, 3H), 1.24 (d, *J* = 6.2 Hz, 3H).

**Example 34** (faster eluting, 12 mg) was obtained as a light yellow solid. MS: calc'd 458 (MH⁺), measured 458 (MH⁺). ¹H NMR (400 MHz, METHANOL-d4) δ = 8.97 (dd, *J* = 1.7, 4.2 Hz, 1H), 8.64 (dd, *J* = 1.7, 8.6 Hz, 1H), 8.15 (d, *J* = 7.9 Hz, 1H), 7.64 (dd, *J* = 4.3, 8.6 Hz, 1H), 7.50 (s, 1H), 7.25 (d, *J* = 8.1 Hz, 1H), 4.99 - 4.91 (m, 1H), 4.08 - 3.82 (m, 5H), 3.60 (q, *J* = 7.3 Hz, 2H), 3.40 - 3.34 (m, 2H), 3.22 - 3.10 (m, 1H), 2.87 - 2.70 (m, 4H), 2.68 - 2.53 (m, 3H), 1.44 (d, *J* = 6.6 Hz, 3H), 1.24 (d, *J* = 6.2 Hz, 3H).

### Example 35

### 5-[(2R,6S)-2-methyl-6-[[4-(4,5,6,7-tetrahydropyrazolo[4,3-c]pyridin-1-yl)-1-piperidyl]methyl]morpholin-4-yl]quinoline-8-carbonitrile

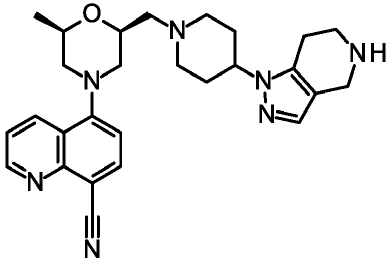

### Example 36

### 5-[(2R,6S)-2-methyl-6-[[4-(4,5,6,7-tetrahydropyrazolo[4,3-c]pyridin-2-yl)-1-piperidyl]methyl]morpholin-4-yl]quinoline-8-carbonitrile

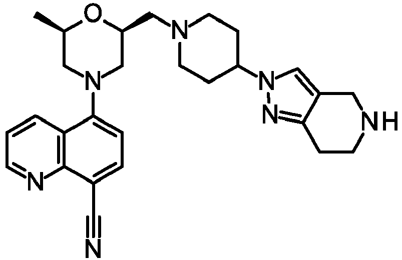

The title compounds were prepared in analogy to the preparation of **Example** 1 and **Example** 2 by using benzyl 4-bromopiperidine-1-carboxylate (CAS: 166953-64-6, Vendor: Bide) instead of benzyl 3-iodoazetidine-1-carboxylate (compound **1b**). **Example 35** and **Example 36** were separated by prep-HPLC (Gradient: 25-70% ACN in Water (0.1% NH₃), Column: Waters RPC18, 30×100mm, 5µm).

**Example 35** (faster eluting, 20 mg) was obtained as a pink solid. MS: calc'd 472 (MH⁺), measured 472 (MH⁺). ¹H NMR (400 MHz, METHANOL-d4) δ = 8.99 (dd, *J=* 1.6, 4.3 Hz, 1H), 8.67 (dd, *J* = 1.6, 8.6 Hz, 1H), 8.16 (d, *J* = 8.1 Hz, 1H), 7.65 (dd, *J* = 4.2, 8.6 Hz, 1H), 7.32 - 7.20 (m, 2H), 4.24 - 4.14 (m, 1H), 4.13 - 4.03 (m, 2H), 3.79 (s, 2H), 3.50 (br d, *J* = 11.9 Hz, 1H), 3.40 (br d, *J* = 11.9 Hz, 1H), 3.26 (br d, *J* = 10.9 Hz, 1H), 3.09 (br t, *J* = 5.8 Hz, 3H), 2.79 - 2.51 (m, 6H), 2.43 - 2.13 (m, 4H), 1.94 - 1.82 (m, 2H), 1.27 (d, *J* = 6.2 Hz, 3H).

**Example 36** (slower eluting, 28 mg) was obtained as a pink solid. MS: calc'd 472 (MH⁺), measured 472 (MH⁺). ¹H NMR (400 MHz, METHANOL-d4) δ = 9.00 (dd, *J* = 1.6, 4.2 Hz, 1H), 8.67 (dd, *J* = 1.6, 8.6 Hz, 1H), 8.17 (d, *J* = 8.1 Hz, 1H), 7.66 (dd, *J* = 4.2, 8.6 Hz, 1H), 7.43 (s, 1H), 7.27 (d, *J* = 8.1 Hz, 1H), 4.24 - 4.15 (m, 1H), 4.14 - 4.03 (m, 2H), 3.84 (s, 2H), 3.52 - 3.37 (m, 2H), 3.24 (br d, *J* = 13.2 Hz, 1H), 3.09 (br t, *J* = 5.9 Hz, 3H), 2.78 - 2.50 (m, 6H), 2.42 - 2.25 (m, 2H), 2.12 - 1.97 (m, 4H), 1.27 (d, *J* = 6.2 Hz, 3H).

### Example 37

### 5-[(2R,6S)-2-methyl-6-[[3-[3-(trifluoromethyl)-4,5,6,7-tetrahydropyrazolo[3,4-c]pyridin-1-yl]azetidin-1-yl]methyl]morpholin-4-yl]quinoline-8-carbonitrile

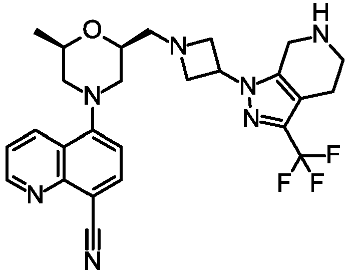

### Example 38

### 5-[(2R,6S)-2-methyl-6-[[3-[3-(trifluoromethyl)-4,5,6,7-tetrahydropyrazolo[3,4-c]pyridin-2-yl]azetidin-1-yl]methyl]morpholin-4-yl]quinoline-8-carbonitrile

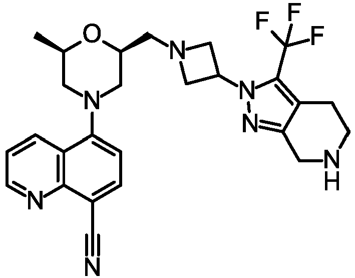

The title compounds were prepared in analogy to the preparation of **Example 1** and **Example 2** by using *tert*-butyl 3-(trifluoromethyl)-2,4,5,7-tetrahydropyrazolo[3,4-c]pyridine-6-carboxylate (CAS: 733757-89-6, Vendor: Shuya) instead of tert-butyl 2,4,6,7-tetrahydropyrazolo[4,3-c]pyridine-5-carboxylate (compound **1a**). **Example 37** and **Example 38** were separated by prep-HPLC (Gradient: 15-35% ACN in Water (0.1% TFA), Column: Waters SunFire C18, 30×100mm, 5µm).Similar to Example 1 and Example 2, the two products were also confirmed by NOESY.

**Example 37** (slower eluting, 57 mg) was obtained as a yellow solid. MS: calc'd 512 (MH⁺), measured 512 (MH⁺). ¹H NMR (400 MHz, METHANOL-d4) δ = 9.01 (dd, *J* = 1.6, 4.2 Hz, 1H), 8.67 (dd, *J* = 1.6, 8.6 Hz, 1H), 8.18 (d, *J* = 8.1 Hz, 1H), 7.67 (dd, *J* = 4.2, 8.6 Hz, 1H), 7.30 (d, *J* = 8.1 Hz, 1H), 5.45 (quin, *J* = 7.1 Hz, 1H), 5.05 - 4.95 (m, 2H), 4.81 - 4.65 (m, 2H), 4.52 (s, 2H), 4.39 (br t, *J* = 9.8 Hz, 1H), 4.24 - 4.08 (m, 1H), 3.74 - 3.56 (m, 2H), 3.52 (t, *J* = 6.2 Hz, 2H), 3.48 - 3.39 (m, 2H), 3.01 (br t, J = 5.9 Hz, 2H), 2.83 (dd, *J* = 10.5, 11.8 Hz, 1H), 2.74 (dd, *J* = 10.4, 12.1 Hz, 1H), 1.30 (d, *J* = 6.2 Hz, 3H).

**Example 38** (faster eluting, 12 mg) was obtained as a yellow solid. MS: calc'd 512 (MH⁺), measured 512 (MH⁺). ¹H NMR (400 MHz, METHANOL-d4) δ = 9.01 (dd, *J* = 1.7, 4.2 Hz, 1H), 8.67 (dd, *J* = 1.6, 8.6 Hz, 1H), 8.18 (d, *J* = 7.9 Hz, 1H), 7.67 (dd, *J* = 4.3, 8.6 Hz, 1H), 7.30 (d, *J* = 8.1 Hz, 1H), 5.68 - 5.53 (m, 1H), 5.02 - 4.77 (m, 4H), 4.47 (s, 2H), 4.42 - 4.31 (m, 1H), 4.21 - 4.08 (m, 1H), 3.72 - 3.58 (m, 2H), 3.55 (t, *J* = 6.2 Hz, 2H), 3.44 (br d, *J* = 12.5 Hz, 2H), 3.08 (br t, *J* = 5.6 Hz, 2H), 2.87 - 2.78 (m, 1H), 2.73 (dd, *J* = 10.4, 12.2 Hz, 1H), 1.30 (d, *J* = 6.2 Hz, 3H).

### Example 42

### 5-[(2R,6S)-2-methyl-6-[[3-(3-methyl-4,5,6,7-tetrahydropyrazolo[3,4-c]pyridin-1-yl)azetidin-1-yl]methyl]morpholin-4-yl]quinoline-8-carbonitrile

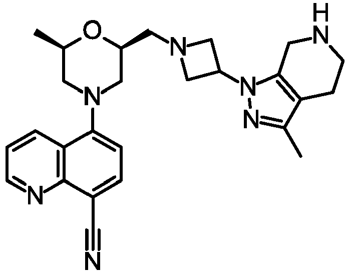

### Example 43

### 5-[(2R,6S)-2-methyl-6-[[3-(3-methyl-4,5,6,7-tetrahydropyrazolo[3,4-c]pyridin-2-yl)azetidin-1-yl]methyl]morpholin-4-yl]quinoline-8-carbonitrile

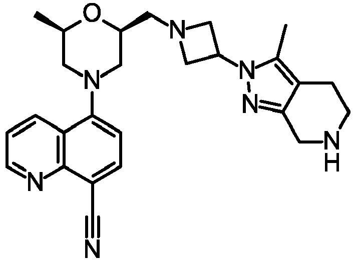

The title compounds were prepared in analogy to the preparation of **Example 16** and **Example 17** by using **Intermediate J** instead of **Intermediate F. Example 42** and **Example 43** were separated by prep-HPLC (Gradient: 10-30% ACN in Water (0.1% TFA), Column: Waters SunFire C18, 30×100mm, 5µm).Similar to Example 1 and Example 2, the two products were also confirmed by NOESY.

**Example 42** (slower eluting, 23 mg) was obtained as a yellow solid. MS: calc'd 458 (MH⁺), measured 458 (MH⁺). ¹H NMR (400 MHz, METHANOL-d4) δ = 9.01 (dd, *J* = 1.6, 4.3 Hz, 1H), 8.68 (dd, *J* = 1.5, 8.6 Hz, 1H), 8.19 (d, *J=* 8.1 Hz, 1H), 7.67 (dd, *J* = 4.3, 8.6 Hz, 1H), 7.31 (d, *J* = 8.1 Hz, 1H), 5.32 - 5.18 (m, 1H), 5.00 - 4.84 (m, 2H), 4.74 - 4.57 (m, 2H), 4.47 - 4.32 (m, 3H), 4.22 - 4.08 (m, 1H), 3.81 - 3.55 (m, 2H), 3.53 - 3.40 (m, 4H), 2.89 - 2.78 (m, 3H), 2.74 (dd, *J* = 10.4, 12.1 Hz, 1H), 2.28 (s, 3H), 1.31 (d, *J=* 6.2 Hz, 3H).

**Example 43** (faster eluting, 24 mg) was obtained as a yellow solid. MS: calc'd 458 (MH⁺), measured 458 (MH⁺). ¹H NMR (400 MHz, METHANOL-d4) δ = 9.02 (dd, *J* = 1.6, 4.3 Hz, 1H), 8.68 (dd, *J* = 1.5, 8.6 Hz, 1H), 8.19 (d, *J* = 7.9 Hz, 1H), 7.67 (dd, *J* = 4.2, 8.6 Hz, 1H), 7.31 (d, *J* = 8.1 Hz, 1H), 5.54 - 5.39 (m, 1H), 4.99 - 4.79 (m, 2H), 4.75 - 4.55 (m, 2H), 4.45 - 4.29 (m, 3H), 4.23 - 4.05 (m, 1H), 3.78 - 3.55 (m, 2H), 3.50 (t, *J* = 6.2 Hz, 2H), 3.47 - 3.41 (m, 2H), 2.90 - 2.78 (m, 3H), 2.73 (dd, *J* = 10.3, 12.2 Hz, 1H), 2.27 (s, 3H), 1.31 (d, *J* = 6.2 Hz, 3H).

### Example 44

The following tests were carried out in order to determine the activity of the compounds of formula (I) and (Ia) in HEK293-Blue-hTLR-7/8/9 cells assay.

### HEK293-Blue-hTLR-7 cells assay:

A stable HEK293-Blue-hTLR-7 cell line was purchased from InvivoGen (Cat.#: hkb-htlr7, San Diego, California, USA). These cells were originally designed for studying the stimulation of human TLR7 by monitoring the activation of NF-κB. A SEAP (secreted embryonic alkaline phosphatase) reporter gene was placed under the control of the IFN-β minimal promoter fused to five NF-κB and AP-1-binding sites. The SEAP was induced by activating NF-κB and AP-1 *via* stimulating HEK-Blue hTLR7 cells with TLR7 ligands. Therefore the reporter expression was declined by TLR7 antagonist under the stimulation of a ligand, such as R848 (Resiquimod), for incubation of 20 hrs. The cell culture supernatant SEAP reporter activity was determined using QUANTI-Blue^{™} kit (Cat.#: rep-qb1, Invivogen, San Diego, Ca, USA) at a wavelength of 640 nm, a detection medium that turns purple or blue in the presence of alkaline phosphatase.

HEK293-Blue-hTLR7 cells were incubated at a density of 250,000-450,000 cells/mL in a volume of 170 µL in a 96-well plate in Dulbecco's Modified Eagle's medium (DMEM) containing 4.5 g/L glucose, 50 U/mL penicillin, 50 mg/mL streptomycin, 100 mg/mL Normocin, 2 mM L-glutamine, 10% (v/v) heat-inactivated fetal bovine serum with addition of 20 µL test compound in a serial dilution in the presence of final DMSO at 1% and 10 µL of 20uM R848 in above DMEM, perform incubation under 37 °C in a CO₂ incubator for 20 hrs. Then 20 µL of the supernatant from each well was incubated with 180 µL Quanti-blue substrate solution at 37 °C for 2 hrs and the absorbance was read at 620-655 nm using a spectrophotometer. The signaling pathway that TLR7 activation leads to downstream NF-κB activation has been widely accepted, and therefore similar reporter assay was modified for evaluating TLR7 antagonist.

### HEK293-Blue-hTLR-8 cells assay:

A stable HEK293-Blue-hTLR-8 cell line was purchased from InvivoGen (Cat.#: hkb-htlr8, San Diego, California, USA). These cells were originally designed for studying the stimulation of human TLR8 by monitoring the activation of NF-κB. A SEAP (secreted embryonic alkaline phosphatase) reporter gene was placed under the control of the IFN-β minimal promoter fused to five NF-κB and AP-1-binding sites. The SEAP was induced by activating NF-κB and AP-1 *via* stimulating HEK-Blue hTLR8 cells with TLR8 ligands. Therefore the reporter expression was declined by TLR8 antagonist under the stimulation of a ligand, such as R848, for incubation of 20 hrs. The cell culture supernatant SEAP reporter activity was determined using QUANTI-Blue^{™} kit (Cat.#: rep-qb1, Invivogen, San Diego, Ca, USA) at a wavelength of 640 nm, a detection medium that turns purple or blue in the presence of alkaline phosphatase.

HEK293-Blue-hTLR8 cells were incubated at a density of 250,000-450,000 cells/mL in a volume of 170 µL in a 96-well plate in Dulbecco's Modified Eagle's medium (DMEM) containing 4.5 g/L glucose, 50 U/mL penicillin, 50 mg/mL streptomycin, 100 mg/mL Normocin, 2 mM L-glutamine, 10% (v/v) heat-inactivated fetal bovine serum with addition of 20 µL test compound in a serial dilution in the presence of final DMSO at 1% and 10 µL of 60uM R848 in above DMEM, perform incubation under 37 °C in a CO₂ incubator for 20 hrs. Then 20 µL of the supernatant from each well was incubated with 180 µL Quanti-blue substrate solution at 37°C for 2 hrs and the absorbance was read at 620-655 nm using a spectrophotometer. The signaling pathway that TLR8 activation leads to downstream NF-κB activation has been widely accepted, and therefore similar reporter assay was modified for evaluating TLR8 antagonist.

### HEK293-Blue-hTLR-9 cells assay:

A stable HEK293-Blue-hTLR-9 cell line was purchased from InvivoGen (Cat.#: hkb-htlr9, San Diego, California, USA). These cells were originally designed for studying the stimulation of human TLR9 by monitoring the activation of NF-κB. A SEAP (secreted embryonic alkaline phosphatase) reporter gene was placed under the control of the IFN-β minimal promoter fused to five NF-κB and AP-1-binding sites. The SEAP was induced by activating NF-κB and AP-1 *via* stimulating HEK-Blue hTLR9 cells with TLR9 ligands. Therefore the reporter expression was declined by TLR9 antagonist under the stimulation of a ligand, such as ODN2006 (Cat.#: tlrl-2006-1, Invivogen, San Diego, California, USA), for incubation of 20 hrs. The cell culture supernatant SEAP reporter activity was determined using QUANTI-Blue^{™} kit (Cat.#: rep-qb1, Invivogen, San Diego, California, USA) at a wavelength of 640 nm, a detection medium that turns purple or blue in the presence of alkaline phosphatase.

HEK293-Blue-hTLR9 cells were incubated at a density of 250,000-450,000 cells/mL in a volume of 170 µL in a 96-well plate in Dulbecco's Modified Eagle's medium (DMEM) containing 4.5 g/L glucose, 50 U/mL penicillin, 50 mg/mL streptomycin, 100 mg/mL Normocin, 2 mM L-glutamine, 10% (v/v) heat-inactivated fetal bovine serum with addition of 20 µL test compound in a serial dilution in the presence of final DMSO at 1% and 10 µL of 20uM ODN2006 in above DMEM, perform incubation under 37 °C in a CO₂ incubator for 20 hrs. Then 20 µL of the supernatant from each well was incubated with 180 µL Quanti-blue substrate solution at 37 °C for 2 h and the absorbance was read at 620-655 nm using a spectrophotometer. The signaling pathway that TLR9 activation leads to downstream NF-κB activation has been widely accepted, and therefore similar reporter assay was modified for evaluating TLR9 antagonist.

The compounds of formula (I) or (Ia) have human TLR7 and/or TLR8 inhibitory activities (IC₅₀ value) <0.5 µM. Moreover, some compounds also have human TLR9 inhibitory activity <0.5µM. Activity data of the compounds of the present invention were shown in Table 2.

**Table 2. The activity of the compounds of present invention in HEK293-Blue-hTLR-7/8/9 cells assays**

| Example No | HEK/hTLR7 IC₅₀ (µM) | HEK/hTLR8 IC₅₀ (µM) | HEK/hTLR9 IC₅₀ (µM) |
|---|---|---|---|
| **R1 (reference compound)** | 0.095 | 0.142 | 6.623 |
| **R2 (reference compound)** | 0.210 | 0.342 | 7.034 |
| **1** | 0.010 | 0.017 | 0.057 |
| **2** | 0.025 | 0.019 | 0.066 |
| **3** | 0.003 | 0.006 | 0.051 |
| **4** | 0.009 | 0.009 | <0.032 |
| **5** | 0.004 | 0.006 | 0.137 |
| **6** | 0.012 | 0.005 | 0.153 |
| **7** | 0.008 | 0.005 | 0.116 |
| **8** | 0.018 | 0.005 | 0.125 |
| **9** | 0.012 | 0.007 | 0.172 |
| **10** | 0.016 | 0.019 | 0.143 |
| **11** | 0.017 | 0.019 | 0.049 |
| **12** | 0.015 | 0.044 | 0.078 |
| **13** | 0.014 | 0.045 | 0.118 |
| **14** | 0.026 | 0.010 | 0.276 |
| **15** | 0.009 | 0.009 | 0.128 |
| **16** | 0.004 | 0.016 | 0.078 |
| **17** | 0.004 | 0.018 | 0.088 |
| **18** | 0.017 | 0.019 | 0.147 |
| **19** | 0.019 | 0.022 | 0.122 |
| **20** | 0.005 | 0.028 | 0.076 |
| **21** | 0.018 | 0.072 | 0.080 |
| **22** | 0.005 | 0.037 | 0.070 |
| **23** | 0.013 | 0.044 | 0.084 |
| **24** | 0.004 | 0.014 | 0.098 |
| **25** | 0.004 | 0.011 | 0.082 |
| **26** | 0.006 | 0.012 | 0.089 |
| **27** | 0.006 | 0.008 | 0.065 |
| **28** | 0.005 | 0.010 | 0.071 |
| **29** | 0.008 | 0.011 | 0.072 |
| **30** | 0.005 | 0.014 | 0.057 |
| **31** | <0.003 | 0.005 | 0.061 |
| **32** | <0.003 | 0.010 | 0.062 |
| **33** | 0.007 | 0.013 | 0.034 |
| **34** | 0.006 | 0.016 | 0.036 |
| **35** | 0.005 | 0.025 | 0.082 |
| **36** | 0.018 | 0.040 | 0.055 |
| **42** | 0.007 | 0.018 | 0.134 |
| **43** | 0.017 | 0.034 | 0.083 |

### Example 45

### hERG channel inhibition assay:

The hERG channel inhibition assay is a highly sensitive measurement that identifies compounds exhibiting hERG inhibition related to cardiotoxicity in vivo. The hERG K⁺ channels were cloned in humans and stably expressed in a CHO (Chinese hamster ovary) cell line. CHO_{hERG} cells were used for patch-clamp (voltage-clamp, whole-cell) experiments. Cells were stimulated by a voltage pattern to activate hERG channels and conduct I_{KhERG} currents (rapid delayed outward rectifier potassium current of the hERG channel). After the cells were stabilized for a few minutes, the amplitude and kinetics of I_{KhERG} were recorded at a stimulation frequency of 0.1 Hz (6 bpm). Thereafter, the test compound was added to the preparation at increasing concentrations. For each concentration, an attempt was made to reach a steady-state effect, usually, this was achieved within 3-10 min at which time the next highest concentration was applied. The amplitude and kinetics of I_{KhERG} are recorded in each concentration of the drug which were compared to the control values (taken as 100%). (references: Redfern WS, Carlsson L, Davis AS, Lynch WG, MacKenzie I, Palethorpe S, Siegl PK, Strang I, Sullivan AT, Wallis R, Camm AJ, Hammond TG. 2003; Relationships between preclinical cardiac electrophysiology, clinical QT interval prolongation and torsade de pointes for a broad range of drugs: evidence for a provisional safety margin in drug development. Cardiovasc. Res. 58:32-45, Sanguinetti MC, Tristani-Firouzi M. 2006; hERG potassium channels and cardiac arrhythmia. Nature 440:463-469, Webster R, Leishman D, Walker D. 2002; Towards a drug concentration effect relationship for QT prolongation and torsades de pointes. Curr. Opin. Drug Discov. Devel. 5:116-26).

Results of hERG are given in Table 3. A safety ratio (hERG IC₂₀ /EC₅₀) > 30 suggests a sufficient window to differentiate the pharmacology by inhibiting TLR7/8/9 pathways from the potential hERG related cardiotoxicity. According to the calculation of hERG IC₂₀ / TLR7/8/9 IC₅₀ below which serves as early selectivity index to assess hERG liability, obviously reference compounds ER-887258, ER-888285, ER-888286, R1 and R2 have much narrower safety window compared to the compounds of this invention.

**Table 3. hERG and safety ratio results**

| Example No | hERG IC₂₀ (µM) | hERG IC₅₀ (µM) | hERG IC₂₀/ TLR7 IC₅₀ | hERG IC₂₀ / TLR8 IC₅₀ | hERG IC₂₀ / TLR9 IC₅₀ |
|---|---|---|---|---|---|
| **ER-887258** | 0.687 | 2.784 | 8.1 | N.A. | 0.3 |
| **ER-888285** | 1.006 | 3.105 | 8.4 | N.A. | 0.5 |
| **ER-888286** | 0.348 | 1.297 | 0.3 | N.A. | 0.2 |
| **R1** | 0.879 | 2.745 | 9.3 | 6.2 | 0.1 |
| **R2** | 0.280 | 0.770 | 1.3 | 0.8 | 0.04 |
| **1** | >10.0 | >20.0 | >1023.8 | >584.4 | >174.0 |
| **2** | >10.0 | >20.0 | >403.5 | >538.4 | >151.6 |
| **3** | >10.0 | >20.0 | >2901.3 | >1739.2 | >194.9 |
| **4** | >10.0 | >20.0 | >1136.4 | >1162.8 | >314.5 |
| **5** | >10.0 | >20.0 | >2857.1 | >1653.6 | >73.1 |
| **11** | >10.0 | >20.0 | >602.4 | >531.9 | >204.5 |
| **12** | >10.0 | >20.0 | >666.7 | >226.8 | >127.6 |
| **13** | >10.0 | >20.0 | >694.4 | >222.2 | >84.5 |
| **16** | >10.0 | >20.0 | >2381.0 | >641.0 | >129.0 |
| **29** | >10.0 | >20.0 | >1265.8 | >877.2 | >138.3 |
| **30** | >10.0 | >20.0 | >2083.3 | >735.3 | >175.7 |
| **33** | >10.0 | >20.0 | >1369.9 | >763.4 | >291.5 |
| **35** | >10.0 | >20.0 | >1924.5 | >397.8 | >121.4 |
| **36** | 10.0 | >20.0 | 566.6 | 246.7 | 180.7 |

### Example 46

The compounds would be desirable to have minimal DDI (drug-drug interaction) liabilities. Therefore, the effects of compounds of formula (I) or (Ia) on CYP2D6 are determined.

### CYP inhibition assay

This is a high throughput screening assay used for assessment of reversible inhibition of CYP2D6 activity of test compounds in human liver microsome (HLM) in early discovery stage.

**Table 4. Chemicals and materials used in the CYP inhibition assay**

| **Substances** | **Description** | **Source** | **Cat. No.** | **Final Concentration in incubation** |
|---|---|---|---|---|
| Human Liver Microsomes | | BD-Gentest | 452117 | 0.2 mg/mL |
| Dextromethorphan | CYP2D6 substrate | Sigma | D-2531 | 5 µM |
| Dextrorphan | CYP2D6 product | | | |
| Dextrorphan-D3 | CYP2D6 internal standard | Promochem | CERD-041 | |
| Quinidine | CYP2D6 inhibitor | | | 0.5 µM |

### Procedure

10 mM DMSO stock solutions of test compounds were diluted in DMSO to generate 2 mM intermediate stock solution. 250 nL of intermediate stock solution were transferred in duplicate into 3 separate 384 well microtitre plates (assay-ready plates). A mixture of HLM and each substrate was made up. 45 µL of HLM substrate mix was then transferred to each well of an assay ready plate and mixed. The negative (solvent) and positive control (standard inhibitor for CYP 2D6) were included in each assay ready plate. The assay ready plate was warmed to 37°C in an incubator over 10 minutes. 5 µL pre-warmed NADPH regenerating system was added to each incubation well to start the reaction. Final incubation volume was 50 µL. The assay plate then was placed back in the 37 °C incubator. After 10 minutes incubation, incubates were quenched by addition of 50 µL 100% acetonitrile containing internal standards (20 ng/mL D3-Dextrorphan). The supernatants were collected for RapidFire/MS/MS analysis.

RapidFire online solid phase extraction/sample injection system (Agilent) coupled with API4000 triple quadrupole mass spectrometer (AB Sciex) were used for sample analysis. The mobile phase composed of acetonitrile and water supplemented with 0.1% formic acid. A C4 solid phase extraction cartridge is used for sample separation. MS detection is achieved in positive ion MRM mode.

### Data analysis

Peak areas for substrate, metabolite and internal standard are determined using the RapidFire integrator software (version 3.6.12009.12296). Peak area ratios (PAR) of metabolite and internal standard (stable-labelled metabolite) are then calculated. The measurement window for each experiment is then defined:
PAR (0% activity)= average PAR for all incubations containing concentrated inhibitor;
Par (100% activity)= average PAR for all incubations containing no inhibitor (DMSO controls);
% Activity (test inhibitor)
=[PAR(test inhibitor)-PAR(0% activity)]/[PAR(100% activity)-PAR(0% activity)];
% Inhibition (test inhibitor)=100-% Activity (test inhibitor).

The compounds of present invention were found to have low CYP inhibition for CYP2D6 determined in the assays described above.

**Table 5. CYP inhibition of the compounds of this invention for CYP2D6**

| Example No | CYP 2D6 inhibition % @ 10µM |
|---|---|
| **ER-888286** | 52.5 |
| **2** | 1.0 |
| **3** | 10.5 |
| **4** | 7.5 |
| **5** | -1.5 |
| **6** | 8.5 |
| **7** | -19.5 |
| **8** | 11.0 |
| **9** | 15.0 |
| **11** | -6.0 |
| **12** | 6.0 |
| **13** | 3.0 |
| **15** | -10.5 |
| **16** | 8.0 |
| **17** | 1.5 |
| **18** | -2.5 |
| **19** | -2.5 |
| **20** | -6.0 |
| **21** | 10.5 |
| **22** | 5.5 |
| **23** | 3.0 |
| **26** | 20.0 |
| **27** | 22.0 |
| **28** | 0.5 |
| **30** | 3.5 |
| **31** | -3.5 |
| **32** | 11.0 |
| **34** | -11.5 |
| **36** | 20.3 |
| **42** | 17.5 |

| | |
|---|---|
| percentage inhibition <0: not or weak inhibitor | |

### Example 47

### Human PBMC Cell-Based Assay

Unlike the HEK reporter cell lines, human peripheral blood mononuclear cell (PBMC) represents primary human immune cells in blood mainly consisting of lymphocytes, monocytes, and dendritic cells. These cells express TLR7, TLR8, or TLR9, and therefore are natural responders to respective ligand stimulation. Upon activation of these TLRs, PBMCs secrete similar cytokines and chemokines in vitro and in vivo, and therefore the in vitro potency of a TLR7/8/9 antagonist in human PBMC is readily translatable to its pharmacodynamics response in vivo.

Human peripheral blood mononuclear cells (PBMC) were isolated from freshly-drawn lithium-heparinized (Lithium Heparin Plus blood Collection tube, BD Vacutainer^{®}) healthy donor whole blood by density gradient (Ficoll-PaqueTM PLUS, GE Healthcare life Sciences). Briefly, 50mL of blood was diluted with 25mL PBS (without Ca²⁺, Mg²⁺) in a 50mL conical tube with porous barrier (Leucosep tube, Greiner bio-one), where 15.5mL Ficoll-Paque was under laid after spinning. Tubes were centrifuged for 20 minutes at 800×g (1946 rpm) with the brake in the off position, and PBMC were collected from the buffy coat. Cells were then washed twice in PBS, and red blood cells were lysed by suspension in 2mL (Red Blood Cell Lysis Buffer, Alfa Aesar) for 5-10 minutes at room temperature. After a final wash in PBS, PBMC were resuspended at a final concentration of 2×10⁶ cells/mL in RPMI-1640 media with GlutaMAXTM (Gibco) supplemented with 10% Fetal Bovine Serum (Sigma) and plated at 150µL/well (3×10⁵ cells/well) in tissue culture treated round bottom 96-well plates (Corning Incorporated). Antagonist compounds (compounds of this invention) solubilized and serial diluted in 100% DMSO were added in duplicate to cells to yield a final concentration of 1% DMSO (v/v). PBMC were incubated with antagonist compounds for 30 minutes at 37°C, 5% CO₂ before adding various TLR agonist reagents in 48 µL complete media per well as follows (final concentrations indicated): CpG ODN 2216 (InvivoGen) at 1µM for TLR9, ORN 06/LyoVec (InvivoGen) at 1µg/mL for TLR8 and R848 (InvivoGen) at 1µg/mL for TLR7 and TLR8. PBMC were incubated overnight at 37°C with 5% CO₂. Cell culture supernatants were collected, and levels of various human cytokines were assessed by Luminex assay (ProcartaPlexTM Multiplex Immunoassay, Invitrogen) or ELISA procedure according to the manufacturer's recommended protocol (eBioscience, ThermoFisher Scientific). Viability of the cells was also checked with Cell Viability Assay (CellTiter Glo^{®}Luminescent Cell Viability Assay, Promega).

**Table 6. hPBMC results**

| Example No | hPBMC/TLR9 IC₅₀ (µM) |
|---|---|
| ER-888286 | 1.629 |
| **3** | 0.118 |
| **4** | 0.198 |
| **5** | 0.318 |
| **7** | 0.195 |
| **12** | 0.092 |
| **13** | 0.119 |
| **15** | 0.110 |
| **16** | 0.084 |
| **17** | 0.113 |
| **20** | 0.097 |
| **21** | 0.092 |
| **23** | 0.143 |
| **25** | 0.346 |
| **27** | 0.081 |
| **28** | 0.214 |
| **29** | 0.115 |
| **30** | 0.162 |
| **31** | 0.235 |
| **32** | 0.145 |
| **33** | 0.072 |
| **34** | 0.202 |

### Example 48

### Human microsome stability assay

The human microsomal stability assay is used for early assessment of metabolic stability of a test compound in human liver microsomes.

Human liver microsomes (Cat.NO.: 452117, Corning, USA;Cat.NO.:H2610, Xenotech, USA) were preincubated with test compound for 10 minutes at 37°C in 100 mM potassium phosphate buffer, pH 7.4. The reactions were initiated by adding NADPH regenerating system. The final incubation mixtures contained 1 µM test compound, 0.5 mg/mL liver microsomal protein, 1 mM MgCl₂, 1 mM NADP, 1 unit/mL isocitric dehydrogenase and 6 mM isocitric acid in 100 mM potassium phosphate buffer, pH 7.4. After incubation times of 0, 3, 6, 9, 15 and 30 minutes at 37°C, 300 µL of cold acetonitrile (including internal standard) was added to 100 µL incubation mixture to terminate the reaction. Following precipitation and centrifugation, the amount of compound remaining in the samples were determined by LC-MS/MS. Controls of no NADPH regenerating system at zero and 30 minutes were also prepared and analyzed. The compounds of present invention showed good human liver microsome stability determined in the above assay, results are shown in Table 7 below.

**Table 7. Human liver microsome stability of the compounds of present invention**

| Example No | Clearance of Human microsome (mL/min/kg) |
|---|---|
| **ER-887258** | 17.86 |
| **ER-888285** | 17.15 |
| **R2** | 15.43 |
| **1** | 6.2* |
| **2** | 6.2 |
| **4** | 6.2 |
| **5** | 6.2 |
| **11** | 6.2 |
| **16** | 7.2 |
| **17** | 6.2 |
| **18** | 7.0 |
| **19** | 6.2 |
| **20** | 6.2 |
| **21** | 6.2 |
| **22** | 6.2 |
| **23** | 6.2 |
| **28** | 6.2 |
| **29** | 6.6 |
| **30** | 6.2 |
| **34** | 6.4 |
| **35** | 7.7 |
| **36** | 6.2 |
| **42** | 7.1 |
| **43** | 6.5 |

| | |
|---|---|
| ^{∗} detection limit | |

## Claims

1. A compound of formula (I), 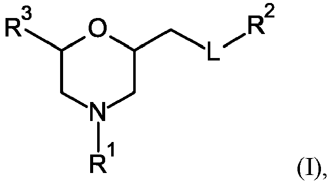 wherein
R¹ is 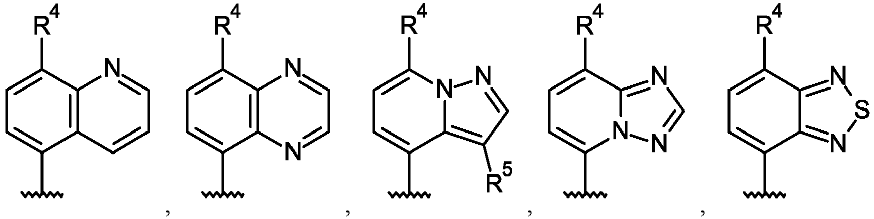 or 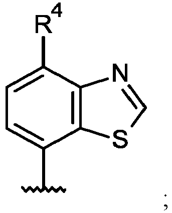 wherein R⁴ is C₁₋₆alkyl, halogen or cyano; R⁵ is halogen;
R² is 4,5,6,7-tetrahydropyrazolo[3,4-c]pyridinyl which is unsubstituted or substituted by C₁₋₆alkyl or haloC₁₋₆alkyl;
4,5,6,7-tetrahydropyrazolo[4,3-c]pyridinyl which is unsubstituted or substituted one or two times by C₁₋₆alkyl; or
5,6-dihydro-4H-pyrrolo[3,4-c]pyrazolyl;
R³ is C₁₋₆alkyl;
L is azetidinyl or piperidinyl;
or a pharmaceutically acceptable salt thereof.

2. A compound according to claim 1, wherein the compound is of formula (Ia), 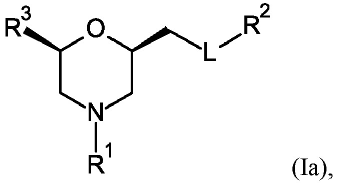 wherein
R¹ is 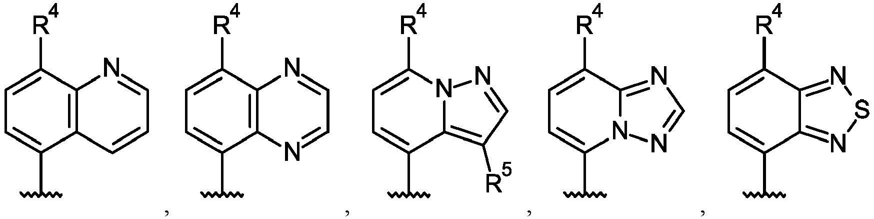 or 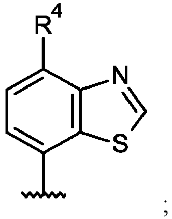 wherein R⁴ is C₁₋₆alkyl, halogen or cyano; R⁵ is halogen;
R² is 4,5,6,7-tetrahydropyrazolo[3,4-c]pyridinyl which is unsubstituted or substituted by C₁₋₆alkyl or haloC₁₋₆alkyl;
4,5,6,7-tetrahydropyrazolo[4,3-c]pyridinyl which is unsubstituted or substituted one or two times by C₁₋₆alkyl; or
5,6-dihydro-4H-pyrrolo[3,4-c]pyrazolyl;
R³ is C₁₋₆alkyl;
L is azetidinyl or piperidinyl;
or a pharmaceutically acceptable salt thereof.

3. A compound according to claim 1 or 2, or a pharmaceutically acceptable salt, wherein
R¹ is 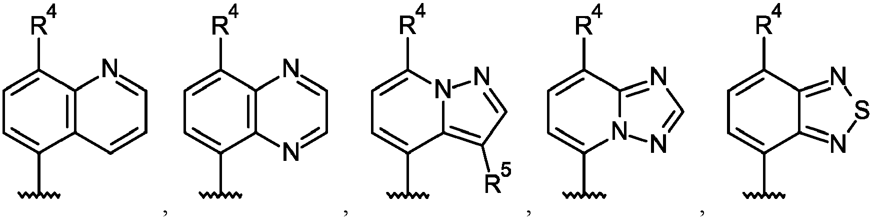 or 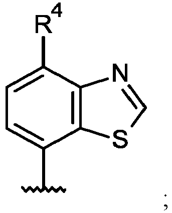 wherein R⁴ is methyl, chloro or cyano; R⁵ is fluoro;
R² is 4,5,6,7-tetrahydropyrazolo[3,4-c]pyridinyl which is unsubstituted or substituted by methyl, ethyl, isopropyl or trifluoromethyl;
4,5,6,7-tetrahydropyrazolo[4,3-c]pyridinyl which is unsubstituted or substituted one or two times by methyl; or
5,6-dihydro-4H-pyrrolo[3,4-c]pyrazolyl;
R³ is methyl;
L is azetidinyl or piperidinyl;
or a pharmaceutically acceptable salt thereof.

4. A compound according to claim 1 or 2, or a pharmaceutically acceptable salt thereof, wherein R¹ is 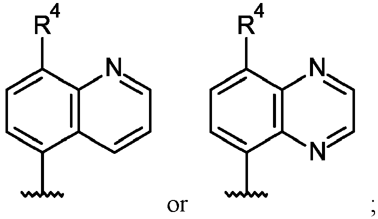 wherein R⁴ is cyano.

5. A compound according to claim 4, or a pharmaceutically acceptable salt thereof, wherein L is azetidinyl.

6. A compound according to claim 5, or a pharmaceutically acceptable salt thereof, wherein R² is 4,5,6,7-tetrahydropyrazolo[3,4-c]pyridinyl substituted by C₁₋₆alkyl; or 4,5,6,7-tetrahydropyrazolo[4,3-c]pyridinyl substituted by C₁₋₆alkyl.

7. A compound according to claim 6, or a pharmaceutically acceptable salt thereof, wherein R² is 4,5,6,7-tetrahydropyrazolo[3,4-c]pyridinyl substituted by methyl; or 4,5,6,7-tetrahydropyrazolo[4,3-c]pyridinyl substituted by methyl.

8. A compound according to claim 1 or 2, wherein
R¹ is 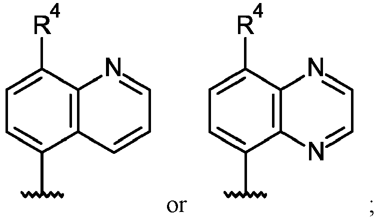 wherein R⁴ is cyano;
R² is 4,5,6,7-tetrahydropyrazolo[3,4-c]pyridinyl substituted by C₁₋₆alkyl; or 4,5,6,7-tetrahydropyrazolo[4,3-c]pyridinyl substituted by C₁₋₆alkyl;
R³ is C₁₋₆alkyl;
L is azetidinyl;
or a pharmaceutically acceptable salt thereof.

9. A compound according to claim 8, wherein
R¹ is 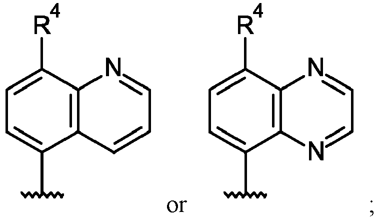 wherein R⁴ is cyano;
R² is 4,5,6,7-tetrahydropyrazolo[3,4-c]pyridinyl substituted by methyl; or 4,5,6,7-tetrahydropyrazolo[4,3-c]pyridinyl substituted by methyl;
R³ is methyl;
L is azetidinyl;
or a pharmaceutically acceptable salt thereof.

10. A compound according to claim 1 or 2, selected from:
5-[(2*R*,6*S*)-2-methyl-6-[[3-(4,5,6,7-tetrahydropyrazolo[4,3-c]pyridin-1-yl)azetidin-1-yl]methyl]morpholin-4-yl]quinoline-8-carbonitrile;
5-[(2*R*,6*S*)-2-methyl-6-[[3-(4,5,6,7-tetrahydropyrazolo[4,3-c]pyridin-2-yl)azetidin-1-yl]methyl]morpholin-4-yl]quinoline-8-carbonitrile;
5-[(2*R*,6*S*)-2-methyl-6-[[3-(4,5,6,7-tetrahydropyrazolo[3,4-c]pyridin-1-yl)azetidin-1-yl]methyl]morpholin-4-yl]quinoline-8-carbonitrile;
5-[(2*R*,6*S*)-2-methyl-6-[[3-(4,5,6,7-tetrahydropyrazolo[3,4-c]pyridin-2-yl)azetidin-1-yl]methyl]morpholin-4-yl]quinoline-8-carbonitrile;
5-[(2*R*,6*S*)-2-methyl-6-[[3-(6-methyl-5,7-dihydro-4*H*-pyrazolo[3,4-c]pyridin-2-yl)azetidin-1-yl]methyl]morpholin-4-yl]quinoline-8-carbonitrile;
5-[(2*S*,6*R*)-2-[[3-(6-ethyl-5,7-dihydro-4*H*-pyrazolo[3,4-c]pyridin-1-yl)azetidin-1-yl]methyl]-6-methyl-morpholin-4-yl]quinoline-8-carbonitrile;
5-[(2*S*,6*R*)-2-[[3-(6-ethyl-5,7-dihydro-4*H*-pyrazolo[3,4-c]pyridin-2-yl)azetidin-1-yl]methyl]-6-methyl-morpholin-4-yl]quinoline-8-carbonitrile;
5-[(2*S*,6*R*)-2-[[3-(6-isopropyl-5,7-dihydro-4*H*-pyrazolo[3,4-c]pyridin-1-yl)azetidin-1-yl]methyl]-6-methyl-morpholin-4-yl]quinoline-8-carbonitrile;
5-[(2*S*,6*R*)-2-[[3-(6-isopropyl-5,7-dihydro-4*H*-pyrazolo[3,4-c]pyridin-2-yl)azetidin-1-yl]methyl]-6-methyl-morpholin-4-yl]quinoline-8-carbonitrile;
5-[(2*S*,6*R*)-2-[[3-(5,6-dihydro-4*H*-pyrrolo[3,4-c]pyrazol-1-yl)azetidin-1-yl]methyl]-6-methyl-morpholin-4-yl]quinoline-8-carbonitrile;
5-[(2*S*,6*R*)-2-[[3-(5,6-dihydro-4*H*-pyrrolo[3,4-c]pyrazol-2-yl)azetidin-1-yl]methyl]-6-methyl-morpholin-4-yl]quinoline-8-carbonitrile;
5-[(2*R*,6*S*)-2-methyl-6-[[3-(3-methyl-4,5,6,7-tetrahydropyrazolo[4,3-c]pyridin-1-yl)azetidin-1-yl]methyl]morpholin-4-yl]quinoline-8-carbonitrile;
5-[(2*R*,6*S*)-2-methyl-6-[[3-(3-methyl-4,5,6,7-tetrahydropyrazolo[4,3-c]pyridin-2-yl)azetidin-1-yl]methyl]morpholin-4-yl]quinoline-8-carbonitrile;
5-[(2*S*,6*R*)-2-[[3-(3,5-dimethyl-6,7-dihydro-4*H*-pyrazolo[4,3-c]pyridin-1-yl)azetidin-1-yl]methyl]-6-methyl-morpholin-4-yl]quinoline-8-carbonitrile;
5-[(2*S*,6*R*)-2-[[3-(3,5-dimethyl-6,7-dihydro-4*H*-pyrazolo[4,3-c]pyridin-2-yl)azetidin-1-yl]methyl]-6-methyl-morpholin-4-yl]quinoline-8-carbonitrile;
5-[(2*R*,6*S*)-2-methyl-6-[[3-[(6*R*)-6-methyl-4,5,6,7-tetrahydropyrazolo[4,3-c]pyridin-1-yl]azetidin-1-yl]methyl]morpholin-4-yl]quinoline-8-carbonitrile;
5-[(2*R*,6*S*)-2-methyl-6-[[3-[(6*R*)-6-methyl-4,5,6,7-tetrahydropyrazolo[4,3-c]pyridin-2-yl]azetidin-1-yl]methyl]morpholin-4-yl]quinoline-8-carbonitrile;
5-[(2*R*,6*S*)-2-methyl-6-[[3-[(6*S*)-6-methyl-4,5,6,7-tetrahydropyrazolo[4,3-c]pyridin-1-yl]azetidin-1-yl]methyl]morpholin-4-yl]quinoline-8-carbonitrile;
5-[(2*R*,6*S*)-2-methyl-6-[[3-[(6*S*)-6-methyl-4,5,6,7-tetrahydropyrazolo[4,3-c]pyridin-2-yl]azetidin-1-yl]methyl]morpholin-4-yl]quinoline-8-carbonitrile;
5-[(2*R*,6*S*)-2-methyl-6-[[3-[(4*R*)-4-methyl-4,5,6,7-tetrahydropyrazolo[4,3-c]pyridin-1-yl]azetidin-1-yl]methyl]morpholin-4-yl]quinoline-8-carbonitrile;
5-[(2*R*,6*S*)-2-methyl-6-[[3-[(4*R*)-4-methyl-4,5,6,7-tetrahydropyrazolo[4,3-c]pyridin-2-yl]azetidin-1-yl]methyl]morpholin-4-yl]quinoline-8-carbonitrile;
5-[(2*R*,6*S*)-2-methyl-6-[[3-[(4*S*)-4-methyl-4,5,6,7-tetrahydropyrazolo[4,3-c]pyridin-1-yl]azetidin-1-yl]methyl]morpholin-4-yl]quinoline-8-carbonitrile;
5-[(2*R*,6*S*)-2-methyl-6-[[3-[(4*S*)-4-methyl-4,5,6,7-tetrahydropyrazolo[4,3-c]pyridin-2-yl]azetidin-1-yl]methyl]morpholin-4-yl]quinoline-8-carbonitrile;
5-[(2*S*,6*R*)-2-[[3-(7,7-dimethyl-5,6-dihydro-4*H*-pyrazolo[4,3-c]pyridin-2-yl)azetidin-1-yl]methyl]-6-methyl-morpholin-4-yl]quinoline-8-carbonitrile;
5-[(2*R*,6*S*)-2-methyl-6-[[3-[(7*R*)-7-methyl-4,5,6,7-tetrahydropyrazolo[4,3-c]pyridin-2-yl]azetidin-1-yl]methyl]morpholin-4-yl]quinoline-8-carbonitrile;
5-[(2*R*,6*S*)-2-methyl-6-[[3-[(7*S*)-7-methyl-4,5,6,7-tetrahydropyrazolo[4,3-c]pyridin-2-yl]azetidin-1-yl]methyl]morpholin-4-yl]quinoline-8-carbonitrile;
5-[(2*R*,6*S*)-2-methyl-6-[[3-[(5*S*)-5-methyl-4,5,6,7-tetrahydropyrazolo[3,4-c]pyridin-1-yl]azetidin-1-yl]methyl]morpholin-4-yl]quinoline-8-carbonitrile;
5-[(2*R*,6*S*)-2-methyl-6-[[3-[(5*R*)-5-methyl-4,5,6,7-tetrahydropyrazolo[3,4-c]pyridin-1-yl]azetidin-1-yl]methyl]morpholin-4-yl]quinoline-8-carbonitrile;
5-[(2*R*,6*S*)-2-methyl-6-[[3-[(5*S*)-5-methyl-4,5,6,7-tetrahydropyrazolo[3,4-c]pyridin-2-yl]azetidin-1-yl]methyl]morpholin-4-yl]quinoline-8-carbonitrile;
5-[(2*R*,6*S*)-2-methyl-6-[[3-[(5*R*)-5-methyl-4,5,6,7-tetrahydropyrazolo[3,4-c]pyridin-2-yl]azetidin-1-yl]methyl]morpholin-4-yl]quinoline-8-carbonitrile;
5-[(2*R*,6*S*)-2-methyl-6-[[3-[(7*R*)-7-methyl-4,5,6,7-tetrahydropyrazolo[3,4-c]pyridin-1-yl]azetidin-1-yl]methyl]morpholin-4-yl]quinoline-8-carbonitrile;
5-[(2*R*,6*S*)-2-methyl-6-[[3-[(7*S*)-7-methyl-4,5,6,7-tetrahydropyrazolo[3,4-c]pyridin-1-yl]azetidin-1-yl]methyl]morpholin-4-yl]quinoline-8-carbonitrile;
5-[(2*R*,6*S*)-2-methyl-6-[[3-[(7*R*)-7-methyl-4,5,6,7-tetrahydropyrazolo[3,4-c]pyridin-2-yl]azetidin-1-yl]methyl]morpholin-4-yl]quinoline-8-carbonitrile;
5-[(2*R*,6*S*)-2-methyl-6-[[3-[(7*S*)-7-methyl-4,5,6,7-tetrahydropyrazolo[3,4-c]pyridin-2-yl]azetidin-1-yl]methyl]morpholin-4-yl]quinoline-8-carbonitrile;
5-[(2*R*,6*S*)-2-methyl-6-[[4-(4,5,6,7-tetrahydropyrazolo[4,3-c]pyridin-1-yl)-1-piperidyl]methyl]morpholin-4-yl]quinoline-8-carbonitrile;
5-[(2*R*,6*S*)-2-methyl-6-[[4-(4,5,6,7-tetrahydropyrazolo[4,3-c]pyridin-2-yl)-1-piperidyl]methyl]morpholin-4-yl]quinoline-8-carbonitrile;
5-[(2*R*,6*S*)-2-methyl-6-[[3-[3-(trifluoromethyl)-4,5,6,7-tetrahydropyrazolo[3,4-c]pyridin-1-yl]azetidin-1-yl]methyl]morpholin-4-yl]quinoline-8-carbonitrile;
5-[(2*R*,6*S*)-2-methyl-6-[[3-[3-(trifluoromethyl)-4,5,6,7-tetrahydropyrazolo[3,4-c]pyridin-2-yl]azetidin-1-yl]methyl]morpholin-4-yl]quinoline-8-carbonitrile;
5-[(2*R*,6*S*)-2-methyl-6-[[3-(3-methyl-4,5,6,7-tetrahydropyrazolo[3,4-c]pyridin-1-yl)azetidin-1-yl]methyl]morpholin-4-yl]quinoline-8-carbonitrile; and
5-[(2*R*,6*S*)-2-methyl-6-[[3-(3-methyl-4,5,6,7-tetrahydropyrazolo[3,4-c]pyridin-2-yl)azetidin-1-yl]methyl]morpholin-4-yl]quinoline-8-carbonitrile;
or a pharmaceutically acceptable salt thereof.

11. A process for the preparation of a compound according to any one of claims 1 to 10 comprising the following step:
a) the coupling of compound of formula (V), 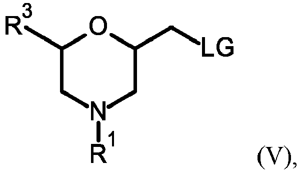 with compound of formula (IX), 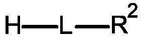 (IX), in the presence of a base;
wherein the base in step a) is K₂CO₃, DIPEA or Cs₂CO_{3;} R¹, R², R³ and L are defined as in any one of claims 1 to 9.

12. A compound or pharmaceutically acceptable salt, enantiomer or diastereomer according to any one of claims 1 to 10 for use as therapeutically active substance.

13. A pharmaceutical composition comprising a compound in accordance with any one of claims 1 to 10 and a therapeutically inert carrier.

14. A compound or pharmaceutically acceptable salt, enantiomer or diastereomer according to any one of claims 1 to 10 for use in a method of treatment or prophylaxis of systemic lupus erythematosus or lupus nephritis.

## Patentansprüche

1. Verbindung der Formel (I) 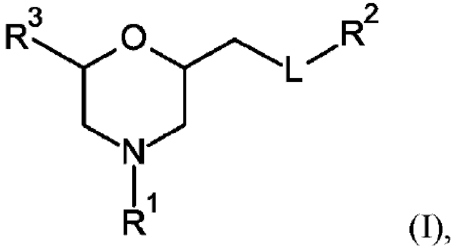 wobei
R1 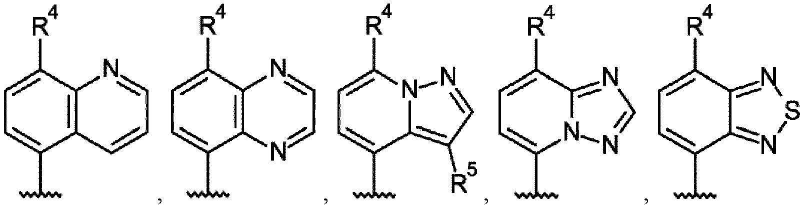 oder 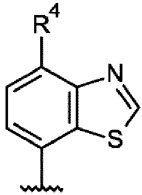 ist; wobei R⁴ C₁₋₆-Alkyl, Halogen oder Cyano ist; R⁵ Halogen ist;
R² 4,5,6,7-Tetrahydropyrazolo[3,4-c]pyridinyl, das unsubstituiert oder mit C₁₋₆-Alkyl oder Halogen-C₁₋₆-alkyl substituiert ist;
4,5,6,7-Tetrahydropyrazolo[4,3-c]pyridinyl, das unsubstituiert oder ein- oder zweimal mit C₁₋₆-Alkyl substituiert ist; oder
5,6-Dihydro-4H-pyrrolo[3,4-c]pyrazolyl ist;
R³ C₁₋₆-Alkyl ist;
L Azetidinyl oder Piperidinyl ist;
oder ein pharmazeutisch unbedenkliches Salz davon.

2. Verbindung nach Anspruch 1, wobei die Verbindung die Formel (Ia) aufweist 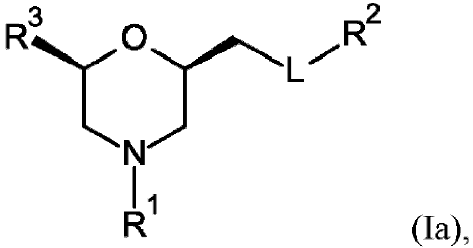 wobei
R1 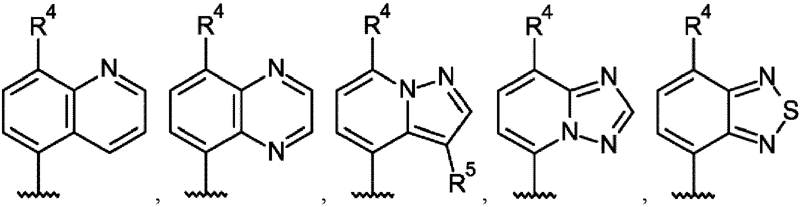 oder 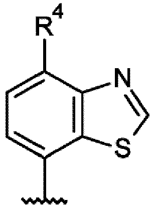 ist; wobei R⁴ C₁₋₆-Alkyl, Halogen oder Cyano ist; R⁵ Halogen ist;
R² 4,5,6,7-Tetrahydropyrazolo[3,4-c]pyridinyl, das unsubstituiert oder mit C₁₋₆-Alkyl oder Halogen-C₁₋₆-alkyl substituiert ist;
4,5,6,7-Tetrahydropyrazolo[4,3-c]pyridinyl, das unsubstituiert oder ein- oder zweimal mit C₁₋₆-Alkyl substituiert ist; oder
5,6-Dihydro-4H-pyrrolo[3,4-c]pyrazolyl ist;
R³ C₁₋₆-Alkyl ist;
L Azetidinyl oder Piperidinyl ist;
oder ein pharmazeutisch unbedenkliches Salz davon.

3. Verbindung nach Anspruch 1 oder 2 oder ein pharmazeutisch unbedenkliches Salz davon, wobei
R1 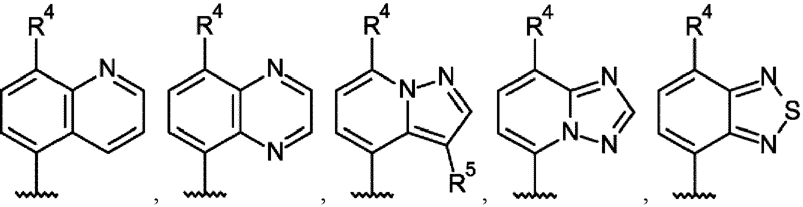 oder 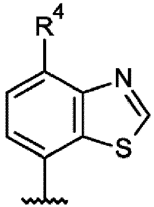 ist; wobei R⁴ Methyl, Chlor oder Cyano ist; R⁵ Fluor ist;
R² 4,5,6,7-Tetrahydropyrazolo[3,4-c]pyridinyl, das unsubstituiert oder mit Methyl, Ethyl, Isopropyl oder Trifluormethyl substituiert ist;
4,5,6,7-Tetrahydropyrazolo[4,3-c]pyridinyl, das unsubstituiert oder ein- oder zweimal mit Methyl substituiert ist; oder
5,6-Dihydro-4H-pyrrolo[3,4-c]pyrazolyl ist;
R³ Methyl ist;
L Azetidinyl oder Piperidinyl ist;
oder ein pharmazeutisch unbedenkliches Salz davon.

4. Verbindung nach Anspruch 1 oder 2 oder ein pharmazeutisch unbedenkliches Salz davon, wobei
R1 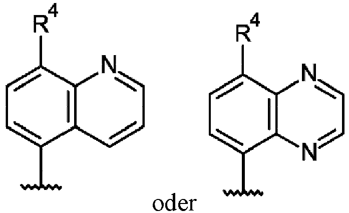 ist; wobei R⁴ Cyano ist.

5. Verbindung nach Anspruch 4 oder ein pharmazeutisch unbedenkliches Salz davon, wobei L Azetidinyl ist.

6. Verbindung nach Anspruch 5 oder ein pharmazeutisch unbedenkliches Salz davon, wobei R² mit C₁₋₆-Alkyl substituiertes 4,5,6,7-Tetrahydropyrazolo[3,4-c]pyridinyl oder mit C₁₋₆-Alkyl substituiertes 4,5,6,7-Tetrahydropyrazolo[4,3-c]pyridinyl ist.

7. Verbindung nach Anspruch 6 oder ein pharmazeutisch unbedenkliches Salz davon, wobei R² mit Methyl substituiertes 4,5,6,7-Tetrahydropyrazolo[3,4-c]pyridinyl oder mit Methyl substituiertes 4,5,6,7-Tetrahydropyrazolo[4,3-c]pyridinyl ist.

8. Verbindung nach Anspruch 1 oder 2, wobei
R1 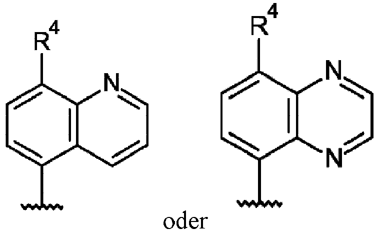 ist; wobei R⁴ Cyano ist;
R² mit C₁₋₆-Alkyl substituiertes 4,5,6,7-Tetrahydropyrazolo[3,4-c]pyridinyl oder mit C₁₋₆-Alkyl substituiertes 4,5,6,7-Tetrahydropyrazolo[4,3-c]pyridinyl ist;
R³ C₁₋₆-Alkyl ist;
L Azetidinyl ist;
oder ein pharmazeutisch unbedenkliches Salz davon.

9. Verbindung nach Anspruch 8, wobei
R1 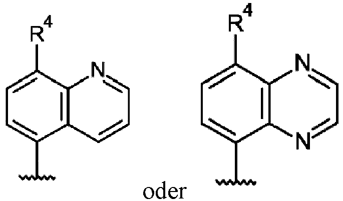 ist; wobei R⁴ Cyano ist;
R² mit Methyl substituiertes 4,5,6,7-Tetrahydropyrazolo[3,4-c]pyridinyl oder mit Methyl substituiertes 4,5,6,7-Tetrahydropyrazolo[4,3-c]pyridinyl ist;
R³ Methyl ist;
L Azetidinyl ist;
oder ein pharmazeutisch unbedenkliches Salz davon.

10. Verbindung nach Anspruch 1 oder 2, ausgewählt aus:
5-[(2*R*,6*S*)-2-Methyl-6-[[3-(4,5,6,7-tetrahydropyrazolo[4,3-c]pyridin-1-yl)azetidin-1-yl]methyl]morpholin-4-yl]chinolin-8-carbonitril;
5-[(2*R*,6*S*)-2-Methyl-6-[[3-(4,5,6,7-tetrahydropyrazolo[4,3-c]pyridin-2-yl)azetidin-1-yl]methyl]morpholin-4-yl]chinolin-8-carbonitril;
5-[(2*R*,6*S*)-2-Methyl-6-[[3-(4,5,6,7-tetrahydropyrazolo[3,4-c]pyridin-1-yl)azetidin-1-yl]methyl]morpholin-4-yl]chinolin-8-carbonitril;
5-[(2*R*,6*S*)-2-Methyl-6-[[3-(4,5,6,7-tetrahydropyrazolo[3,4-c]pyridin-2-yl)azetidin-1-yl]methyl]morpholin-4-yl]chinolin-8-carbonitril;
5-[(2*R*,6*S*)-2-Methyl-6-[[3-(6-methyl-5,7-dihydro-4*H*-pyrazolo[3,4-c]pyridin-2-yl)azetidin-1-yl]methyl]morpholin-4-yl]chinolin-8-carbonitril;
5-[(2*S*,6*R*)-2-[[3-(6-Ethyl-5,7-dihydro-4*H*-pyrazolo[3,4-c]pyridin-1-yl)azetidin-1-yl]methyl]-6-methylmorpholin-4-yl]chinolin-8-carbonitril;
5-[(2*S*,6*R*)-2-[[3-(6-Ethyl-5,7-dihydro-4*H*-pyrazolo[3,4-c]pyridin-2-yl)azetidin-1-yl]methyl]-6-methylmorpholin-4-yl]chinolin-8-carbonitril;
5-[(2*S*,6*R*)-2-[[3-(6-Isopropyl-5,7-dihydro-4*H*-pyrazolo[3,4-c]pyridin-1-yl)azetidin-1-yl]methyl]-6-methylmorpholin-4-yl]chinolin-8-carbonitril;
5-[(2*S*,6*R*)-2-[[3-(6-Isopropyl-5,7-dihydro-4*H*-pyrazolo[3,4-c]pyridin-2-yl)azetidin-1-yl]methyl]-6-methylmorpholin-4-yl]chinolin-8-carbonitril;
5-[(2*S*,6*R*)-2-[[3-(5,6-Dihydro-4*H*-pyrrolo[3,4-c]pyrazol-1-yl)azetidin-1-yl]methyl]-6-methylmorpholin-4-yl] chinolin-8-carbonitril;
5-[(2*S*,6*R*)-2-[[3-(5,6-Dihydro-4*H*-pyrrolo[3,4-c]pyrazol-2-yl)azetidin-1-yl]methyl]-6-methylmorpholin-4-yl] chinolin-8-carbonitril;
5-[(2*R*,6*S*)-2-Methyl-6-[[3-(3-methyl-4,5,6,7-tetrahydropyrazolo[4,3-c]pyridin-1-yl)azetidin-1-yl]methyl]morpholin-4-yl]chinolin-8-carbonitril;
5-[(2*R*,6*S*)-2-Methyl-6-[[3-(3-methyl-4,5,6,7-tetrahydropyrazolo[4,3-c]pyridin-2-yl)azetidin-1-yl]methyl]morpholin-4-yl]chinolin-8-carbonitril;
5-[(2*S*,6*R*)-2-[[3-(3,5-Dimethyl-6,7-dihydro-4*H*-pyrazolo[4,3-c]pyridin-1-yl)azetidin-1-yl]methyl]-6-methylmorpholin-4-yl]chinolin-8-carbonitril;
5-[(2*S*,6*R*)-2-[[3-(3,5-Dimethyl-6,7-dihydro-4*H*-pyrazolo[4,3-c]pyridin-2-yl)azetidin-1-yl]methyl]-6-methylmorpholin-4-yl]chinolin-8-carbonitril;
5-[(2*R*,6*S*)-2-Methyl-6-[[3-[(6*R*)-6-methyl-4,5,6,7-tetrahydropyrazolo[4,3-c]pyridin-1-yl]azetidin-1-yl]methyl]morpholin-4-yl]chinolin-8-carbonitril;
5-[(2*R*,6*S*)-2-Methyl-6-[[3-[(6*R*)-6-methyl-4,5,6,7-tetrahydropyrazolo[4,3-c]pyridin-2-yl]azetidin-1-yl]methyl]morpholin-4-yl]chinolin-8-carbonitril;
5-[(2*R*,6*S*)-2-Methyl-6-[[3-[(6*S*)-6-methyl-4,5,6,7-tetrahydropyrazolo[4,3-c]pyridin-1-yl]azetidin-1-yl]methyl]morpholin-4-yl]chinolin-8-carbonitril;
5-[(2*R*,6*S*)-2-Methyl-6-[[3-[(6*S*)-6-methyl-4,5,6,7-tetrahydropyrazolo[4,3-c]pyridin-2-yl]azetidin-1-yl]methyl]morpholin-4-yl]chinolin-8-carbonitril;
5-[(2*R*,6*S*)-2-Methyl-6-[[3-[(4*R*)-4-methyl-4,5,6,7-tetrahydropyrazolo[4,3-c]pyridin-1-yl]azetidin-1-yl]methyl]morpholin-4-yl]chinolin-8-carbonitril;
5-[(2*R*,6*S*)-2-Methyl-6-[[3-[(4*R*)-4-methyl-4,5,6,7-tetrahydropyrazolo[4,3-c]pyridin-2-yl]azetidin-1-yl]methyl]morpholin-4-yl]chinolin-8-carbonitril;
5-[(2*R*,6*S*)-2-Methyl-6-[[3-[(4*S*)-4-methyl-4,5,6,7-tetrahydropyrazolo[4,3-c]pyridin-1-yl]azetidin-1-yl]methyl]morpholin-4-yl]chinolin-8-carbonitril;
5-[(2*R*,6*S*)-2-Methyl-6-[[3-[(4*S*)-4-methyl-4,5,6,7-tetrahydropyrazolo[4,3-c]pyridin-2-yl]azetidin-1-yl]methyl]morpholin-4-yl]chinolin-8-carbonitril;
5-[(2*S*,6*R*)-2-[[3-(7,7-Dimethyl-5,6-dihydro-4*H*-pyrazolo[4,3-c]pyridin-2-yl)azetidin-1-yl]methyl]-6-methylmorpholin-4-yl]chinolin-8-carbonitril;
5-[(2*R*,6*S*)-2-Methyl-6-[[3-[(7*R*)-7-methyl-4,5,6,7-tetrahydropyrazolo[4,3-c]pyridin-2-yl]azetidin-1-yl]methyl]morpholin-4-yl]chinolin-8-carbonitril;
5-[(2*R*,6*S*)-2-Methyl-6-[[3-[(7*S*)-7-methyl-4,5,6,7-tetrahydropyrazolo[4,3-c]pyridin-2-yl]azetidin-1-yl]methyl]morpholin-4-yl]chinolin-8-carbonitril;
5-[(2*R*,6*S*)-2-Methyl-6-[[3-[(5*S*)-5-methyl-4,5,6,7-tetrahydropyrazolo[3,4-c]pyridin-1-yl]azetidin-1-yl]methyl]morpholin-4-yl]chinolin-8-carbonitril;
5-[(2*R*,6*S*)-2-Methyl-6-[[3-[(5*R*)-5-methyl-4,5,6,7-tetrahydropyrazolo[3,4-c]pyridin-1-yl]azetidin-1-yl]methyl]morpholin-4-yl]chinolin-8-carbonitril;
5-[(2*R*,6*S*)-2-Methyl-6-[[3-[(5*S*)-5-methyl-4,5,6,7-tetrahydropyrazolo[3,4-c]pyridin-2-yl]azetidin-1-yl]methyl]morpholin-4-yl]chinolin-8-carbonitril;
5-[(2*R*,6*S*)-2-Methyl-6-[[3-[(5*R*)-5-methyl-4,5,6,7-tetrahydropyrazolo[3,4-c]pyridin-2-yl]azetidin-1-yl]methyl]morpholin-4-yl]chinolin-8-carbonitril;
5-[(2*R*,6*S*)-2-Methyl-6-[[3-[(7*R*)-7-methyl-4,5,6,7-tetrahydropyrazolo[3,4-c]pyridin-1-yl]azetidin-1-yl]methyl]morpholin-4-yl]chinolin-8-carbonitril;
5-[(2*R*,6*S*)-2-Methyl-6-[[3-[(7*S*)-7-methyl-4,5,6,7-tetrahydropyrazolo[3,4-c]pyridin-1-yl]azetidin-1-yl]methyl]morpholin-4-yl]chinolin-8-carbonitril;
5-[(2*R*,6*S*)-2-Methyl-6-[[3-[(7*R*)-7-methyl-4,5,6,7-tetrahydropyrazolo[3,4-c]pyridin-2-yl]azetidin-1-yl]methyl]morpholin-4-yl]chinolin-8-carbonitril;
5-[(2*R*,6*S*)-2-Methyl-6-[[3-[(7*S*)-7-methyl-4,5,6,7-tetrahydropyrazolo[3,4-c]pyridin-2-yl]azetidin-1-yl]methyl]morpholin-4-yl]chinolin-8-carbonitril;
5-[(2*R*,6*S*)-2-Methyl-6-[[4-(4,5,6,7-tetrahydropyrazolo[4,3-c]pyridin-1-yl)-1-piperidyl]methyl]morpholin-4-yl]chinolin-8-carbonitril;
5-[(2*R*,6*S*)-2-Methyl-6-[[4-(4,5,6,7-tetrahydropyrazolo[4,3-c]pyridin-2-yl)-1-piperidyl]methyl]morpholin-4-yl]chinolin-8-carbonitril;
5-[(2*R*,6*S*)-2-Methyl-6-[[3-[3-(trifluormethyl)-4,5,6,7-tetrahydropyrazolo[3,4-c]pyridin-1-yl]azetidin-1-yl]methyl]morpholin-4-yl]chinolin-8-carbonitril;
5-[(2*R*,6*S*)-2-Methyl-6-[[3-[3-(trifluormethyl)-4,5,6,7-tetrahydropyrazolo[3,4-c]pyridin-2-yl] azetidin-1-yl] methyl] morpholin-4-yl] chinolin-8-carb onitril;
5-[(2*R*,6*S*)-2-Methyl-6-[[3-(3-methyl-4,5,6,7-tetrahydropyrazolo[3,4-c]pyridin-1-yl)azetidin-1-yl]methyl]morpholin-4-yl]chinolin-8-carbonitril; und
5-[(2*R*,6*S*)-2-Methyl-6-[[3-(3-methyl-4,5,6,7-tetrahydropyrazolo[3,4-c]pyridin-2-yl)azetidin-1-yl]methyl]morpholin-4-yl]chinolin-8-carbonitril;
oder ein pharmazeutisch unbedenkliches Salz davon.

11. Verfahren zur Herstellung einer Verbindung nach einem der Ansprüche 1 bis 10, umfassend den folgenden Schritt:
a) das Koppeln einer Verbindung der Formel (V) 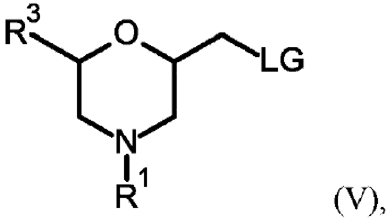 mit einer Verbindung der Formel (IX), **H-L-R²** (IX), in der Gegenwart einer Base;
wobei die Base in Schritt a) K₂CO₃, DIPEA oder Cs₂CO₃ ist; R¹, R², R³ und L wie in einem der Ansprüche 1 bis 9 definiert sind.

12. Verbindung oder pharmazeutisch unbedenkliches Salz, Enantiomer oder Diastereomer nach einem der Ansprüche 1 bis 10 zur Verwendung als therapeutisch wirksame Substanz.

13. Pharmazeutische Zusammensetzung, umfassend eine Verbindung nach einem der Ansprüche 1 bis 10 und einen therapeutisch inerten Träger.

14. Verbindung oder pharmazeutisch unbedenkliches Salz, Enantiomer oder Diastereomer nach einem der Ansprüche 1 bis 10 zur Verwendung in einem Verfahren zur Behandlung oder Prophylaxe von systemischem Lupus erythematodes oder Lupusnephritis.

## Revendications

1. Composé de formule (I), 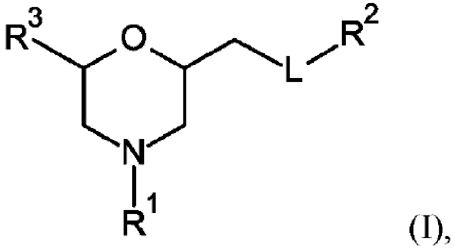 dans lequel
R¹ représente 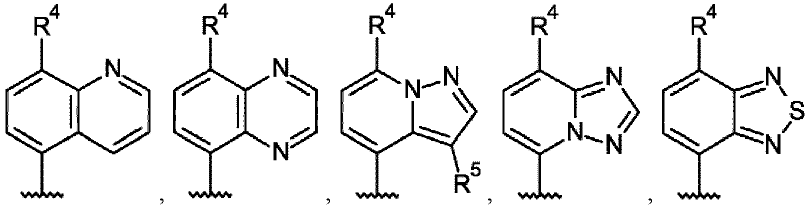 ou 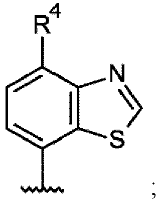 dans lequel R⁴ représente un alkyle en C₁₋₆, un halogène ou un cyano ; R⁵ représente un halogène ;
R² représente un 4,5,6,7-tétrahydropyrazolo[3,4-c]pyridinyle qui est non substitué ou substitué par un alkyle en C₁₋₆ ou un halogénoalkyle en C₁₋₆ ;
un 4,5,6,7-tétrahydropyrazolo[4,3-c]pyridinyle qui est non substitué ou substitué une ou deux fois par un alkyle en C₁₋₆ ; ou
un 5,6-dihydro-4H-pyrrolo[3,4-c]pyrazolyle ;
R³ représente un alkyle en C₁₋₆ ;
L représente un azétidinyle ou un pipéridinyle ;
ou un sel pharmaceutiquement acceptable de celui-ci.

2. Composé selon la revendication 1, dans lequel le composé est de formule (Ia), 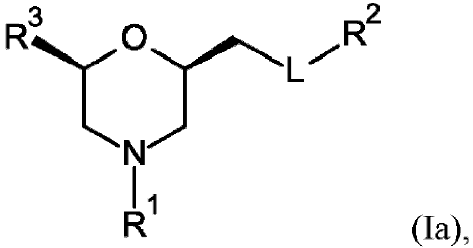 dans lequel
R¹ représente 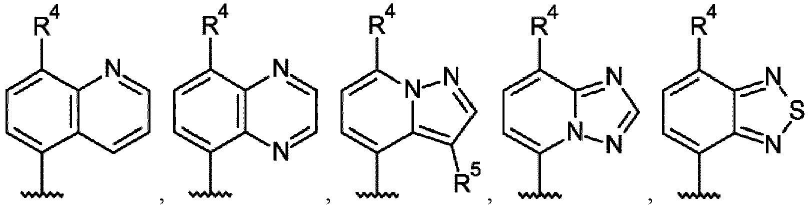 ou 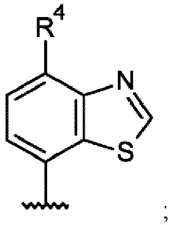 dans lequel R⁴ représente un alkyle en C₁₋₆, un halogène ou un cyano ; R⁵ représente un halogène ;
R² représente un 4,5,6,7-tétrahydropyrazolo[3,4-c]pyridinyle qui est non substitué ou substitué par un alkyle en C₁₋₆ ou un halogénoalkyle en C₁₋₆ ;
un 4,5,6,7-tétrahydropyrazolo[4,3-c]pyridinyle qui est non substitué ou substitué une ou deux fois par un alkyle en C₁₋₆ ; ou
un 5,6-dihydro-4H-pyrrolo[3,4-c]pyrazolyle ;
R³ représente un alkyle en C₁₋₆ ;
L représente un azétidinyle ou un pipéridinyle ;
ou un sel pharmaceutiquement acceptable de celui-ci.

3. Composé selon la revendication 1 ou 2, ou un sel pharmaceutiquement acceptable de celui-ci, dans lequel
R¹ représente 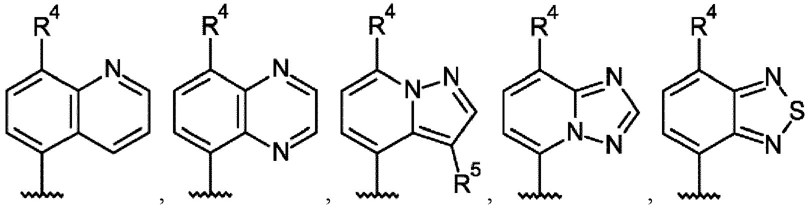 ou 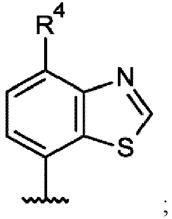 dans lequel R⁴ représente un méthyle, un chlore ou un cyano ; R⁵ représente un fluor ;
R² représente un 4,5,6,7-tétrahydropyrazolo[3,4-c]pyridinyle qui est non substitué ou substitué par un méthyle, un éthyle, un isopropyle ou un trifluorométhyle ;
un 4,5,6,7-tétrahydropyrazolo[4,3-c]pyridinyle qui est non substitué ou substitué une ou deux fois par un méthyle ; ou
un 5,6-dihydro-4H-pyrrolo[3,4-c]pyrazolyle ;
R³ représente un méthyle ;
L représente un azétidinyle ou un pipéridinyle ;
ou un sel pharmaceutiquement acceptable de celui-ci.

4. Composé selon la revendication 1 ou 2, ou un sel pharmaceutiquement acceptable de celui-ci, dans lequel
R¹ représente 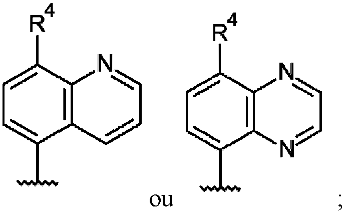 dans lequel R⁴ représente un cyano.

5. Composé selon la revendication 4, ou un sel pharmaceutiquement acceptable de celui-ci, dans lequel L représente un azétidinyle.

6. Composé selon la revendication 5, ou un sel pharmaceutiquement acceptable de celui-ci, dans lequel R² représente un 4,5,6,7-tétrahydropyrazolo[3,4-c]pyridinyle substitué par un alkyle en C₁₋₆ ; ou un 4,5,6,7-tétrahydropyrazolo[4,3-c]pyridinyle substitué par un alkyle en C₁₋₆.

7. Composé selon la revendication 6, ou un sel pharmaceutiquement acceptable de celui-ci, dans lequel R² représente un 4,5,6,7-tétrahydropyrazolo[3,4-c]pyridinyle substitué par un méthyle ; ou un 4,5,6,7-tétrahydropyrazolo[4,3-c]pyridinyle substitué par un méthyle.

8. Composé selon la revendication 1 ou 2, dans lequel
R¹ représente 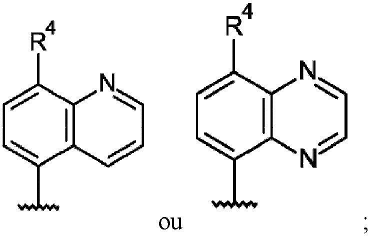 dans lequel R⁴ représente un cyano ;
R² représente un 4,5,6,7-tétrahydropyrazolo[3,4-c]pyridinyle substitué par un alkyle en C₁₋₆ ; ou un 4,5,6,7-tétrahydropyrazolo[4,3-c]pyridinyle substitué par un alkyle en C₁₋₆ ;
R³ représente un alkyle en C₁₋₆ ;
L représente un azétidinyle ;
ou un sel pharmaceutiquement acceptable de celui-ci.

9. Composé selon la revendication 8, dans lequel
R¹ représente 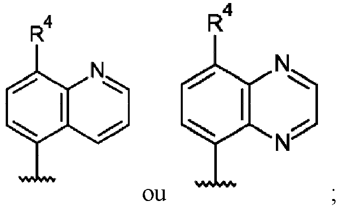 dans lequel R⁴ représente un cyano ;
R² représente un 4,5,6,7-tétrahydropyrazolo[3,4-c]pyridinyle substitué par un méthyle ; ou un 4,5,6,7-tétrahydropyrazolo[4,3-c]pyridinyle substitué par un méthyle ;
R³ représente un méthyle ;
L représente un azétidinyle ;
ou un sel pharmaceutiquement acceptable de celui-ci.

10. Composé selon la revendication 1 ou 2, choisi parmi :
le 5-[(2*R*,6*S*)-2-méthyl-6-[[3-(4,5,6,7-tétrahydropyrazolo[4,3-c]pyridin-1-yl)azétidin-1-yl]méthyl]morpholin-4-yl]quinoléine-8-carbonitrile ;
le 5-[(2*R*,6*S*)-2-méthyl-6-[[3-(4,5,6,7-tétrahydropyrazolo[4,3-c]pyridin-2-yl)azétidin-1-yl]méthyl]morpholin-4-yl]quinoléine-8-carbonitrile ;
le 5-[(2*R*,6*S*)-2-méthyl-6-[[3-(4,5,6,7-tétrahydropyrazolo[3,4-c]pyridin-1-yl)azétidin-1-yl]méthyl]morpholin-4-yl]quinoléine-8-carbonitrile ;
le 5-[(2*R*,6*S*)-2-méthyl-6-[[3-(4,5,6,7-tétrahydropyrazolo[3,4-c]pyridin-2-yl)azétidin-1-yl]méthyl]morpholin-4-yl]quinoléine-8-carbonitrile ;
le 5-[(2*R*,6*S*)-2-méthyl-6-[[3-(6-méthyl-5,7-dihydro-4*H*-pyrazolo[3,4-c]pyridin-2-yl)azétidin-1-yl]méthyl]morpholin-4-yl]quinoléine-8-carbonitrile ;
le 5-[(2*S*,6*R*)-2-[[3-(6-éthyl-5,7-dihydro-4*H*-pyrazolo[3,4-c]pyridin-1-yl)azétidin-1-yl]méthyl]-6-méthyl-morpholin-4-yl]quinoléine-8-carbonitrile ;
le 5-[(2*S*,6*R*)-2-[[3-(6-éthyl-5,7-dihydro-4*H*-pyrazolo[3,4-c]pyridin-2-yl)azétidin-1-yl]méthyl]-6-méthyl-morpholin-4-yl]quinoléine-8-carbonitrile ;
le 5-[(2*S*,6*R*)-2-[[3-(6-isopropyl-5,7-dihydro-4*H*-pyrazolo[3,4-c]pyridin-1-yl)azétidin-1-yl]méthyl]-6-méthyl-morpholin-4-yl]quinoléine-8-carbonitrile ;
le 5-[(2*S*,6*R*)-2-[[3-(6-isopropyl-5,7-dihydro-4*H*-pyrazolo[3,4-c]pyridin-2-yl)azétidin-1-yl]méthyl]-6-méthyl-morpholin-4-yl]quinoléine-8-carbonitrile ;
le 5-[(2*S*,6*R*)-2-[[3-(5,6-dihydro-4*H*-pyrrolo[3,4-c]pyrazol-1-yl)azétidin-1-yl]méthyl]-6-méthyl-morpholin-4-yl]quinoléine-8-carbonitrile ;
le 5-[(2*S*,6*R*)-2-[[3-(5,6-dihydro-4*H*-pyrrolo[3,4-c]pyrazol-2-yl)azétidin-1-yl]méthyl]-6-méthyl-morpholin-4-yl]quinoléine-8-carbonitrile ;
le 5-[(2*R*,6*S*)-2-méthyl-6-[[3-(3-méthyl-4,5,6,7-tétrahydropyrazolo[4,3-c]pyridin-1-yl)azétidin-1-yl]méthyl]morpholin-4-yl]quinoléine-8-carbonitrile ;
le 5-[(2*R*,6*S*)-2-méthyl-6-[[3-(3-méthyl-4,5,6,7-tétrahydropyrazolo[4,3-c]pyridin-2-yl)azétidin-1-yl]méthyl]morpholin-4-yl]quinoléine-8-carbonitrile ;
le 5-[(2*S*,6*R*)-2-[[3-(3,5-diméthyl-6,7-dihydro-4*H*-pyrazolo[4,3-c]pyridin-1-yl)azétidin-1-yl]méthyl]-6-méthyl-morpholin-4-yl]quinoléine-8-carbonitrile ;
le 5-[(2*S*,6*R*)-2-[[3-(3,5-diméthyl-6,7-dihydro-4*H*-pyrazolo[4,3-c]pyridin-2-yl)azétidin-1-yl]méthyl]-6-méthyl-morpholin-4-yl]quinoléine-8-carbonitrile ;
le 5-[(2*R*,6*S*)-2-méthyl-6-[[3-[(6*R*)-6-méthyl-4,5,6,7-tétrahydropyrazolo[4,3-c]pyridin-1-yl]azétidin-1-yl]méthyl]morpholin-4-yl]quinoléine-8-carbonitrile ;
le 5-[(2*R*,6*S*)-2-méthyl-6-[[3-[(6*R*)-6-méthyl-4,5,6,7-tétrahydropyrazolo[4,3-c]pyridin-2-yl]azétidin-1-yl]méthyl]morpholin-4-yl]quinoléine-8-carbonitrile ;
le 5-[(2*R*,6*S*)-2-méthyl-6-[[3-[(6*S*)-6-méthyl-4,5,6,7-tétrahydropyrazolo[4,3-c]pyridin-1-yl]azétidin-1-yl]méthyl]morpholin-4-yl]quinoléine-8-carbonitrile ;
le 5-[(2*R*,6*S*)-2-méthyl-6-[[3-[(6*S*)-6-méthyl-4,5,6,7-tétrahydropyrazolo[4,3-c]pyridin-2-yl]azétidin-1-yl]méthyl]morpholin-4-yl]quinoléine-8-carbonitrile ;
le 5-[(2*R*,6*S*)-2-méthyl-6-[[3-[(4*R*)-4-méthyl-4,5,6,7-tétrahydropyrazolo[4,3-c]pyridin-1-yl]azétidin-1-yl]méthyl]morpholin-4-yl]quinoléine-8-carbonitrile ;
le 5-[(2*R*,6*S*)-2-méthyl-6-[[3-[(4*R*)-4-méthyl-4,5,6,7-tétrahydropyrazolo[4,3-c]pyridin-2-yl]azétidin-1-yl]méthyl]morpholin-4-yl]quinoléine-8-carbonitrile ;
le 5-[(2*R*,6*S*)-2-méthyl-6-[[3-[(4*S*)-4-méthyl-4,5,6,7-tétrahydropyrazolo[4,3-c]pyridin-1-yl]azétidin-1-yl]méthyl]morpholin-4-yl]quinoléine-8-carbonitrile ;
le 5-[(2*R*,6*S*)-2-méthyl-6-[[3-[(4*S*)-4-méthyl-4,5,6,7-tétrahydropyrazolo[4,3-c]pyridin-2-yl]azétidin-1-yl]méthyl]morpholin-4-yl]quinoléine-8-carbonitrile ;
le 5-[(2*S*,6*R*)-2-[[3-(7,7-diméthyl-5,6-dihydro-4*H*-pyrazolo[4,3-c]pyridin-2-yl)azétidin-1-yl]méthyl]-6-méthyl-morpholin-4-yl]quinoléine-8-carbonitrile ;
le 5-[(2*R*,6*S*)-2-méthyl-6-[[3-[(7*R*)-7-méthyl-4,5,6,7-tétrahydropyrazolo[4,3-c]pyridin-2-yl]azétidin-1-yl]méthyl]morpholin-4-yl]quinoléine-8-carbonitrile ;
le 5-[(2*R*,6*S*)-2-méthyl-6-[[3-[(7*S*)-7-méthyl-4,5,6,7-tétrahydropyrazolo[4,3-c]pyridin-2-yl]azétidin-1-yl]méthyl]morpholin-4-yl]quinoléine-8-carbonitrile ;
le 5-[(2*R*,6*S*)-2-méthyl-6-[[3-[(5*S*)-5-méthyl-4,5,6,7-tétrahydropyrazolo[3,4-c]pyridin-1-yl]azétidin-1-yl]méthyl]morpholin-4-yl]quinoléine-8-carbonitrile ;
le 5-[(2*R*,6*S*)-2-méthyl-6-[[3-[(5*R*)-5-méthyl-4,5,6,7-tétrahydropyrazolo[3,4-c]pyridin-1-yl]azétidin-1-yl]méthyl]morpholin-4-yl]quinoléine-8-carbonitrile ;
le 5-[(2*R*,6*S*)-2-méthyl-6-[[3-[(5*S*)-5-méthyl-4,5,6,7-tétrahydropyrazolo[3,4-c]pyridin-2-yl]azétidin-1-yl]méthyl]morpholin-4-yl]quinoléine-8-carbonitrile ;
le 5-[(2*R*,6*S*)-2-méthyl-6-[[3-[(5*R*)-5-méthyl-4,5,6,7-tétrahydropyrazolo[3,4-c]pyridin-2-yl]azétidin-1-yl]méthyl]morpholin-4-yl]quinoléine-8-carbonitrile ;
le 5-[(2*R*,6*S*)-2-méthyl-6-[[3-[(7*R*)-7-méthyl-4,5,6,7-tétrahydropyrazolo[3,4-c]pyridin-1-yl]azétidin-1-yl]méthyl]morpholin-4-yl]quinoléine-8-carbonitrile ;
le 5-[(2*R*,6*S*)-2-méthyl-6-[[3-[(7*S*)-7-méthyl-4,5,6,7-tétrahydropyrazolo[3,4-c]pyridin-1-yl]azétidin-1-yl]méthyl]morpholin-4-yl]quinoléine-8-carbonitrile ;
le 5-[(2*R*,6*S*)-2-méthyl-6-[[3-[(7*R*)-7-méthyl-4,5,6,7-tétrahydropyrazolo[3,4-c]pyridin-2-yl]azétidin-1-yl]méthyl]morpholin-4-yl]quinoléine-8-carbonitrile ;
le 5-[(2*R*,6*S*)-2-méthyl-6-[[3-[(7*S*)-7-méthyl-4,5,6,7-tétrahydropyrazolo[3,4-c]pyridin-2-yl]azétidin-1-yl]méthyl]morpholin-4-yl]quinoléine-8-carbonitrile ;
le 5-[(2*R*,6*S*)-2-méthyl-6-[[4-(4,5,6,7-tétrahydropyrazolo[4,3-c]pyridin-1-yl)-1-pipéridyl]méthyl]morpholin-4-yl]quinoléine-8-carbonitrile ;
le 5-[(2*R*,6*S*)-2-méthyl-6-[[4-(4,5,6,7-tétrahydropyrazolo[4,3-c]pyridin-2-yl)-1-pipéridyl]méthyl]morpholin-4-yl]quinoléine-8-carbonitrile ;
le 5-[(2*R*,6*S*)-2-méthyl-6-[[3-[3-(trifluorométhyl)-4,5,6,7-tétrahydropyrazolo[3,4-c]pyridin-1-yl]azétidin-1-yl]méthyl]morpholin-4-yl]quinoléine-8-carbonitrile ;
le 5-[(2*R*,6*S*)-2-méthyl-6-[[3-[3-(trifluorométhyl)-4,5,6,7-tétrahydropyrazolo[3,4-c]pyridin-2-yl]azétidin-1-yl]méthyl]morpholin-4-yl]quinoléine-8-carbonitrile ;
le 5-[(2*R*,6*S*)-2-méthyl-6-[[3-(3-méthyl-4,5,6,7-tétrahydropyrazolo[3,4-c]pyridin-1-yl)azétidin-1-yl]méthyl]morpholin-4-yl]quinoléine-8-carbonitrile ; et
le 5-[(2*R*,6*S*)-2-méthyl-6-[[3-(3-méthyl-4,5,6,7-tétrahydropyrazolo[3,4-c]pyridin-2-yl)azétidin-1-yl]méthyl]morpholin-4-yl]quinoléine-8-carbonitrile ;
ou un sel pharmaceutiquement acceptable de ceux-ci.

11. Procédé de préparation d'un composé selon l'une quelconque des revendications 1 à 10 comprenant l'étape suivante :
a) le couplage du composé de formule (V), 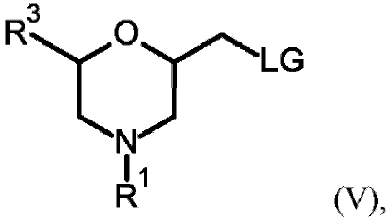 avec un composé de formule (IX), **H-L-R²** (IX), en présence d'une base ;
dans lequel la base de l'étape a) est K₂CO₃, DIPEA ou Cs₂CO₃ ; R¹, R², R³ et L sont tels que définis dans l'une quelconque des revendications 1 à 9.

12. Composé ou sel pharmaceutiquement acceptable, énantiomère ou diastéréoisomère selon l'une quelconque des revendications 1 à 10 pour une utilisation comme substance thérapeutiquement active.

13. Composition pharmaceutique comprenant un composé selon l'une quelconque des revendications 1 à 10 et un véhicule thérapeutiquement inerte.

14. Composé ou sel pharmaceutiquement acceptable, énantiomère ou diastéréoisomère selon l'une quelconque des revendications 1 à 10 pour une utilisation dans une méthode de traitement ou de prophylaxie du lupus érythémateux disséminé ou de la néphropathie lupique.
